# EUROPÄISCHE PATENTSCHRIFT

(11) **EP 1 917 311 B1**
(45) Veröffentlichungstag und Bekanntmachung des Hinweises auf die Patenterteilung: **23.09.2015**
(21) Anmeldenummer: 06778024.7
(22) Anmeldetag: 27.07.2006
(51) Int. Cl.: C09K 11/06, C09K 19/60, C09B 5/62

(54) **MEHRFACHCHROMOPHORE AUF RYLENBASIS**
Rylene based multiple chromophores
CHROMOPHORES MULTIPLES A BASE DE RYLENE

(30) Priorität: 03.08.2005 DE 102005037115
(43) Veröffentlichungstag der Anmeldung: 07.05.2008
(73) Patentinhaber: BASF SE, 67056 Ludwigshafen (DE)
(72) Erfinder: KÖNEMANN, Martin, 68163 Mannheim (DE)
(86) Internationale Anmeldenummer: PCT/EP2006/064733
(87) Internationale Veröffentlichungsnummer: WO 2007/014902

(56) Entgegenhaltungen:
- WO-A-03/104232
- DE-A1- 10 108 156
- NOLDE F ET AL: "Synthesis and modification of terrylenediimides as high-performance fluorescent dyes" CHEMISTRY - A EUROPEAN JOURNAL, VCH PUBLISHERS, US, Bd. 11, Nr. 13, 2005, Seiten 3959-3967, XP002365259 ISSN: 0947-6539
- FRANK WÜRTHNER ET.AL.: "Core-substituted naphthalene bisimides: new fluorophors with tunable emission wavelength for FRET studies" CHEM. EUR. J., Bd. 8, Nr. 20, 2002, Seiten 4742-4750, XP002439542 in der Anmeldung erwähnt

## Beschreibung

Die vorliegende Erfindung betrifft neue Mehrfachchromophore auf Rylenbasis der allgemeinen Formel I' gemäss Anspruch 1.

Weiterhin beschreibt die vorliegende Anmeldung Mehrfachchromophore auf Rylenbasis der allgemeinen Formel I 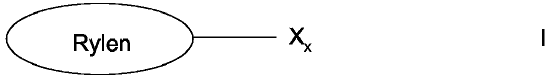 in der die Variablen folgende Bedeutung haben:
Rylen ein Rest der Formel 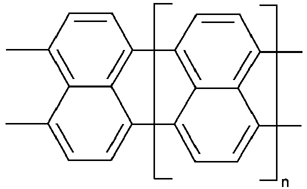 der durch mindestens eine Imidgruppe, Estergruppe oder Amidgruppe funktionalisiert ist und der zusätzlich durch Acyloxy, Arylthio, Hetaryloxy und/oder Hetarylthio substituiert sein kann;
   - X: ein Rylendicarbonsäureimidrest, der bei einer anderen Wellenlänge absorbiert als der Rest Rylen, in peri-Position über eine Gruppierung

   -Y'-A-Y-

   die über Y an den Rest X gebunden ist, mit dem Rylenrest verknüpft ist und ebenfalls durch Aryloxy, Arylthio, Hetaryloxy und/oder Hetarylthio substituiert sein kann;
   - A: ein mindestens einen aromatischen oder hetaromatischen Rest aufweisendes Brückenglied, wobei die Gruppen Y oder Y und Y' an den aromatischen oder hetaromatischen Rest gebunden sind;
   - Y: eine Gruppierung 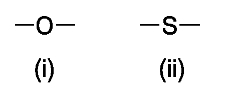
   - Y': eine Gruppierung 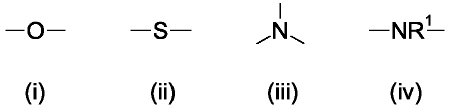 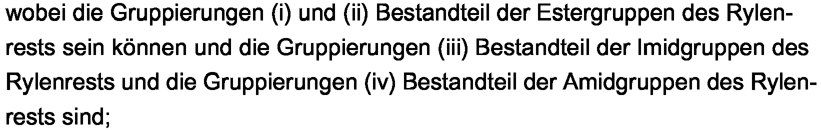
   - R¹: Wasserstoff oder C₁-C₁₈-Alkyl, wobei die Reste R¹ gleich oder verschieden sein können, wenn sie mehrfach auftreten;
   - n: 1, 2 oder 3;
   - x: 1 bis 7.

Weiterhin betrifft die Erfindung neue Rylendicarbonsäureimidderivate der allgemeinen Formel III 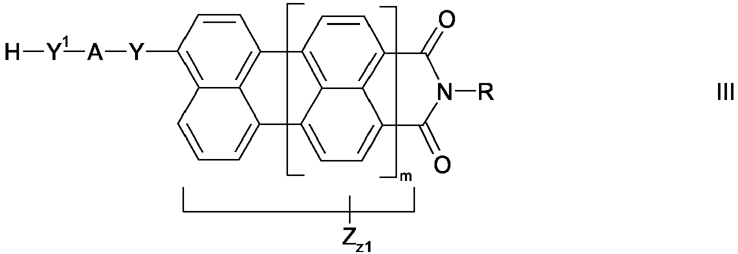 in der die Variablen folgende Bedeutung haben:
- Z: Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder -SO₂- unterbrochen sein kann, anneliert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch:
   C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² und/oder -SO₃R²;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder Hetaryl, das jeweils ein- oder mehrfach durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR² und/oder -SO₃R² substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂- bedeutet;
(v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² oder -SO₃R²,
   wobei die Reste Z für z > 1 und/oder z1 > 1 gleich oder verschieden sein können;
- A: ein mindestens einen aromatischen oder hetaromatischen Rest aufweisendes Brückenglied, wobei die Gruppen Y oder Y und Y¹ an den aromatischen oder hetaromatischen Rest gebunden sind;
- Y: -O- oder -S-;
- Y¹: -O-, -S- oder -NR¹-;
- R: Wasserstoff;
C₁-C₃₀-Alkyl, dessen Kohlenstoffkette durch eine oder mehrere Gruppierungen-O-, -S-, -NR¹-, -N=CR¹-, -C≡C-, -CR¹=CR¹-, -CO-, -SO- und/oder -SO₂- unterbrochen sein kann und das ein- oder mehrfach durch die als Substituenten für die Reste Z genannten Reste (ii), (iii), (iv) und/oder (v) substituiert sein kann; C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach durch die als Substituenten für die Reste Z genannten Reste (i), (ii), (iii), (iv) und/oder (v) substituiert sein kann;
Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch die als Substituenten für die Reste Z genannten Reste (i), (ii), (iii), (iv), (v) und/oder Aryl- und/oder Hetarylazo, das jeweils ein- oder mehrfach durch C₁-C₁₀-Alkyl, C₁-C₆-Alkoxy und/oder Cyano substituiert sein kann,
wobei die Reste R gleich oder verschieden sein können, wenn sie mehrfach in Formel I auftreten;
- R¹: Wasserstoff oder C₁-C₁₈-Alkyl, wobei die Reste R¹ gleich oder verschieden sein können, wenn sie mehrfach auftreten;
- R², R³: unabhängig voneinander Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR¹ substituiert sein kann;
Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann;
- m: 0, 1 oder 2;
- z1: für m = 0: 0;
für m = 1: 0 bis 2;
für m = 2: 2 bis 4,
als Zwischenprodukte für die Mehrfachchromophore I.

Außerdem betrifft die Erfindung Verfahren zur Herstellung der Mehrfachchromophore I und der Rylendicarbonsäureimidderivate III.

Schließlich betrifft die Erfindung die Verwendung der Mehrfachchromophore I zur Einfärbung von organischen und anorganischen Materialien, zur Herstellung elektromagnetische Strahlung absorbierender und/oder emittierender wäßriger Polymerisatdispersionen, zur Erzeugung für das menschliche Auge nicht sichtbarer Markierungen und Beschriftungen, als Filter oder Emitter in Display-Anwendungen, als Emitter in Chemilumineszenzanwendungen und als Aktivkomponenten in der Photovoltaik.

Rylenchromophore, insbesondere die Rylentetracarbonsäurediimide und -dicarbonsäureimide, stellen eine chemisch sehr stabile Klasse von Effektstoffen dar und sind daher für viele technische Anwendungsgebiete von Interesse. Die Chromophore auf Perylen- und Terrylenbasis zeichnen sich dabei insbesondere durch ihre Fluoreszenzeigenschaften aus, während bei den höheren Homologen vor allem ihre Absorption im Nahinfrarotbereich des elektromagnetischen Spektrums von Bedeutung ist.

Die Rylenchromophore sind bekannt und z.B. in EP-A-596 292, den WO-A-96/22331, 96/22332, 97/22607, 02/66438, 02/76988 und 03/104232 und den älteren deutschen Patentanmeldungen 10 2005 021 362.6 und 10 2005 032 583.1 beschrieben.

Durch Modifizierung ihres Absorptionsverhaltens kann die Wirkung der Rylenchromophore noch weiter verbessert werden. So ist eine Erweiterung des absorbierten Spektralbereichs für viele Anwendungen, z.B. für eine optimale Ausnutzung des Fluoreszenzeffekts bei Tageslicht, von Vorteil. Diese Erweiterung kann vorteilhaft dadurch erreicht werden, daß unterschiedlich absorbierende Chromophore über chemische Bindungen miteinander verknüpft werden. In diesen Mehrfachchromophoren wird die Energie vom kürzerwellig absorbierenden Chromophor auf den längerwellig absorbierenden Chromophor übertragen.

In Chem. Eur. J. 5, Seite 2388-2395 (1999) werden Bichromophore auf Terrylen/Perylen-Basis beschrieben, bei denen ein peri-hexylsubstituiertes Perylen über einen Hexamethylenrest an das zweite Imidstickstoffatom von N-(2,6-Diisopropylphenyl)-1,6-di(4-tert.-octylphenoxy)terrylentetracarbonsäurediimid gebunden ist.

Auch die in der DE-A-102 12 358 beschriebenen Bichromophore basieren auf Perylentetracarbonsäureimiden, die an einem Imidstickstoffatom über eine Ester- oder Amidfunktion oder direkt mit einem kürzerwellig absorbierenden Chromophor aus der Gruppe Anthrachinon, Anthracen, Naphthalin, Pyren oder Fluoren verknüpft sind.

Dendritische Mehrfachchromophore sind aus Angew. Chem. 114, 1980-1984 (2002) bekannt. Hier wird ein zentrales N,N'-Bis(2,6-diisopropylphenoxy)terrylentetracarbon-säurediimidmolekül mit 4 oder 8 N-(2,6-Diisopropylphenyl)perylendicarbonsäureimid-molekülen bzw. mit 4 N-(2,6-Diisopropylphenyl)perylendicarbonsäureimidmolekülen und 8 N-(2,6-Diisopropylphenyl)naphthalindicarbonsäureimidmolekülen kombiniert. Die Verknüpfung erfolgt hier über (Pentaphenyl)phenyleinheiten.

In Chem. Eur. 8, 4742-4750 (2002) sind Bichromophore auf Basis zweier über eine Amidfunktion verbrückter Naphthalintetracarbonsäurediimide beschrieben, bei denen das chromophore System eines Diimids durch Einführung von Butylaminosubstituenten modifiziert wurde, um seine Absorption bathochrom zu verschieben.

Schließlich sind in J. Am. Chem. Soc. 126, 8284-8294 (2004) Bichromophore auf Basis von unterschiedlich substituierten Perylentetracarbonsäurediimiden beschrieben, bei denen die außen stehenden Chromophore über Phenylenbrücken an die Imidstickstoffatome und direkt an das Rylengerüst des zentralen Chromophors gebunden sind. Die aufgrund ihrer Absorptions- und Emissionseigenschaften besonders interessanteren bekannten Mehrfachchromophore sind nur durch aufwendige, vielstufige Synthesen unter Einsatz von Schutzgruppenchemie zugänglich.

Der Erfindung lag daher die Aufgabe zugrunde, Mehrfachchromophore bereitzustellen, die auf verfahrenstechnisch vorteilhafte Weise, insbesondere auch im technischen Maßstab über wenige Synthesestufen, herzustellen sind und deren Absorptions- und Emissionseigenschaften gezielt an den jeweiligen Anwendungszweck angepaßt werden können.

Demgemäß wurden die Mehrfachchromophore der Formel I 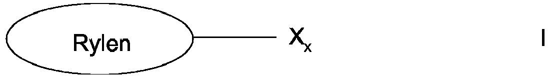 gefunden.

Die anmeldungsgemäßen Mehrfachchromophore I bestehen aus dem Chromophor Rylen auf Basis von Perylen, Terrylen oder Quaterrylen, an den mindestens ein Rylendicarbonsäureimid, das bei einer anderen Wellenlänge absorbiert als der Chromophor Rylen, als weiterer Chromophor X gebunden ist.

Der Chromophor X kann dieselbe Anzahl Naphthalineinheiten aufweisen wie der Chromophor Rylen, muß sich dann jedoch durch die Art der Substitution von diesem unterscheiden (andere oder mehr oder weniger Substituenten), so daß die Wellenlänge seiner Absorption gegenüber der des Chromophors Rylen verschoben ist.

Bevorzugt unterscheidet sich der Chromophor X in der Anzahl der Naphthalineinheiten vom Chromophor Rylen. Besonders bevorzugt handelt es sich bei dem Chromophor X um das jeweils nächstniedere Homologe, also ein Naphthalin-, Perylen- bzw. Terrylendicarbonsäureimid.

Der Chromophor Rylen der erfindungsgemäßen Mehrfachchromophore ist durch mindestens eine Imid-, Ester- oder Amidgruppe oder durch 2 vorzugsweise gleiche Gruppen, funktionalisiert. Bevorzugt sind die Rylentetracarbonsäurediimide, Rylendicarbonsäureester, Rylendicarbonsäureamide und Rylendicarbonsäureimide, wobei die Rylentetracarbonsäurediimide und Rylendicarbonsäureimide besonders bevorzugt und die Rylentetracarbonsäurediimide ganz besonders bevorzugt sind.

Der Chromophor X ist in peri-Position über die Gruppierung

-Y'-A-Y-

die über Y (-O oder -S-) an X gebunden ist, mit dem Chromophor Rylen verknüpft.

Der Chromophor X kann an das Kohlenstoffgerüst des Chromophors Rylen oder über die ihn funktionalisierenden Gruppen gebunden sein.

Die Bindung an das Kohlenstoffgerüst erfolgt dabei über eine Gruppierung -O- oder -S-(Y' = (i) oder (ii)). Die Bindung über die funktionalisierenden Gruppen ist auf verschiedene Weise möglich. So kann Y' Bestandteil der Imidgruppe (Y' = (iii)), der Amidgruppe (Y' = (iv)) oder der Estergruppe (Y' = (i) oder (ii)) sein.

Das die Gruppierungen Y und Y' verbindende Brückenglied A weist mindestens einen aromatischen oder hetaromatischen Rest auf, an den Y und Y' gebunden sind.

Vorzugsweise ist A ein Arylen- oder Hetarylenrest der Formeln 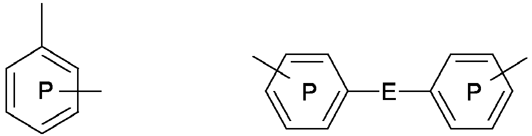 in denen die Ringe P gleich oder verschieden sein können, Heteroatome als Ringatome enthalten können und/oder annelierte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, aufweisen können, wobei das gesamte Ringsystem ein- oder mehrfach durch die vorstehend als Substituenten für die Reste Z genannten Reste (i), (ii), (iii) und/oder (v) substituiert sein kann.

E bedeutet dabei:
eine chemische Bindung;
eine Gruppierung -O-, -S-, -NR¹-, -N=CR¹-, -C≡C-, -CR¹=CR¹-, -CO-, -SO- oder -SO₂- oder C₁-C₁₂-Alkylen oder C₄-C₇-Cycloalkylen, dessen Kohlenstoffkette jeweils ein- oder mehrfach durch diese Gruppierungen unterbrochen sein kann und das jeweils ein- oder mehrfach durch die vorstehend als Substituenten für die Reste Z genannten Reste (i), (ii), (iii) und/oder (v) substituiert sein kann;
Arylen oder Hetarylen, das ebenfalls jeweils ein- oder mehrfach durch die Reste (i), (ii), (iii) und/oder (v) substituiert sein kann, wobei Hydroxy und Mercapto als Reste (v) ausgeschlossen sind.

Besonders bevorzugte Brückenglieder A sind Arylenreste der Formeln 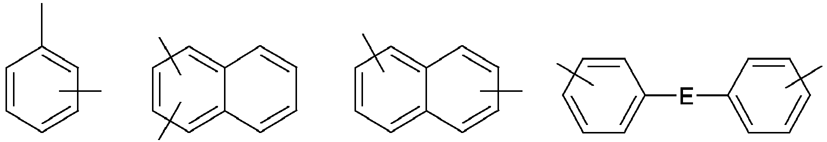 in denen die Phenylen- oder Naphthylenringe ein- oder mehrfach durch C₁-C₁₈-Alkyl substituiert sein können und E eine chemische Bindung, Methylen oder Isopropylen bedeutet.

Beispiele für besonders bevorzugte Brückenglieder A sind im einzelnen:
1,4-, 1,3- und 1,2-Phenylen, 1,4-[2,5-Di(tert.-butyl)]phenylen, 1,4-(2,5-Dihexyl)-phenylen, 1,4-[2,5-Di(tert.-octyl)]phenylen, 1,4-(2,5-Didodecyl)phenylen, 1,4-[2,5-Di(2-dodecyl)]phenylen,1,4- und 1,8-Naphthylen, 4,4'-, 3,3'- und 2,2'-Biphenylen, 4,4'-Di(2,2',6,6'-tetramethyl)phenylen, 4,4'-Di(2,2',6,6'-tetraethyl)phenylen, 4,4'-Di(2,2',6,6'-tetraisopropyl)phenylen, 4,4'-Di(2,2',6,6'-tetrahexyl)phenylen, 4,4'-Di[2,2',6,6'-tetra(2-hexyl)]phenylen, 4,4'-Di[2,2',6,6'-tetra(tert.-octyl)]phenylen, 4,4'-Di(2,2',6,6'-tetra-dodecyl)phenylen und 4,4'-Di[2,2',6,6'-tetra(2-dodecyl)]phenylen sowie 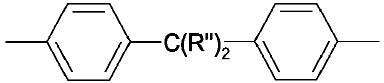
wobei R" Wasserstoff, Methyl, Ethyl oder Phenyl bedeutet.

Ganz besonders bevorzugte Brückenglieder A sind 1,4-Phenylen und 4,4'-Di(2,2',6,6'-tetramethyl)phenylen.

Der Chromophor Rylen und/oder der Chromophor X können weiterhin im Rylengerüst durch (Het)Aryloxy- oder (Het)Arylthioreste substituiert sein.

Als Beispiele für besonders geeignete Substituenten seien dabei Phenoxy, Thiophenoxy, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio genannt, wobei Phenoxy und Thiophenoxy, bevorzugt sind und Phenoxy- besonders bevorzugt ist.

Durch die Einführung dieser Substituenten kann das jeweilige chromophore System vergrößert und damit die Absorption gezielt bathochrom verschoben werden.

Die (Het)Aryloxy- oder (Het)Arylthioreste selbst können wiederum beliebig substituiert sein. Geeignete Substituenten sind nachfolgend bei der Beschreibung der bevorzugten erfindungsgemäßen Mehrfachchromophoren aufgeführt.

Durch gezielte Substitution der (Het)Aryloxy- oder (Het)Arylthioreste kann zusätzlich die Form der Absorptionsbande des jeweiligen Chromophors beeinflußt werden. So bewirken in ortho,ortho'-Position substituierte (Het)Aryloxy- oder (Het)Arylthioreste eine Erhöhung der Fluoreszenzquantenausbeuten, weshalb diese Reste von besonderem Interesse als Substituenten für den längerwellig absorbierenden, emittierenden Chromophor sind. Durch diese Reste substituierte Rylentetra- und -dicarbonsäurederivate sind in der älteren deutschen Patentanmeldung 10 2005 032 583.1 beschrieben.

Besonders geeignete ortho,ortho'-substituierte Phenoxy- und Thiophenoxyreste weisen die Formel 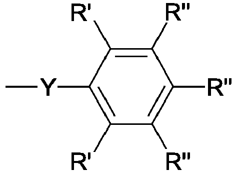 auf, in der die Variablen folgende Bedeutung haben:
- Y: -O- oder -S-, vorzugsweise -O-;
- R': gleiche oder verschiedene Reste:
(i) C₁-C₁₈-Alkyl, dessen Kohlenstoffkette durch eine oder mehrere Gruppierungen -O-, -S-, -NR¹- und/oder -CO- unterbrochen sein kann und das ein- oder mehrfach durch C₁-C₁₂-Alkoxy, Hydroxy und/oder Halogen substituiert sein kann, wobei höchstens ein Alkylrest R' in der 1-Position ein tertiäres Kohlenstoffatom aufweisen kann;
(ii) C₃-C₈-Cycloalkyl, das ein- oder mehrfach durch C₁-C₁₈-Alkyl und/oder C₁-C₁₂-Alkoxy substituiert sein kann, wobei höchstens ein Cycloalkylrest R' in der 1-Position ein tertiäres Kohlenstoffatom aufweisen kann;
(iii) Aryl oder Hetaryl, das jeweils ein- oder mehrfach durch C₁-C₁₈-Alkyl, C₁-C₁₂-Alkoxy, Hydroxy und/oder Halogen substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S- oder -NR¹- bedeutet;
(v) C₁-C₁₂-Alkoxy, Hydroxy, Halogen oder Cyano;
- R": gleiche oder verschiedene Reste:
Wasserstoff;
einer der für R' genannten Reste (i), (ii), (iii), (iv) und (v), wobei die Alkylreste (i) und die Cycloalkylreste (ii) in der 1-Position tertiäre Kohlenstoffatome aufweisen können;
- R¹: Wasserstoff oder C₁-C₆-Alkyl.

Ganz besonders bevorzugte Reste R' sind dabei die Alkyl-, Cycloalkyl- und Phenylreste, vor allem die Alkylreste R' mit sekundärem oder primärem Kohlenstoffatom in der 1-Position sowie Methyl und die Cycloalkylreste R' mit sekundärem Kohlenstoffatom in der 1-Position, wobei die Alkyl- und Cycloalkylreste mit sekundärem Kohlenstoffatom in der 1-Position besonders hervorzuheben sind.

Vorzugsweise sind diese Phenoxy- und Thiophenoxyreste nur in ortho- und ortho'-Position oder zusätzlich in para-Position substituiert.

Als Beispiele für ganz besonders bevorzugte Phenoxy- und Thiophenoxyreste seien im einzelnen genannt:
2,6-Dimethylphenoxy, 2,6-Diethylphenoxy, 2,6-Diisopropylphenoxy, 2,6-Di(2-butyl)-phenoxy, 2,6-Di(n-butyl)phenoxy, 2,6-Di(2-hexyl)phenoxy, 2,6-Di(n-hexyl)phenoxy, 2,6-Di(2-dodecyl)phenoxy, 2,6-Di(n-dodecyl)phenoxy, 2,6-Dicyclohexylphenoxy, 2,6-Diphenylphenoxy, 2,6-Di-methyl-4-(n-butyl)phenoxy, 2,6-Diethyl-4-(n-butyl)phenoxy, 2,6-Diisopropyl-4-(n-butyl)phenoxy, 2,6-Di(2-butyl)-4-(n-butyl)phenoxy, 2,4,6-Tri(n-butyl)phenoxy, 2,6-Di(2-hexyl)-4-(n-butyl)phenoxy, 2,6-Di(n-hexyl)-4-(n-butyl)phenoxy, 2,6-Di(2-dodecyl)-4-(n-butyl)-phenoxy, 2,6-Di(n-dodecyl)-4-(n-butyl)phenoxy, 2,6-Dicyclohexyl-4-(n-butyl)phenoxy, 2,6-Diphenyl-4-(n-butyl)phenoxy, 2,6-Dimethyl-4-(n-nonyl)phenoxy, 2,6-Diethyl-4-(n-nonyl)phenoxy, 2,6-Diisopropyl-4-(n-nonyl)phenoxy, 2,6-Di(2-butyl)-4-(n-nonyl)phenoxy, 2,6-Di(2-butyl)-4-(n-nonyl)phenoxy, 2,6-Di(2-hexyl)-4-(n-nonyl)phenoxy, 2,6-Di(n-hexyl)-4-(n-nonyl)phenoxy, 2,6-Di(2-dodecyl)-4-(n-nonyl)-phenoxy, 2,6-Di(n-dodecyl)-4-(n-nonyl)phenoxy, 2,6-Dicyclohexyl-4-(n-nonyl)phenoxy, 2,6-Diphenyl-4-(n-nonyl)phenoxy, 2,6-Dimethyl-4-(n-octadecyl)phenoxy, 2,6-Diethyl-4-(n-octadecyl)phenoxy, 2,6-Diisopropyl-4-(n-octadecyl)phenoxy, 2,6-Di(2-butyl)-4-(n-octadecyl)phenoxy, 2,6-Di(2-butyl)-4-(n-octadecyl)phenoxy, 2,6-Di(2-hexyl)-4-(n-octa-decyl)phenoxy, 2,6-Di(n-hexyl)-4-(n-octadecyl)phenoxy, 2,6-Di(2-dodecyl)-4-(n-octa-decyl)phenoxy, 2,6-Di(n-dodecyl)-4-(n-octadecyl)phenoxy, 2,6-Dicyclohexyl-4-(n-octa-decyl)phenoxy,2,6-Dimethyl-4-(tert.-butyl)phenoxy, 2,6-Diethyl-4-(tert.-butyl)phenoxy, 2,6-Diisopropyl-4-(tert.-butyl)phenoxy, 2,6-Di(2-butyl)-4-(tert.-butyl)phenoxy, 2,6-Di-(n-butyl)-4-(tert.-butyl)phenoxy, 2,6-Di(2-hexyl)-4-(tert.-butyl)phenoxy, 2,6-Di(n-hexyl)-4-(tert.-butyl)phenoxy, 2,6-Di(2-dodecyl)-4-(tert.-butyl)phenoxy, 2,6-Di(n-dodecyl)-4-(tert.-butyl)phenoxy, 2,6-Dicyclohexyl-4-(tert.-butyl)phenoxy, 2,6-Diphenyl-4-(tert.-butyl)-phenoxy, 2,6-Dimethyl-4-(tert.-octyl)phenoxy, 2,6-Diethyl-4-(tert.-octyl)phenoxy, 2,6-Diisopropyl-4-(tert.-octyl)phenoxy, 2,6-Di(2-butyl)-4-(tert.-octyl)phenoxy, 2,6-Di-(n-butyl)-4-(tert.-octyl)phenoxy, 2,6-Di(2-hexyl)-4-(tert.-octyl)phenoxy, 2,6-Di(n-hexyl)-4-(tert.-octyl)phenoxy, 2,6-Di(2-dodecyl)-4-(tert.-octyl)phenoxy, 2,6-Di(n-dodecyl)-4-(tert.-octyl)phenoxy, 2,6-Dicyclohexyl-4-(tert.-octyl)phenoxy und 2,6-Diphenyl-4-(tert.-octyl)-phenxoy;
2,6-Dimethylthiophenoxy, 2,6-Diethylthiophenoxy, 2,6-Diisopropylthiophenoxy, 2,6-Di(2-butyl)thiophenoxy, 2,6-Di(n-butyl)thiophenxoy, 2,6-Di(2-hexyl)thiophenoxy, 2,6-Di(n-hexyl)thiophenoxy, 2,6-Di(2-dodecyl)thiophenoxy, 2,6-Di(n-dodecyl)thiophenoxy, 2,6-Dicyclohexylthiophenoxy, 2,6-Diphenylthiophenoxy, 2,6-Dimethyl-4-(n-butyl)thiophenoxy, 2,6-Diethyl-4-(n-butyl)thiophenoxy, 2,6-Diisopropyl-4-(n-butyl)thiophenoxy, 2,6-Di(2-butyl)-4-(n-butyl)thiophenoxy, 2,4,6-Tri(n-butyl)thiophenoxy, 2,6-Di(2-hexyl)-4-(n-butyl)thiophenoxy, 2,6-Di(n-hexyl)-4-(n-butyl)thiophenoxy, 2,6-Di(2-dodecyl)-4-(n-butyl)thiophenoxy, 2,6-Di(n-dodecyl)-4-(n-butyl)thiophenoxy, 2,6-Dicyclohexyl-4-(n-butyl)thiophenoxy, 2,6-Diphenyl-4-(n-butyl)phenxoy, 2,6-Di-methyl-4-(n-nonyl)thiophenoxy, 2,6-Diethyl-4-(n-nonyl)thiophenoxy, 2,6-Diisopropyl-4-(n-nonyl)thiophenoxy, 2,6-Di(2-butyl)-4-(n-nonyl)thiophenoxy, 2,6-Di(2-butyl)-4-(n-nonyl)thiophenoxy, 2,6-Di(2-hexyl)-4-(n-nonyl)thiophenoxy, 2,6-Di(n-hexyl)-4-(n-nonyl)thiophenoxy, 2,6-Di(2-dodecyl)-4-(n-nonyl)thiophenoxy, 2,6-Di(n-dodecyl)-4-(n-nonyl)thiophenoxy, 2,6-Dicyclohexyl-4-(n-nonyl)thiophenoxy, 2,6-Diphenyl-4-(n-nonyl)thiophenoxy, 2,6-Dimethyl-4-(n-octadecyl)thiophenoxy, 2,6-Diethyl-4-(n-octadecyl)thiophenoxy, 2,6-Diiso-propyl-4-(n-octadecyl)thiophenoxy, 2,6-Di(2-butyl)-4-(n-octadecyl)thiophenoxy, 2,6-Di(2-butyl)-4-(n-octadecyl)thiophenoxy, 2,6-Di(2-hexyl)-4-(n-octadecyl)thiophenoxy, 2,6-Di(n-hexyl)-4-(n-octadecyl)thiophenoxy, 2,6-Di(2-dodecyl)-4-(n-octadecyl)thiophenoxy, 2,6-Di(n-do-decyl)-4-(n-octadecyl)thiophenoxy, 2,6-Dicyclohexyl-4-(n-octadecyl)-thiophenoxy, 2,6-Dimethyl-4-(tert.-butyl)thiophenoxy, 2,6-Diethyl-4-(tert.-butyl)thiophenoxy, 2,6-DÜso-propyl-4-(tert.-butyl)thiophenoxy, 2,6-Di(2-butyl)-4-(tert.-butyl)thiophenoxy, 2,6-Di-(n-butyl)-4-(tert.-butyl)thiophenoxy, 2,6-Di(2-hexyl)-4-(tert.-butyl)thiophenoxy, 2,6-Di(n-hexyl)-4-(tert.-butyl)thiophenoxy, 2,6-Di(2-dodecyl)-4-(tert.-butyl)thiophenoxy, 2,6-Di(n-dodecyl)-4-(tert.-butyl)thiophenoxy, 2,6-Dicyclohexyl-4-(tert.-butyl)-thiophenoxy, 2,6-Diphenyl-4-(tert.-butyl)thiophenoxy, 2,6-Dimethyl-4-(tert.-octyl)thiophenoxy, 2,6-Diethyl-4-(tert.-octyl)thiophenoxy, 2,6-Diiso-propyl-4-(tert.-octyl)thiophenoxy, 2,6-Di(2-butyl)-4-(tert.-octyl)thiophenoxy, 2,6-Di-(n-butyl)-4-(tert.-octyl)thiophenoxy, 2,6-Di(2-hexyl)-4-(tert.-octyl)thiophenoxy, 2,6-Di(n-hexyl)-4-(tert.-octyl)thiophenoxy, 2,6-Di(2-dodecyl)-4-(tert.-octyl)thiophenoxy, 2,6-Di(n-dodecyl)-4-(tert.-octyl)thiophenoxy, 2,6-Dicyclohexyl-4-(tert.-octyl)thiophenoxy und 2,6-Diphenyl-4-(tert.-octyl)-thiophenoxy.

Die vorliegende Erfindung betrifft neue Mehrfachchromophore der Formel I' gemäß Anspruch 1 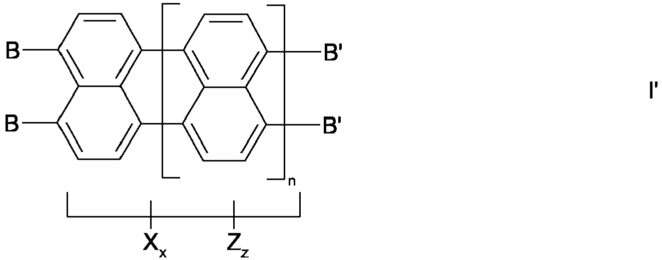 auf, in der die Variablen die folgende Bedeutung haben:
- X: ein Rylendicarbonsäureimidrest der Formel 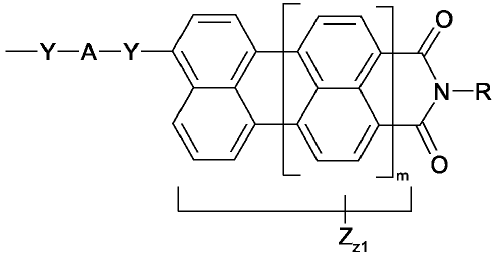 wobei die Reste X gleich oder verschieden sein können;
- Z: Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder -SO₂- unterbrochen sein kann, anneliert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C=CR¹, -CR¹=CR¹₂, Hydroxy, Mercapto, Halogen, Cyano, Nitro, -NR²R³, -NR²COR³, -CONR²R³, -SO₂NR²R³, -COOR² und/oder -SO₃R²;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C=CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder Hetaryl, das jeweils ein- oder mehrfach durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR² und/oder -SO₃R² substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂- bedeutet;
(v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² oder -SO₃R²,
   wobei die Reste Z für z > 1 und/oder z1 > 1 gleich oder verschieden sein können;
- B: für n = 1, 2 oder 3:
miteinander unter Ausbildung eines Sechsrings verbunden zu einem Rest der Formel (a) oder (b) 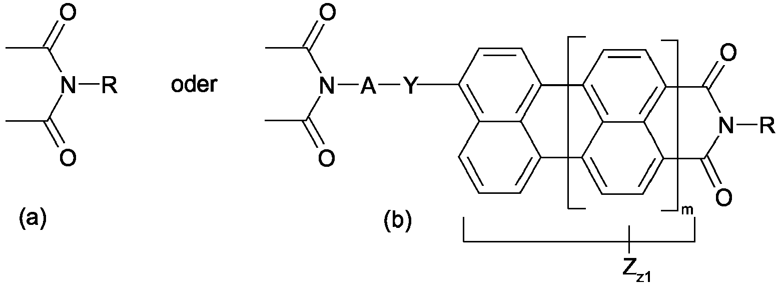 oder
ein Rest B Wasserstoff und der andere Rest einen Rest X; für n = 1 zusätzlich:
beide Wasserstoff, ein Rest B Wasserstoff und der andere Rest Z oder
ein Rest B Wasserstoff, Halogen oder Cyano und der andere Rest einen Rest der Formel (c) 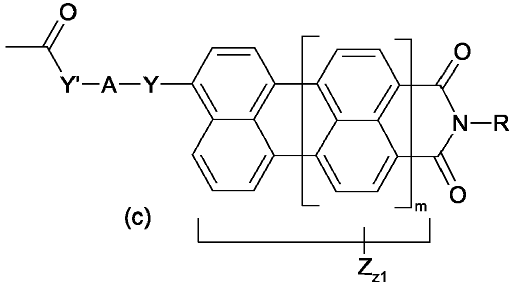
- B': für n = 1, 2 oder 3:
miteinander unter Ausbildung eines Sechsrings verbunden zu einem Rest der Formel (a), wenn die Reste B zusammen einen Rest der Formel (a) bedeuten; miteinander unter Ausbildung eines Sechsrings verbunden zu einem Rest der Formel (a), wenn ein Rest B Wasserstoff und der andere Rest einen Rest X bedeutet, wobei für n = 2 oder 3 gilt: x = 0 und z ≠ 0;
miteinander unter Ausbildung eines Sechsrings verbunden zu einem Rest der Formel (b), wenn die Reste B zusammen einen Rest der Formel (a) oder (b) bedeuten oder ein Rest B Wasserstoff und der andere Rest einen Rest X oder
Z bedeutet, wobei für n = 2 oder 3 gilt: x = 0 und z ≠ 0;
für n = 1 zusätzlich:
miteinander unter Ausbildung eines Sechsrings verbunden zu einem Rest der Formel (a) oder (b), wenn ein Rest B Wasserstoff und der andere Rest einen Rest Z bedeutet;
ein Rest B' Wasserstoff, Halogen oder Cyano und der andere Rest einen Rest der Formel (c), wenn ein Rest B Wasserstoff, Halogen oder Cyano und der andere Rest einen Rest der Formel (c) bedeutet;
- A: ein mindestens einen aromatischen oder hetaromatischen Rest aufweisendes Brückenglied, wobei die Gruppen Y oder Y und Y¹ an den aromatischen oder hetaromatischen Rest gebunden sind;
- Y: -O- oder -S-;
- Y¹: -O-, -S- oder -NR¹-;
- R: Wasserstoff;
C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach durch die als Substituenten für die Reste Z genannten Reste (ii), (iii), (iv) und/oder (v) substituiert sein kann; C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach durch die als Substituenten für die Reste Z genannten Reste (i), (ii), (iii), (iv) und/oder (v) substituiert sein kann;
Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch die als Substituenten für die Reste Z genannten Reste (i), (ii), (iii), (iv), (v) und/oder Aryl- und/oder Hetarylazo, das jeweils ein- oder mehrfach durch C₁-C₁₀-Alkyl, C₁-C₆-Alkoxy und/oder Cyano substituiert sein kann,
wobei die Reste R gleich oder verschieden sein können, wenn sie mehrfach in Formel I auftreten;
- R¹: Wasserstoff oder C₁-C₁₈-Alkyl, wobei die Reste R¹ gleich oder verschieden sein können, wenn sie mehrfach auftreten;
- R², R³: unabhängig voneinander Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR¹ substituiert sein kann;
Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann;
- n: 1, 2 oder 3;
- m: 0, 1 oder 2;
- x: für n = 1:
2 bis 4 oder auch 0, wenn die Reste B und die Reste B' jeweils einen Rest der Formel (b) oder ein Rest B und ein Rest B' jeweils einen Rest der Formel (c) bedeuten;
für n = 2:
2 bis 6, wenn die Reste B und die Reste B' jeweils einen Rest der Formel (a) bedeuten;
0 für alle weiteren Bedeutungen der Reste B und B'; für n = 3:
2 bis 4, wenn die Reste B und die Reste B' jeweils einen Rest der Formel (a) bedeuten;
0 für alle weiteren Bedeutungen der Reste B und B';
- z: 0 bis 8 mit x + z ≤ 8 ist, wobei für n = 2 oder 3 gilt: z ≠ 0, wenn x = 0 ist;
- z1: für m = 0: 0;
für m = 1: 0 bis 2;
für m = 2: 2 bis 4.

Bei besonders bevorzugten Mehrfachchromophoren der Formel I' haben die Variablen folgende Bedeutung:
- Z: Phenoxy oder Thiophenoxy, das jeweils ein- oder mehrfach durch gleiche oder verschiedene Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -C≡C-, -CR¹=CR¹- und/oder -CO- und/oder -SO₂- unterbrochen sein kann und das ein- oder mehrfach substituiert sein kann durch: C₁-C₁₂-Alkoxy, Hydroxy, Halogen, Cyano und/oder Aryl, das ein- oder mehrfach durch C₁-C₁₈-Alkyl und/oder C₁-C₆-Alkoxy substituiert sein kann;
(ii) C₃-C₈-Cycloalkyl, dessen Kohlenstoffgerüst durch eine oder mehrere Gruppierungen -O-, -S-, -NR¹-, -CR¹=CR¹- und/oder -CO- unterbrochen sein kann und das ein- oder mehrfach durch C₁-C₁₈-Alkyl, C₁-C₁₂-Alkoxy und/oder C₁-C₆-Alkylthio substituiert sein kann;
(iii) Aryl oder Hetaryl, an das jeweils weitere 5- bis 7-gliedrige gesättigte oder ungesättigte Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, -C=CR¹-, -CR¹=CR¹-, Hydroxy, Halogen, Cyano, -NR²R³, -NR²COR³, -CONR²R³, -SO₂NR²R³, -COOR² und/oder -SO₃R², Aryl und/oder Hetaryl, das jeweils ein- oder mehrfach durch C₁-C₁₈-Alkyl, C₁-C₁₈-Alkoxy und/oder Cyano substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂- bedeutet;
(v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² oder -SO₃R²,
   wobei die Reste Z für z > 1 und/oder z1 > 1 gleich oder verschieden sein können;
- A: ein Arylen- oder Hetarylenrest der Formeln 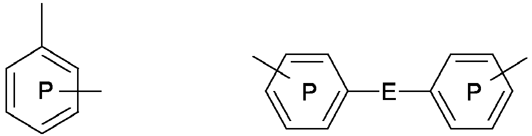 in denen die Ringe P gleich oder verschieden sein können, Heteroatome als Ringatome enthalten können und/oder annelierte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, aufweisen können, wobei das gesamte Ringsystem ein- oder mehrfach durch die als Substituenten für die Reste Z genannten Reste (i), (ii), (iii) und/oder (v) substituiert sein kann,
wobei die Reste A gleich oder verschieden sein können, wenn sie mehrfach in Formel I auftreten;
- E: eine chemische Bindung, eine Gruppierung -O-, -S-, -NR¹-, -N=CR¹-, -C≡C-, -CR¹=CR¹-, -CO-, -SO- oder -SO₂- oder C₁-C₁₂-Alkylen oder C₄-C₇-Cyclo-alkylen, dessen Kohlenstoffkette jeweils ein- oder mehrfach durch diese Gruppierungen unterbrochen sein kann und das jeweils ein- oder mehrfach durch die als Substituenten für die Reste Z genannten Reste (i), (ii), (iii) und/oder (v) substituiert sein kann;
Arylen oder Hetarylen, das jeweils ein- oder mehrfach die als Substituenten für die Reste Z genannten Reste (i), (ii), (iii) und/oder (v) substituiert sein kann, wobei Hydroxy und Mercapto als Reste (v) ausgeschlossen sind;
- Y: -O-;
- Y¹: -O- oder -NR¹-;
- R: C₁-C₃₀-Alkyl, dessen Kohlenstoffkette durch eine oder mehrere Gruppierungen -O- und/oder -CO- unterbrochen sein kann und das ein- oder mehrfach substituiert sein kann durch: C₁-C₆-Alkoxy, Cyano und/oder Aryl, das ein- oder mehrfach durch C₁-C₁₈-Alkyl und/oder C₁-C₆-Alkoxy substituiert sein kann; Phenyl, Naphthyl, Pyridyl oder Pyrimidyl, das jeweils ein- oder mehrfach substituiert sein kann durch: C₁-C₁₈-Alkyl, C₁-C₆-Alkoxy, Halogen, Cyano, Nitro, -CONR²R³, -SO₂NR²R³ und/oder Phenyl- und/oder Naphthylazo, das jeweils ein- oder mehrfach durch C₁-C₁₀-Alk_{Y}1, C₁-C₆-Alkoxy und/oder Cyano substituiert sein kann;
C₅-C₈-Cycloalkyl, das ein- oder mehrfach durch C₁-C₆-Alkyl substituiert sein kann,
wobei die Reste R gleich oder verschieden sein können, wenn sie mehrfach in Formel I auftreten;
- R¹: Wasserstoff oder C₁-C₆-Alkyl,
- R², R³: unabhängig voneinander Wasserstoff;
C₁-C₁₈-Alkyl, das ein- oder mehrfach durch C₁-C₆-Alkoxy, Hydroxy, Halogen und/oder Cyano substituiert sein kann;
Aryl oder Hetaryl, das jeweils ein- oder mehrfach durch C₁-C₆-Alkyl und/oder die vorstehenden, als Substituenten für Alkyl genannten Reste substituiert sein kann;
- n: 1 oder 2;
- m: 0 oder 1, wobei m = n - 1 ist;
- x: für n = 1:
2 bis 4 oder auch 0, wenn die Reste B und die Reste B' jeweils einen Rest der Formel (b) oder ein Rest B und ein Rest B'jeweils einen Rest der Formel (c) bedeuten;
für n = 2:
2 bis 4, wenn die Reste B und die Reste B' jeweils einen Rest der Formel (a) bedeuten;
0 für alle weiteren Bedeutungen der Reste B und B';
- z: 0 bis 4, wobei x + z ≤ 4 ist, wobei für n = 2 gilt: z ≠ 0, wenn x = 0 ist;
- z1: für m = 0: 0;
für m = 1: 0 bis 2.

Bei ganz besonders bevorzugten Mehrfachchromophoren der Formel I' haben die Variablen schließlich folgende Bedeutung:
- Z: Phenoxy, das ein- oder mehrfach durch gleiche oder verschiedene Reste (i), (ii), (iii), (iv) und/oder (v) substituiert sein kann:
(i) C₁-C₁₈-Alkyl, dessen Kohlenstoffkette durch eine oder mehrere Gruppierungen -O-, -S-, -NR¹- und/oder -CO- unterbrochen sein kann und das ein- oder mehrfach durch C₁-C₁₂-Alkoxy, Hydroxy und/oder Halogen substituiert sein kann;
(ii) C₃-C₈-Cycloalkyl, das ein- oder mehrfach durch C₁-C₁₈-Alkyl und/oder C₁-C₁₂-Alkoxy substituiert sein kann;
(iii) Aryl oder Hetaryl, das ein- oder mehrfach durch C₁-C₁₈-Alkyl, C₁-C₁₂-Alkoxy, Hydroxy und/oder Halogen substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S- oder -NR¹- bedeutet;
(v) C₁-C₁₂-Alkoxy, Hydroxy, Halogen oder Cyano;
- B, B': jeweils miteinander verbunden zu einem Rest der Formel (a) oder (b);
- A: ein Arylenrest der Formeln 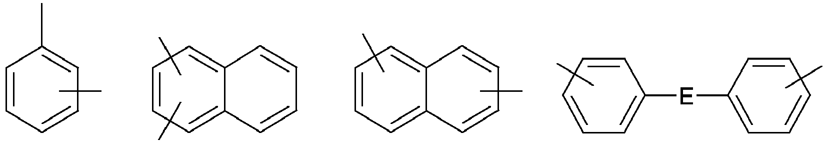 in denen die Phenylen- oder Naphthylenringe ein- oder mehrfach durch C₁-C₁₈-Alkyl substituiert sein können,
wobei die Reste A gleich oder verschieden sein können, wenn sie mehrfach in Formel I auftreten;
- E: eine chemische Bindung, Methylen oder Isopropylen;
- Y, Y¹: -O-;
- R: gleiche Reste:
C₁-C₃₀-Alkyl, dessen Kohlenstoffkette durch eine oder mehrere Gruppierungen -O- und/oder -CO- unterbrochen sein kann und das ein- oder mehrfach substituiert sein kann durch: C₁-C₆-Alkoxy, Cyano und/oder Aryl, das ein- oder mehrfach durch C₁-C₁₈-Alkyl und/oder C₁-C₆-Alkoxy substituiert sein kann;
Phenyl, Naphthyl, Pyridyl oder Pyrimidyl, das jeweils ein- oder mehrfach substituiert sein kann durch: C₁-C₁₈-Alkyl, C₁-C₆-Alkoxy, Halogen, Cyano, Nitro, -CONR²R³, -SO₂NR²R³ und/oder Phenyl- und/oder Naphthylazo, das jeweils ein- oder mehrfach durch C₁-C₁₀-Alkyl, C₁-C₆-Alkoxy und/oder Cyano substituiert sein kann;
C₅-C₈-Cycloalkyl, das ein- oder mehrfach durch C₁-C₆-Alkyl substituiert sein kann;
- R¹: Wasserstoff oder C₁-C₆-Alkyl,
- R², R³: unabhängig voneinander Wasserstoff;
C₁-C₁₈-Alkyl, das ein- oder mehrfach durch C₁-C₆-Alkoxy, Hydroxy, Halogen und/oder Cyano substituiert sein kann;
Aryl oder Hetaryl, das jeweils ein- oder mehrfach durch C₁-C₆-Alkyl und/oder die vorstehenden, als Substituenten für Alkyl genannten Reste substituiert sein kann;
- n: 1 oder 2;
- m: 0 oder 1, wobei m = n - 1 ist;
- x: für n = 1:
2 bis 4 oder auch 0, wenn die Reste B und die Reste B' jeweils einen Rest der Formel (b) bedeuten;
für n = 2:
2 bis 4, wenn die Reste B und die Reste B' jeweils einen Rest der Formel (a) bedeuten;
0, wenn die Reste B und die Reste B' jeweils einen Rest der Formel (b) bedeuten;
- z: 0 bis 4, wobei x + z ≤ 4 ist;
- z1: 0.

Als Beispiele für die in den erfindungsgemäßen Formeln auftretenden Reste R, R', R" und R¹ bis R³ sowie deren Substituenten seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
2-Methoxyethyl, 2-Ethoxyethyl, 2-Propoxyethyl, 2-Isopropoxyethyl, 2-Butoxyethyl, 2-und 3-Methoxypropyl, 2- und 3-Ethoxypropyl, 2- und 3-Propoxypropyl, 2- und 3-Butoxy-propyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxa-octyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxa-tetradecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2-und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
(1-Ethylethyliden)aminoethylen, (1-Ethylethyliden)aminopropylen, (1-Ethylethyliden)-aminobutylen, (1-Ethylethyliden)aminodecylen und (1-Ethylethyliden)aminododecylen;
Propan-2-on-1-yl, Butan-3-on-1-yl, Butan-3-on-2-yl und 2-Ethylpentan-3-on-1-yl;
2-Methylsulfoxidoethyl, 2-Ethylsulfoxidoethyl, 2-Propylsulfoxidoethyl, 2-Isopropylsulfoxidoethyl, 2-Butylsulfoxidoethyl, 2- und 3-Methylsulfoxidopropyl, 2- und 3-Ethylsulfoxidopropyl, 2- und 3-Propylsulfoxidopropyl, 2- und 3-Butylsulfoxidopropyl, 2- und 4-Methylsulfoxidobutyl, 2- und 4-Ethylsulfoxidobutyl, 2- und 4-Propylsulfoxidobutyl und 4-Butylsulfoxidobutyl;
2-Methylsulfonylethyl, 2-Ethylsulfonylethyl, 2-Propylsulfonylethyl, 2-Isopropylsulfonyl-ethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylproypl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxy-tetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2- und 3-Hydroxypropyl, 1-Hydroxyprop-2-yl, 3- und 4-Hydroxybutyl, 1-Hydroxybut-2-yl und 8-Hydroxy-4-oxaoctyl;
2-Cyanoethyl, 3-Cyanopropyl, 3- und 4-Cyanobutyl, 2-Methyl-3-ethyl-3-cyanopropyl, 7-Cyano-7-ethylheptyl und 4-Methyl-7-methyl-7-cyanoheptyl;
2-Chlorethyl, 2- und 3-Chlorpropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Methylthio, Ethylthio, Propylthio, Isopropylthio, Butylthio, Isobutylthio, sec.-Butylthio, tert.-Butylthio, Pentylthio, Isopentylthio, Neopentylthio, tert.-Pentylthio und Hexylthio;
Ethinyl, 1- und 2-Propinyl, 1-, 2- und 3-Butinyl, 1-, 2-, 3- und 4-Pentinyl, 1-, 2-, 3-, 4-und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16-und 17-Octadecinyl;
Ethenyl, 1- und 2-Propenyl, 1-, 2- und 3-Butenyl, 1-, 2-, 3- und 4-Pentenyl, 1-, 2-, 3-, 4-und 5-Hexenyl, 1-, 2-, 3-, 4-, 5-, 6-, 7-, 8- und 9-Decenyl, 1-, 2-, 3-, 4-, 5-, 6-, 7-, 8-, 9-, 10- und 11-Dodecenyl und 1-, 2-, 3-, 4-, 5-, 6-, 7-, 8-, 9-, 10-, 11-, 12-, 13-, 14-, 15-, 16-und 17-Octadecenyl;
Methylamino, Ethylamino, Propylamino, Isopryplamino, Butylamino, Isobutylamino, Pentylamino, Hexylamino, Dimethylamino, Methylethylamino, Diethylamino, Dipropylamino, Diisopropylamino, Dibutylamino, Diisobutylamino, Dipentylamino, Dihexylamino, Dicyclopentylamino, Dicyclohexylamino, Dicycloheptylamino, Diphenylamino und Dibenzylamino;
Formylamino, Acetylamino, Propionylamino und Benzoylamino;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Aminosulfonyl, N,N-Dimethylaminosulfonyl, N,N-Diethylaminosulfonyl, N-Methyl-N-ethylaminosulfonyl, N-Methyl-N-dodecylaminosulfonyl, N-Dodecylaminosulfonyl, (N,N-Dimethylamino)ethylaminosulfonyl, N,N-(Propoxyethyl)dodecylaminosulfonyl, N,N-Diphenylaminosulfonyl, N,N-(4-tert.-Butylphenyl)octadecylaminosulfonyl und N,N-Bis(4-Chlorphenyl)aminosulfonyl;
Methoxycarbonyl, Ethoxycarbonyl, Propoxycarbonyl, Isopropoxycarbonyl, Hexoxycarbonyl, Dodecyloxycarbonyl, Octadecyloxycarbonyl, Phenoxycarbonyl, (4-tert.-Butyl-phenoxy)carbonyl und (4-Chlorphenoxy)carbonyl;
Methoxysulfonyl, Ethoxysulfonyl, Propoxysulfonyl, Isopropoxysulfonyl, Butoxysulfonyl, Isobutoxysulfonyl, tert.-Butoxysulfonyl, Hexoxysulfonyl, Dodecyloxysulfonyl, Octadecyloxysulfonyl, Phenoxysulfonyl, 1- und 2-Naphthyloxysulfonyl, (4-tert.-Butylphenoxy)-sulfonyl und (4-Chlorphenoxy)sulfonyl;
Chlor, Brom und Iod,
Phenylazo, 2-Napthylazo, 2-Pyridylazo und 2-Pyrimidylazo;
Cyclopropyl, Cyclobutyl, Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Iso-propylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl und 3-, 4- und 5-Propylcyclooctyl; 3- und 4-Hydroxycyclohexyl, 3- und 4- Nitrocyclohexyl und 3- und 4-Chlorcyclohexyl;
1-, 2- und 3-Cyclopentenyl, 1-, 2-, 3- und 4-Cyclohexenyl, 1-, 2- und 3-Cycloheptenyl und 1-, 2-, 3- und 4-Cyclooctenyl;
2-Dioxanyl, 1-Morpholinyl, 1-Thiomorpholinyl, 2- und 3-Tetrahydrofuryl, 1-, 2- und 3-Pyrrolidinyl, 1-Piperazyl, 1-Diketopiperazyl und 1-, 2-, 3- und 4-Piperidyl;
Phenyl, 2-Naphthyl, 2- und 3-Pyrryl, 2-, 3- und 4-Pyridyl, 2-, 4- und 5-Pyrimidyl, 3-, 4-und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothia-zolyl, 5-Benzothiadiazolyl, 2- und 5-Benzimidazolyl und 1- und 5-Isochinolyl;
1-, 2-, 3-, 4-, 5-, 6- und 7-Indolyl, 1-, 2-, 3-, 4-, 5-, 6- und 7-Isoindolyl, 5-(4-Methyliso-indolyl), 5-(4-Phenylisoindolyl), 1-, 2-, 4-, 6-, 7- und 8-(1,2,3,4-Tetrahydroisochinolinyl), 3-(5-Phenyl)-(1,2,3,4-tetrahydroisochinolinyl), 5-(3-Dodecyl-(1,2,3,4-tetrahydroiso-chinolinyl), 1-, 2-, 3-, 4-, 5-, 6-, 7- und 8-(1,2,3,4-Tetrahydrochinolinyl) und 2-, 3-, 4-, 5-, 6-, 7- und 8-Chromanyl, 2-, 4- und 7-Chinolinyl, 2-(4-Phenylchinolinyl) und 2-(5-Ethyl-chinolinyl);
2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3-und 4-Butylphenyl, 2,4-, 3,5- und 2,6-Dibutylphenyl, 2,4,6-Tributylphenyl, 2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butylphenyl; 2-, 3- und 4-Methoxyphenyl, 2,4-, 3,5- und 2,6-Dimethoxyphenyl, 2,4,6-Trimethoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3-und 4-Isopropoxyphenyl, 2,4- und 2,6-Diisopropoxyphenyl und 2-, 3- und 4-Butoxy-phenyl; 2-, 3- und 4-Chlorphenyl und 2,4-, 3,5- und 2,6-Dichlorphenyl; 2-, 3- und 4-Hydroxyphenyl und 2,4-, 3,5- und 2,6-Dihydroxyphenyl; 2-, 3- und 4-Cyanophenyl; 3-und 4-Carboxyphenyl; 3- und 4-Carboxamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Buturylaminophenyl; 3- und 4-N-Phenylaminophenyl, 3- und 4-N-(o-Tolyl)aminophenyl, 3- und 4-N-(m-Tolyl)aminophenyl und 3- und 4-(p-Tolyl)aminophenyl; 3- und 4-(2-Pyridyl)aminophenyl, 3- und 4-(3-Pyridyl)aminophenyl, 3- und 4-(4-Pyridyl)aminophenyl, 3- und 4-(2-Pyrimidyl)aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthylazo)phenyl,4-(2-Pyriylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;

Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.

Die besondere Bedeutung der erfindungsgemäßen Mehrfachchromophore I besteht darin, daß sie in technischem Maßstab über wenige Synthesestufen herstellbar sind und gezielt an den jeweiligen Anwendungszweck angepaßt werden können. So kann in einfacher Weise je nach den erforderlichen Absorptions- und Emissionseigenschaften ein entsprechender Chromophor Rylen mit dem jeweils passenden Chromophor X kombiniert werden.

Die Herstellung der erfindungsgemäßen Mehrfachchromophore erfolgt über die Umsetzung entsprechender bekannter Rylenedukte mit den ebenfalls erfindungsgemäßen Rylendicarbonsäureimidderivaten der eingangs definierten Formel III 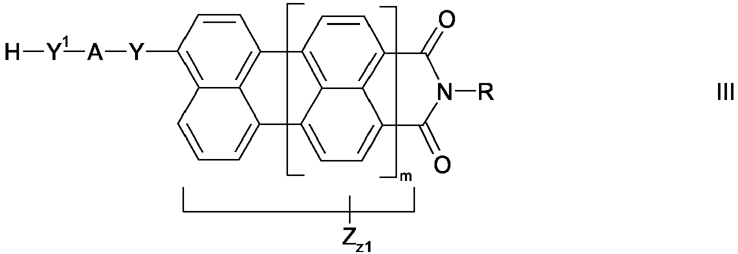 die die Zwischenprodukte der erfindungsgemäßen Mehrfachchromophore I darstellen.

Bevorzugte und besonders bevorzugte Bedeutungen der Variablen Z, A, Y, R und z1 entsprechen dabei den vorstehend genannten bevorzugten und besonders bevorzugten Bedeutungen dieser Variablen in der Formel I'.

Abhängig von der Position, in der der Chromophor X an den Chromophor Rylen gebunden sein soll, werden die Chromophore Rylen und X vorteilhaft durch nucleophile Substitution, Imidierung, Veresterung oder Amidierung miteinander verknüpft.

Diese Reaktionen werden im folgenden gruppenweise näher beschrieben.

Hierbei wird nicht jeweils explizit erwähnt, daß Mischungen der Lösungsmittel oder der weiteren Hilfsstoffe eingesetzt werden können, dies versteht sich jedoch von selbst.

Wenn im folgenden keine näheren Angaben zu den in den Formeln auftretenden Variablen gemacht werden, weisen diese die oben für Formel I' genannte Bedeutung auf.

### Nucleophile Substitution

Mehrfachchromophore I, die an das Kohlenstoffgerüst des Chromophors Rylen gebundene Chromophore X aufweisen, sind vorteilhaft durch Umsetzung der entsprechenden halogenierten (vorzugsweise chlorierten oder bromierten) Rylencarbonsäureimide (II) mit erfindungsgemäßen Rylendicarbonsäureimidderivaten der Formel IIIa 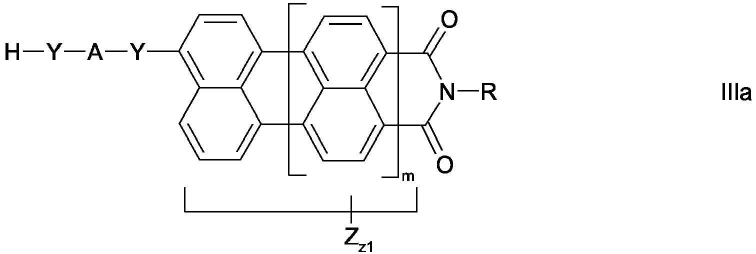 in der Y Sauerstoff oder Schwefel bedeutet, zu erhalten.

Wird der Halogenaustausch nicht quantitativ vorgenommen, so können durch zusätzliche Umsetzung mit einer Verbindung der allgemeinen Formel IV

H-Z IV

auch die (Het)Aryloxy- und (Het)Arylthiosubstituenten Z in das Rylengerüst eingeführt werden.

Die Umsetzungsreihenfolge ist dabei beliebig, d.h., es können a) zunächst die Reste X und dann die Reste Z oder b) zunächst die Reste Z und dann die Reste X oder c) die Reste X und Z auch gleichzeitig eingeführt werden.

Wenn diese Umsetzungen nicht quantitativ erfolgen, können die erhaltenen Mehrfachchromophore noch Spuren Halogen enthalten. Gewünschtenfalls kann in diesen Fällen eine zusätzliche Enthalogenierung, z.B. eine übergangsmetallkatalysierte reduktive oder eine baseninduzierte Enthalogenierung, wie sie in der WO-A-02/76988 beschrieben ist, vorgenommen werden.

Im folgenden werden die ebenfalls einen Gegenstand dieser Erfindung bildenden Herstellungsverfahren zusammengefaßt, die auf dieser erfindungsgemäß in Gegenwart einer Base und eines nichtnucleophilen Lösungsmittels vorgenommenen nucleophilen Substitution basieren.

So wurde ein Verfahren zur Herstellung von Mehrfachchromophoren auf Basis von Rylentetracarbonsäurediimiden der allgemeinen Formel la 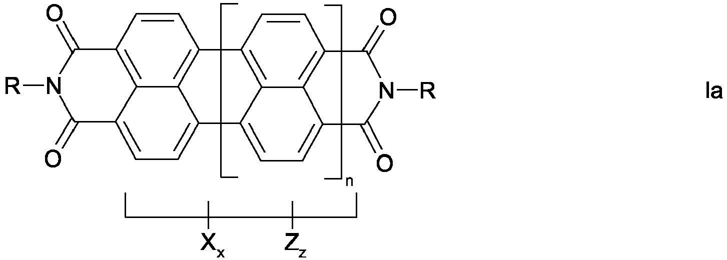 gefunden, welches dadurch gekennzeichnet ist, daß man ein halogeniertes Rylentetracarbonsäurediimid der allgemeinen Formel IIa 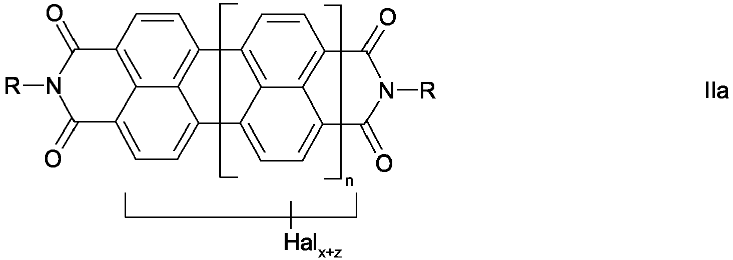 in der Hal Chlor oder Brom bedeutet, in Gegenwart einer Base und eines nichtnucleophilen Lösungsmittels
a) mit 0,8 bis 1,2 mol pro mol auszutauschendes Halogenatom eines Rylendicarbonsäureimidderivats der allgemeinen Formel IIIa 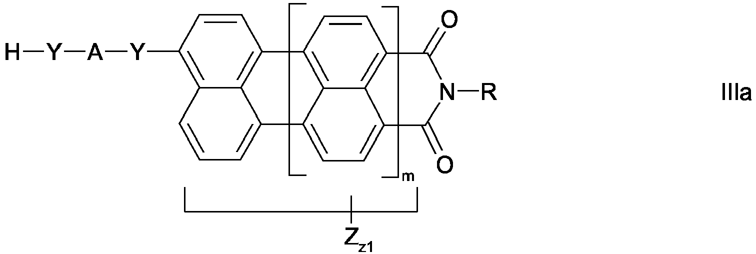 zu einem Rylentetracarbonsäurediimid der Formel la, in der z = 0 ist, umsetzt oder
b) zunächst mit 0,8 bis 1,2 mol pro mol auszutauschendes Halogenatom eines Rylendicarbonsäureimidderivats der Formel IIIa und dann mit 0,8 bis 3 mol pro mol weiteres auszutauschendes Halogenatom einer Verbindung der allgemeinen Formel IV

   H-Z IV

   oder zunächst mit 0,8 bis 1,2 mol pro mol auszutauschendes Halogenatom einer Verbindung der Formel IV und dann mit 0,8 bis 1,2 mol pro mol weiteres auszutauschendes Halogenatom eines Rylendicarbonsäureimidderivats der Formel IIIa
   oder gleichzeitig mit jeweils 0,8 bis 1,2 mol pro mol auszutauschendes Halogenatom des Rylendicarbonsäureimidderivats der Formel IIIa und der Verbindung der Formel IV
   zu einem Rylentetracarbonsäurediimid der Formel Ia, in der z ≠ 0 ist, umsetzt.

Außerdem wurde ein Verfahren zur Herstellung von Mehrfachchromophoren auf Basis von Rylendicarbonsäureimiden der allgemeinen Formel Ib 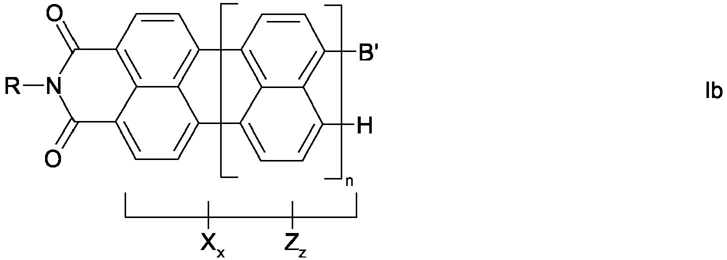 in der n = 1 ist, B' Wasserstoff, einen Rest X oder einen Rest Z bedeutet, gefunden, welches dadurch gekennzeichnet ist, daß man ein halogeniertes Rylendicarbonsäureimid der allgemeinen Formel IIb 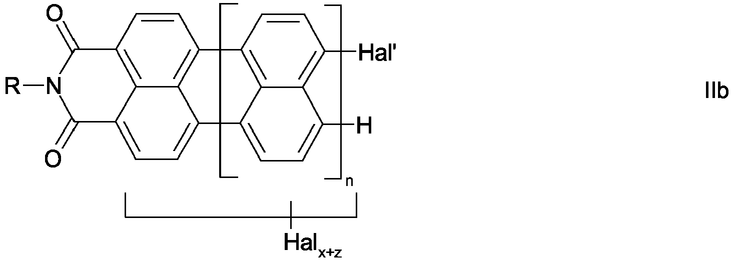 in der n = 1 ist und Hal' Wasserstoff oder Hal bedeutet, wobei Hal für Chlor oder Brom steht, in Gegenwart einer Base und eines nichtnucleophilen Lösungsmittels
a) mit 0,8 bis 1,2 mol pro mol auszutauschendes Halogenatom eines Rylendicarbonsäureimidderivats der allgemeinen Formel IIIa 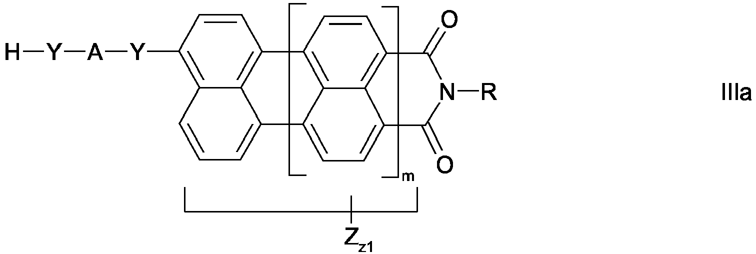 zu einem Rylendicarbonsäureimid der Formel Ib, in der z = 0 ist, umsetzt
   oder
b) zunächst mit 0,8 bis 1,2 mol pro mol auszutauschendes Halogenatom eines Rylendicarbonsäureimidderivats der Formel IIIa und dann mit 0,8 bis 3 mol pro mol weiteres auszutauschendes Halogenatom einer Verbindung der allgemeinen Formel IV

   H-Z IV

   oder zunächst mit 0,8 bis 1,2 mol pro mol auszutauschendes Halogenatom einer Verbindung der Formel IV und dann mit 0,8 bis 1,2 mol pro mol weiteres auszutauschendes Halogenatom eines Rylendicarbonsäureimidderivats der Formel IIIa
   oder gleichzeitig mit jeweils 0,8 bis 1,2 mol pro mol auszutauschendes Halogenatom des Rylendicarbonsäureimidderivats der Formel IIIa und der Verbindung der Formel IV
   zu eimen Rylendicarbonsäureimid der Formel Ib, in der z ≠ 0 ist, umsetzt.

Weiterhin wurde ein Verfahren zur Herstellung vong Mehrfachchromophoren auf Basis von Rylendicarbonsäureimiden der allgemeinen Formel Ib' 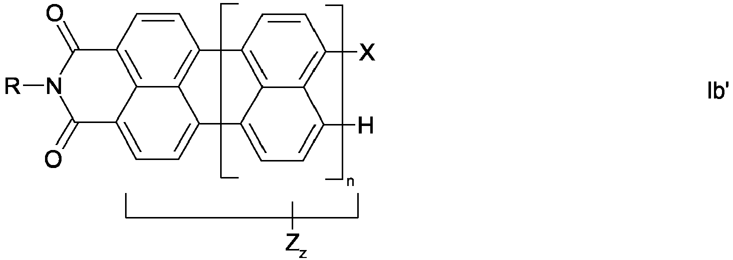 in der n = 2 oder 3 und z = 2 bis 8 ist, gefunden, welches dadurch gekennzeichnet ist, daß man ein peri-Halogenrylendicarbonsäureimid der allgemeinen Formel IIb' 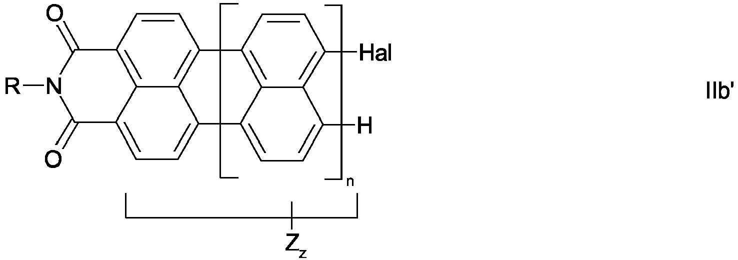 in der n = 2 oder 3 und z = 2 bis 8 ist und Hal Chlor oder Brom bedeutet, in Gegenwart einer Base und eines nichtnucleophilen Lösungsmittels mit 0,8 bis 1,2 mol pro mol eines Rylendicarbonsäureimidderivats der allgemeinen Formel IIIa 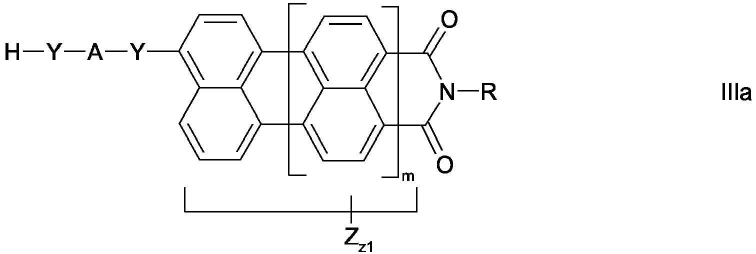 umsetzt.

Da es sich bei den Resten Z vorzugsweise um Phenoxy- oder Thiophenoxyreste handelt, wird die Verbindung der Formel IV der Einfachheit halber in der folgenden genaueren Beschreibung der nucleophilen Substitution an den halogenierten Rylenderivaten II als (Thio)Phenol IV bezeichnet.

Da die nucleophile Substitution durch das Rylendicarbonsäureimidderivat IIIa und durch das (Thio)Phenol IV unter vergleichbaren Reaktionsbedingungen in Gegenwart eines nichtnucleophilen Lösungsmittels und einer Base vorgenommen werden, beziehen sich die folgenden Angaben, soweit nicht anders angegeben, auf beide Umsetzungen.

Als nichtnucleophile Lösungsmittel eignen sich vor allem polare aprotische Lösungsmittel, insbesondere N,N-disubstituierte aliphatische Carbonsäureamide (vorzugsweise N,N-Di(C₁-C₄-alkyl)-(C₁-C₄)carbonsäureamide) und N-Alkyllactame, wie Dimethylformamid, Diethylformamid, Dimethylacetamid, Dimethylbutyramid und N-Methylpyrrolidon, wobei N-Methylpyrrolidon bevorzugt ist.

Als nichtnucleophile Lösungsmittel können auch unpolare aprotische Lösungsmittel eingesetzt werden, diese Lösungsmittel sind jedoch nicht bevorzugt. Beispielhaft seien aromatische Kohlenwasserstoffe, wie Benzol, Toluol und Xylol, genannt.

Die Lösungsmittelmenge hängt von der Löslichkeit der Edukte ab. In der Regel werden 5 bis 200 ml, insbesondere 10 bis 100 ml, Lösungsmittel pro g Rylencarbonsäureimid (II) benötigt.

Als Base eignen sich vor allem anorganische und organische alkali- oder erdalkalimetallhaltige Basen, wobei die alkalimetallhaltigen Basen besonders geeignet sind. Beispiele für anorganische Basen sind Alkali- und Erdalkalimetallcarbonate und -hydrogencarbonate, -hydroxide, -hydride und -amide, Beispiele für organische Basen sind Alkali- und Erdalkalimetallalkoholate (insbesondere die C₁-C₁₀-Alkoholate, vor allem tert.-C₄-C₆-Alkoholate) sowie -(phenyl)alkylamide (insbesondere die Bis(C₁-C₄-alkyl)-amide) und Triphenylmethylmetallate. Bevorzugte Basen sind die Carbonate und Hydrogencarbonate, wobei die Carbonate besonders bevorzugt sind. Bevorzugte Alkalimetalle sind Lithium, Natrium, Kalium und Caesium, besonders geeignete Erdalkalimetalle sind Magnesium und Calcium.

Als Beispiele für die metallhaltigen Basen seien im einzelnen genannt: Lithiumcarbonat, Natriumcarbonat, Kaliumcarbonat und Caesiumcarbonat; Natriumhydrogencarbonat und Kaliumhydrogencarbonat; Lithiumhydroxid, Natriumhydroxid, Kaliumhydroxid und Caesiumhydroxid; Lithiumhydrid, Natriumhydrid und Kaliumhydrid; Lithiumamid, Natriumamid und Kaliumamid; Lithiummethylat, Natriummethylat, Kaliummethylat, Lithiumethylat, Natriumethylat, Kaliumethylat, Natriumisopropylat, Kaliumisopropylat, Natrium-tert.-butylat, Kalium-tert.-butylat, Lithium-(1,1-dimethyl)octylat, Natrium-(1,1-dimethyl)octylat und Kalium-(1,1-dimethyl)octylat; Lithiumdimethylamid, Lithiumdiethylamid, Lithiumdüsopropylamid, Natriumdiisopropylamid, Lithiumhexamethyldisilazid, Natriumhexamethyldisilazid, Kaliumhexamethyldisilazid, Triphenylmethyllithium, Triphenylmethylnatrium und Triphenylmethylkalium.

Neben diesen metallhaltigen Basen eignen sich auch rein organische stickstoffhaltige Basen.

Geeignete Beispiele hierfür sind Alkylamine, insbesondere Tri(C₂-C₆-alkyl)amine, wie Triethylamin, Tripropylamin und Tributylamin, Alkoholamine, insbesondere Mono-, Di- und Tri(C₂-C₄-alkohol)amine, wie Mono-, Di- und Triethanolamin, und heterocyclische Basen, wie Pyridin, 4-(N,N-Dimethylamino)pyridin, (4-Pyrrolidino)pyridin, N-Methylpiperidin, N-Methylpiperidon, N-Methylmorpholin, N-Methyl-2-pyrrolidon, Pyrimidin, Chinolin, Isochinolin, Chinaldin, Diazabicyclooctan (DABCO), Diazabicyclononen (DBN) und Diazabicycloundecen (DBU).

Ganz besonders bevorzugte Basen sind Lithiumcarbonat, Natriumcarbonat, Kaliumcarbonat und Caesiumcarbonat.

In der Regel werden mindestens 0,5 Äquivalente Base pro mol Rylendicarbonsäureimidderivat IIIa bzw. (Thio)Phenol IV benötigt. Besonders geeignete Einsatzmengen liegen für die Carbonate bei 0,5 bis 5, insbesondere 0,5 bis 2, Äquivalenten pro mol IIIa bzw. IV. Bei den rein organischen Basen liegt die Einsatzmenge bevorzugt bei 0,4 bis 10, besonders bevorzugt bei 0,4 bis 3, Äquivalenten pro mol IIIa bzw. IV. Wird die organische Base gleichzeitig als Lösungsmittel eingesetzt, was insbesondere bei den heterocyclischen Basen der Fall sein kann, ist eine mengenmäßige Beschränkung selbstverständlich überflüssig.

Werden die beiden Umsetzungen nacheinander vorgenommen, so kann die insgesamt benötigte Basenmenge direkt vorgelegt werden oder für die zweite Umsetzung nachdosiert werden.

Die Umsetzung kann in Gegenwart von Phasentransferkatalysatoren vorgenommen werden.

Als Phasentransferkatalysatoren sind vor allem quartäre Ammoniumsalze und Phosphoniumsalze, wie Tetra(C₁-Ci₈-alkyl)ammoniumhalogenide und -tetrafluoroborate, Benzyltri(C₁-C₁₈-alkyl)ammoniumhalogenide und -tetrafluoroborate und Tetra(C₁-C₁₈-alkyl)- und Tetraphenylphosphoniumhalogenide, und Kronenether geeignet. Bei den Halogeniden handelt es sich in der Regel um die Fluoride, Chloride, Bromide und lodide, wobei die Chloride und Bromide bevorzugt sind. Besonders geeignete Beispiele sind im einzelnen: Tetraethylammoniumchlorid, Tetrabutylammoniumbromid, Tetrabutylammoniumiodid, Tetrabutylammoniumtetrafluoroborat und Benzyltriethylammmoniumchlorid; Tetrabutylphosphoniumbromid und Tetraphenylphosphoniumchlorid und -bromid; 18-Krone-6, 12-Krone-4 und 15-Krone-5.

Wird ein Phasentransferkatalysator verwendet, so liegt seine Einsatzmenge üblicherweise bei 0,4 bis 10, insbesondere 0,4 bis 3, Äquivalenten pro mol Rylendicarbonsäureimidderivat IIIa bzw. (Thio)Phenol IV.

In der Regel werden je mol auszutauschendes Halogenatom 0,8 bis 1,2 mol Rylendicarbonsäureimidderivat IIIa bzw. (Thio)Phenol IV eingesetzt. Wird die Umsetzung mit dem (Thio)Phenol IV als zweiter Schritt vorgenommen, so können bis zu 3 mol IV je mol auszutauschendes Halogenatom eingesetzt werden, um eine vollständige Umsetzung sicherzustellen.

Die Reaktionstemperatur hängt wiederum von der Reaktivität des Substrats ab und liegt im allgemeinen im Bereich von 30 bis 150°C.

Es ist zweckmäßig, die Umsetzung unter Inertgas, z.B. Stickstoff oder Argon, vorzunehmen.

Die Reaktionszeit beträgt üblicherweise 0,5 bis 96 h, insbesondere 2 bis 72 h.

Verfahrenstechnisch kann man bei der nucleophilen Substitution auf unterschiedliche Weise vorgehen.

Man kann die Reaktionspartner zunächst mischen und dann gemeinsam auf die Reaktionstemperatur erwärmen. Insbesondere zur Erzielung höherer Substitutionsgrade kann es von Vorteil sein, zunächst nur einen Teil des Rylendicarbonsäureimidderivats IIIa bzw. (Thio)Phenols IV und der Base vorzulegen und den Rest, gegebenenfalls nach Zwischenisolierung des in den reaktiveren Positionen umgesetzten Produkts, erst später zuzugeben.

Die Isolierung der erhaltenen Mehrfachchromophore I kann man bei Einsatz anorganischer Basen wie folgt vornehmen:
Man kann die angefallenen anorganischen Salze zunächst abfiltrieren, den Mehrfachchromophor I dann durch Zugabe von niederen aliphatischen Alkoholen, wie Methanol, Ethanol, Isopropanol, Butanol oder Ethylenglykolmonobutylether, von Wasser oder von Wasser/Alkohol-Gemischen oder durch Verdampfen des Lösungsmittels ausfällen und abschließend abfiltrieren.

Man kann die anorganischen Salze jedoch auch zusammen mit dem ausgefällten Mehrfachchromophor I abfiltrieren und durch Waschen mit Wasser und/oder verdünnten anorganischen Säuren, wie Salzsäure oder Schwefelsäure, auswaschen.

Gewünschtenfalls können die erhaltenen Mehrfachchromophore I zur zusätzlichen Reinigung einer Filtration oder Säulenchromatographie an Kieselgel unterzogen werden. Als Eluens eignen sich dabei insbesondere halogenierte aliphatische Kohlenwasserstoffe, wie Methylenchlorid und Chloroform, aliphatische und aromatische Kohlenwasserstoffe, wie Cyclohexan, Petrolether, Benzol, Toluol und Xylol, aliphatische Alkohole, wie Methanol und Ethanol, und aliphatische Carbonsäureester, wie Essigsäureethylester, die vorzugsweise in Form von Mischungen eingesetzt werden.

Die erhaltenen Mehrfachchromophore I können gewünschtenfalls auch durch Umkristallisation bzw. fraktionierte Kristallisation gereinigt werden. Hierfür sind neben den oben genannten Lösungsmitteln auch N,N-disubstituierte aliphatische Carbonsäureamide, wie Dimethylformamid und Dimethylacetamid, und N-Alkyllactame, wie N-Methylpyrrolidon, sowie Schwefelsäure geeignet. Bevorzugte Lösungsmittel sind die halogenierten Kohlenwasserstoffe, die Carbonsäureamide und die N-Alkyllactame und deren Mischungen mit Alkoholen und/oder Wasser.

### Imidierung

Mehrfachchromophore I, bei denen die Chromophore X über die Imidgruppen des Chromophors Rylen gebunden sind, können vorteilhaft durch Umsetzung der entsprechenden Rylencarbonsäureanhydride (II) mit erfindungsgemäßen Rylendicarbonsäureimidderivaten der Formel IIIc 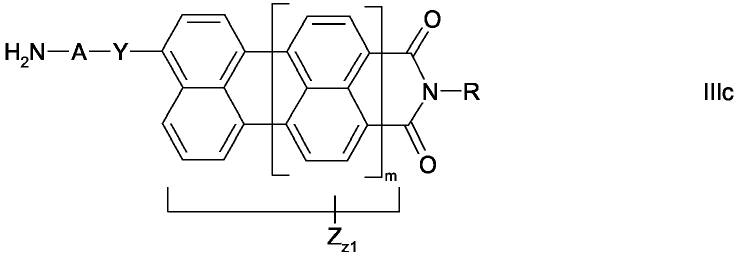 erhalten werden.

Sind die eingesetzten Rylencarbonsäureanhydride (II) im Rylengerüst halogeniert, was bei den Perylentetracarbonsäuredianhydriden und -dicarbonsäureanhydriden der Fall sein kann, so können durch die oben beschriebene nucleophile Substitution zusätzlich Chromophore X und/oder Substituenten Z in das Gerüst des Chromophors Rylen eingeführt werden.

Im folgenden werden die ebenfalls den Gegenstand dieser Erfindung bildenden Verfahren zur Herstellung erfindungsgemäßer Mehrfachchromophore zusammengefaßt, die auf dieser erfindungsgemäß in Gegenwart eines hochsiedenden Lösungsmittels und gewünschtenfalls einer Lewis-Säure vorgenommenen Imidierungsreaktion basieren.

So wurde ein Verfahren zur Herstellung von Mehrfachchromophoren auf Basis von Rylentetracarbonsäurediimiden der allgemeinen Formel Ic 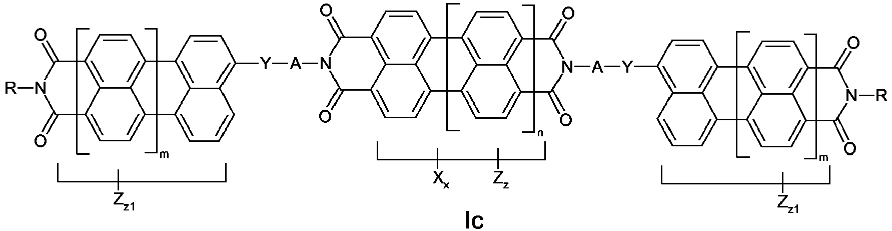 in der n = 1 ist, x 0 oder 2 bis 4 bedeutet, gefunden, welches dadurch gekennzeichnet ist, daß man ein gegebenenfalls halogeniertes Rylentetracarbonsäuredianhydrid der allgemeinen Formel IIc 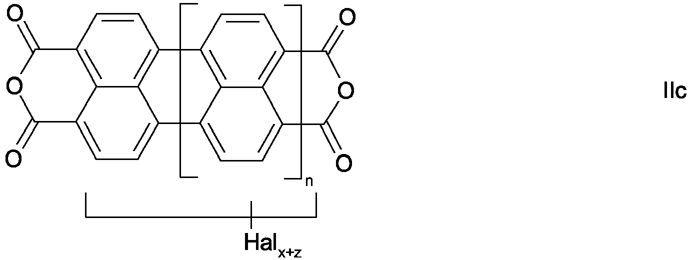 in der n = 1 ist und Hal Chlor oder Brom bedeutet, in Gegenwart eines hochsiedenden Lösungsmittels und gewünschtenfalls einer Lewis-Säure mit 1,8 bis 3 mol pro mol eines Rylendicarbonsäureimidderivats der allgemeinen Formel IIIc 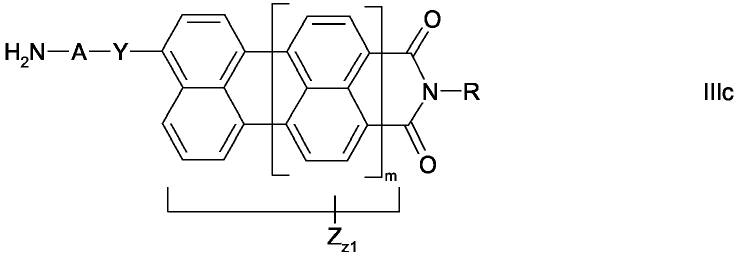 zu einem Rylentetracarbonsäurediimid der Formel Ic, in der x = z = 0 ist, umsetzt und gewünschtenfalls das erhaltene im Rylenkern halogenierte Rylentetracarbonsäurediimid Ic nach Zwischenisolierung in Gegenwart einer Base und eines nichtnucleophilen Lösungsmittels
a) mit 0,8 bis 1,2 mol pro mol auszutauschendes Halogenatom eines Rylendicarbonsäureimidderivats der allgemeinen Formel IIIa 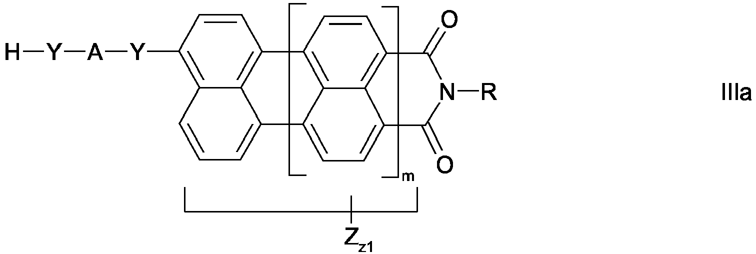 zu einem Rylentetracarbonsäurediimid der Formel Ic, in der x ≠ 0 und z = 0 ist, umsetzt
   oder
b) mit 0,8 bis 3 mol pro mol auszutauschendes Halogenatom einer Verbindung der allgemeinen Formel IV

   H-Z IV

   zu einem Rylentetracarbonsäurediimid der Formel Ic, in der z ≠ 0 und x = 0 ist, umsetzt
   oder
c) zunächst mit 0,8 bis 1,2 mol pro mol auszutauschendes Halogenatom eines Rylendicarbonsäureimidderivats der Formel IIIa und dann mit 0,8 bis 3 mol pro mol weiteres auszutauschendes Halogenatom einer Verbindung der Formel IV
   oder zunächst mit 0,8 bis 1,2 mol pro mol auszutauschendes Halogenatom einer Verbindung der Formel IV und dann mit 0,8 bis 1,2 mol pro mol weiteres auszutauschendes Halogenatom eines Rylendicarbonsäureimidderivats der Formel IIIa
   oder gleichzeitig mit jeweils 0,8 bis 1,2 mol pro mol auszutauschendes Halogenatom des Rylendicarbonsäureimidderivats der Formel IIIa und der Verbindung der Formel IV
   zu einem Rylentetracarbonsäurediimid der Formel Ic, in der x ≠ 0 und z ≠ 0 ist, umsetzt.

Weiterhin wurde ein Verfahren zur Herstellung von Mehrfachchromophoren auf Basis von Rylendicarbonsäureimiden der allgemeinen Formel Id 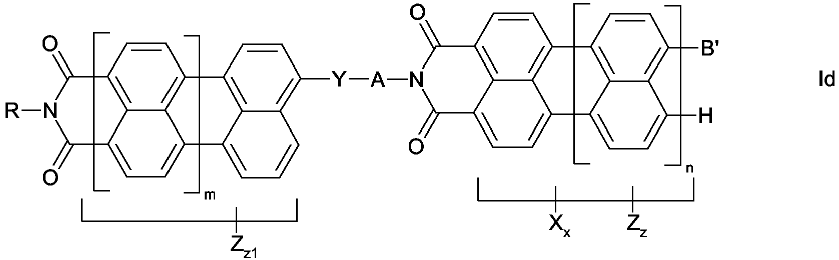 in der n = 1 ist, B' Wasserstoff, einen Rest X oder einen Rest Z bedeutet und x für 0 oder 2 bis 4 steht, gefunden, welches dadurch gekennzeichnet ist, daß man ein gegebenenfalls halogeniertes Rylendicarbonsäureanhydrid der allgemeinen Formel IId 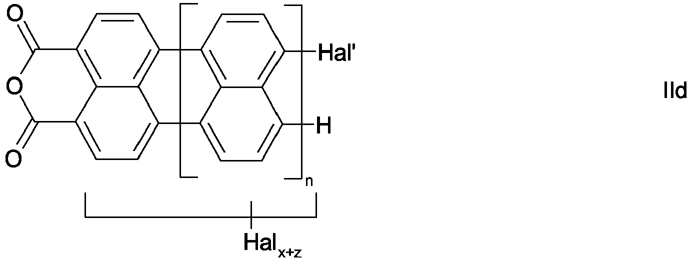 in der n = 1 ist und Hal' Wasserstoff oder Hal bedeutet, wobei Hal für Chlor oder Brom steht, in Gegenwart eines hochsiedenden Lösungsmittels und gewünschtenfalls einer Lewis-Säure mit 0,8 bis 1,2 mol pro mol eines Rylendicarbonsäureimidderivats der allgemeinen Formel IIIc 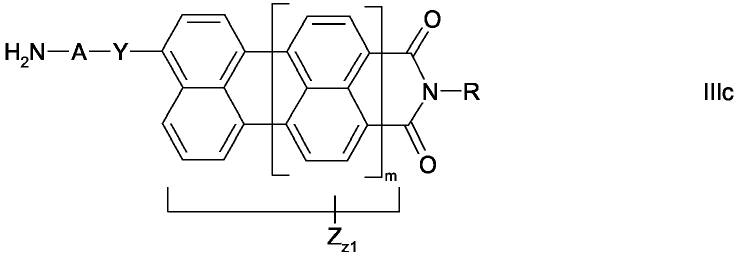 zu einem Rylendicarbonsäureimid der Formel Id, in der x = z = 0 ist, umsetzt
und gewünschtenfalls das erhaltene im Rylenkern halogenierte Rylendicarbonsäureimid Id nach Zwischenisolierung in Gegenwart einer Base und eines nichtnucleophilen Lösungsmittels
a) mit 0,8 bis 1,2 mol pro mol auszutauschendes Halogenatom eines Rylendicarbonsäureimidderivats der allgemeinen Formel IIIa 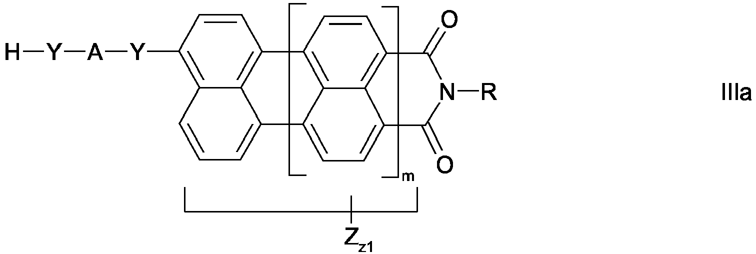 zu einem Rylendicarbonsäureimid der Formel Id, in der x ≠ 0 und z = 0 ist, umsetzt
   oder
b) mit 0,8 bis 3 mol pro mol auszutauschendes Halogenatom einer Verbindung der allgemeinen Formel IV

   H-Z IV

   zu einem Rylendicarbonsäureimid der Formel Id, in der z ≠ 0 und x = 0 ist, umsetzt
   oder
c) zunächst mit 0,8 bis 1,2 mol pro mol auszutauschendes Halogenatom eines Rylendicarbonsäureimidderivats der Formel IIIa und dann mit 0,8 bis 3 mol pro mol weiteres auszutauschendes Halogenatom einer Verbindung der Formel IV
   oder zunächst mit 0,8 bis 1,2 mol pro mol auszutauschendes Halogenatom einer Verbindung der Formel IV und dann mit 0,8 bis 1,2 mol pro mol weiteres auszutauschendes Halogenatom eines Rylendicarbonsäureimidderivats der Formel IIIa oder gleichzeitig mit jeweils 0,8 bis 1,2 mol pro mol auszutauschendes Halogenatom des Rylendicarbonsäureimidderivats der Formel IIIa und der Verbindung der Formel IV
   zu einem Rylendicarbonsäureimid der Formel Id, in der x ≠ 0 und z ≠ 0 ist, umsetzt.

Auch wurde ein Verfahren zur Herstellung von Herstellung von Mehrfachchromophoren auf Basis von Rylentetracarbonsäurediimiden der allgemeinen Formel le 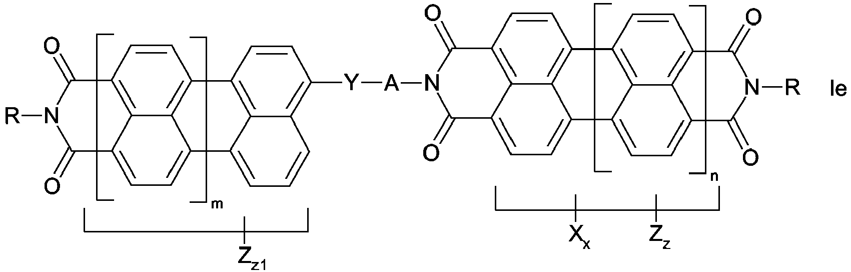 in der n = 1 ist, gefunden, welches dadurch gekennzeichnet ist, daß man ein gegebenenfalls halogeniertes Rylentetracarbonsäuremonoanhydridmonoimid der allgemeinen Formel IIe 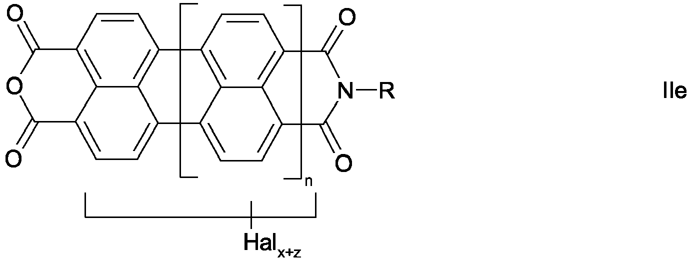 in der n = 1 ist und Hal Chlor oder Brom bedeutet, in Gegenwart eines hochsiedenden Lösungsmittels und gewünschtenfalls einer Lewis-Säure mit 0,8 bis 1,2 mol pro mol eines Rylendicarbonsäureimidderivats der allgemeinen Formel IIIc 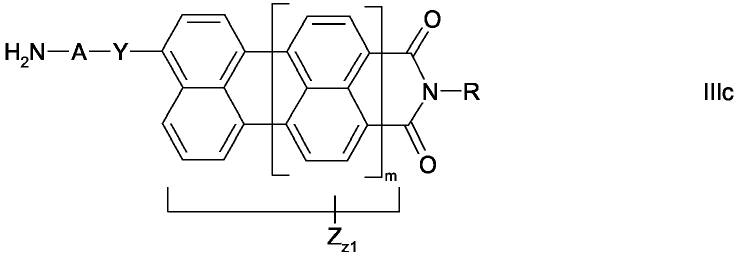 zu einem Rylentetracarbonsäurediimid der Formel le, in der x = z = 0 ist, umsetzt
und gegebenenfalls das erhaltene im Rylenkern halogenierte Rylendicarbonsäureimid le nach Zwischenisolierung in Gegenwart einer Base und eines nichtnucleophilen Lösungsmittels
a) mit 0,8 bis 1,2 mol pro mol auszutauschendes Halogenatom eines Rylendicarbonsäureimidderivats der allgemeinen Formel IIIa 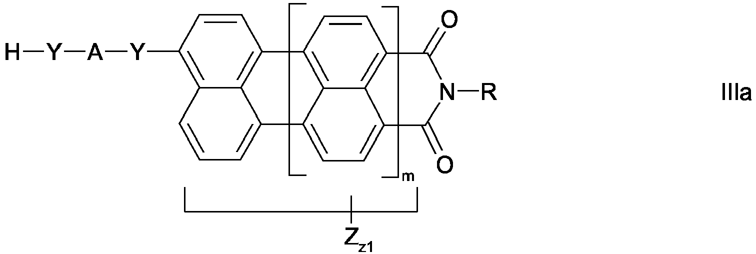 zu einem Rylentetracarbonsäurediimid der Formel le, in der x ≠ 0 und z = 0 ist, umsetzt
   oder
b) mit 0,8 bis 3 mol pro mol auszutauschendes Halogenatom einer Verbindung der allgemeinen Formel IV

   H-Z IV

   zu einem Rylentetracarbonsäurediimid der Formel le, in der z ≠ 0 und x = 0 ist, umsetzt
   oder
c) zunächst mit 0,8 bis 1,2 mol pro mol auszutauschendes Halogenatom eines Rylendicarbonsäureimidderivats der Formel IIIa und dann mit 0,8 bis 3 mol pro mol weiteres auszutauschendes Halogenatom einer Verbindung der Formel IV
   oder zunächst mit 0,8 bis 1,2 mol pro mol auszutauschendes Halogenatom einer Verbindung der Formel IV und dann mit 0,8 bis 1,2 mol pro mol weiteres auszutauschendes Halogenatom eines Rylendicarbonsäureimidderivats der Formel IIIa
   oder gleichzeitig mit jeweils 0,8 bis 1,2 mol pro mol auszutauschendes Halogenatom des Rylendicarbonsäureimidderivats der Formel IIIa und der Verbindung der Formel IV
   zu einem Rylentetracarbonsäurediimid der Formel Ie, in der x ≠ 0 und z ≠ 0 ist, umsetzt.

Außerdem wurde ein Verfahren zur Herstellung von Mehrfachchromophoren auf Basis von Rylentetracarbonsäurediimiden der allgemeinen Formel Ic' 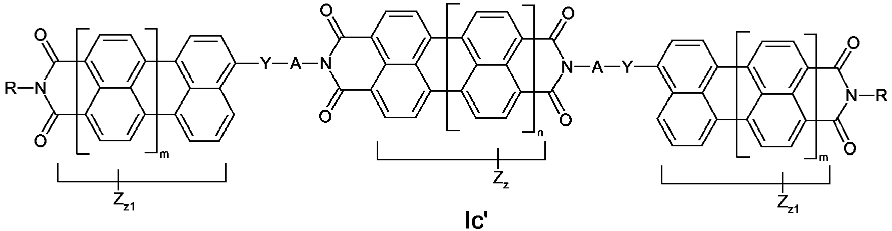 in der n = 2 oder 3 und z = 2 bis 8 ist, gefunden, welches dadurch gekennzeichnet ist, daß man ein Rylentetracarbonsäuredianhydrid der allgemeinen Formel IIc' 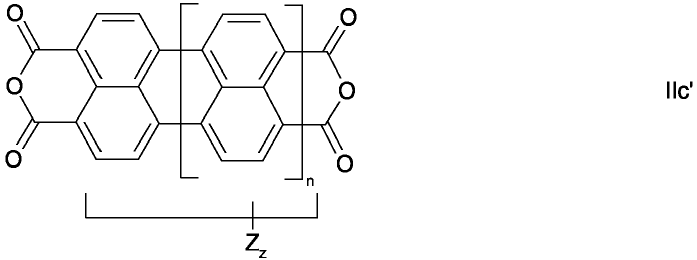 in der n = 2 oder 3 und z = 2 bis 8 ist, in Gegenwart eines hochsiedenden Lösungsmittels und einer Lewis-Säure mit 0,8 bis 1,2 mol pro mol eines Rylendicarbonsäureimidderivats der allgemeinen Formel IIIc 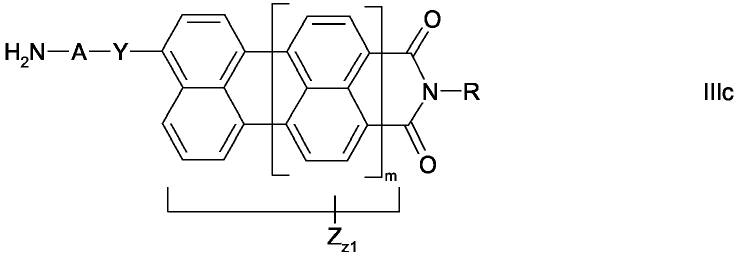 umsetzt.

Schließlich wurde ein Verfahren zur Herstellung von Mehrfachchromophoren auf Basis von Rylendicarbonsäureimiden der allgemeinen Formel Id' 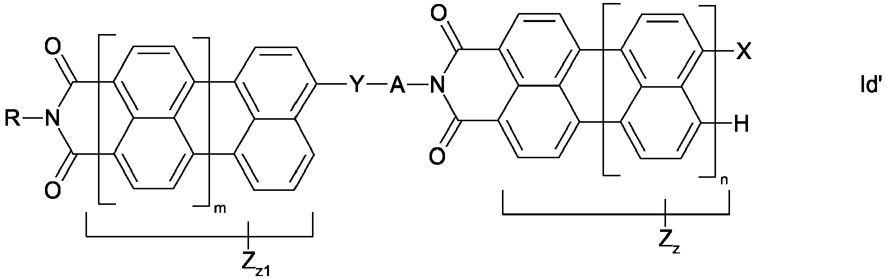 in der n = 2 oder 3 und z = 2 bis 8 ist, gefunden, welches dadurch gekennzeichnet ist, daß man ein peri-Halogenrylendicarbonsäureanhydrid der allgemeinen Formel Id' 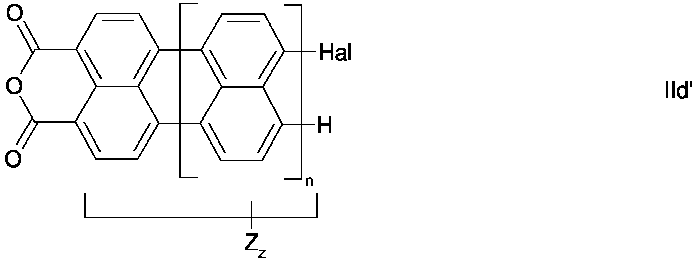 in der n = 2 oder 3 und z = 2 bis 8 ist und Hal Chlor oder Brom bedeutet, in Gegenwart eines hochsiedenden Lösungsmittels und einer Lewis-Säure mit 0,8 bis 1,2 mol pro mol eines Rylendicarbonsäureimidderivats der allgemeinen Formel IIIc 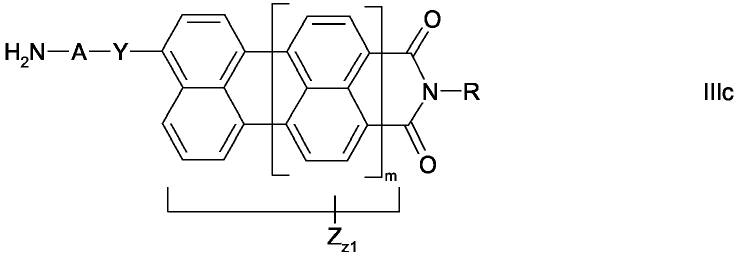 umsetzt.

Nicht zuletzt wurde ein Verfahren zur Herstellung von Mehrfachchromophoren auf Basis von Rylentetracarbonsäurediimiden der allgemeinen Formel le' 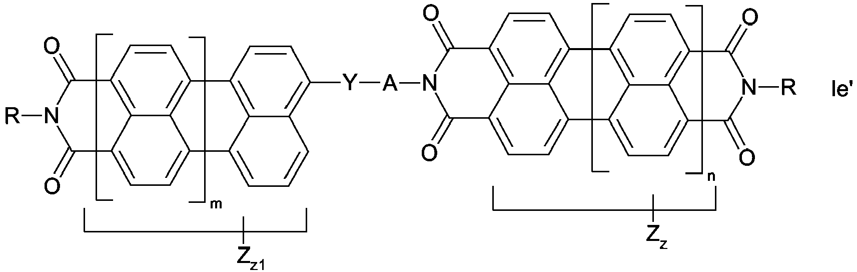 in der n = 2 oder 3 und z = 2 bis 8 ist, gefunden, welches dadurch gekennzeichnet ist, daß man ein Rylentetracarbonsäuremonoanhydridmonoimid der allgemeinen Formel IIe' 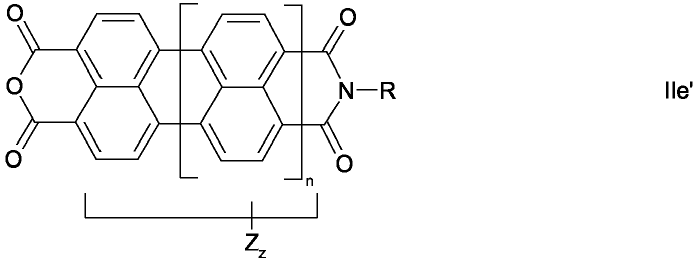 in der n = 2 bis 8 ist, in Gegenwart eines hochsiedenden Lösungsmittels und einer Lewis-Säure mit 0,8 bis 1,2 mol pro mol eines Rylendicarbonsäureimidderivats der allgemeinen Formel IIIc 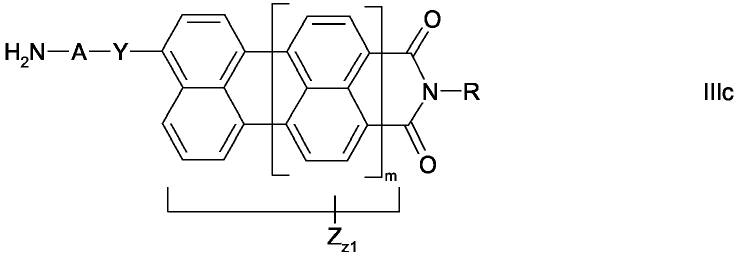 zu einem Rylentetracarbonsäurediimid der Formel le' umsetzt.

Die Imidierungsreaktion zwischen dem entsprechenden Rylencarbonsäureanhydrid (II) und dem aminofunktionalisierten Rylendicarbonsäureimidderivat IIIc wird erfindungsgemäß in Gegenwart eines hochsiedenden Lösungsmittels vorgenommen.

Als Lösungsmittel sind dabei polare aprotische Lösungsmittel, wie Trialkylamine, stickstoffhaltige Heterocyclen, N,N-disubstituierte aliphatische Carbonsäureamide und N-Alkyllactame, geeignet.

Als Beispiele für besonders geeignete Lösungsmittel seien im einzelnen genannt: Triethylamin, Tripropylamin und Tributylamin; Chinolin, Isochinolin, Chinaldin, Pyrimidin, N-Methylpiperidin und Pyridin; Dimethylformamid, Diethylformamid, Dimethylacetamid und Dimethylbutyramid; N-Methylpyrrolidon. Bevorzugtes Lösungsmittel dieser Gruppe ist Chinolin.

Als Lösungsmittel eignen sich auch protische Lösungsmittel, insbesondere aliphatische Carbonsäuren, vorzugsweise C₂-C₁₂-Carbonsäuren, wie Essigsäure, Propionsäure, Butansäure und Hexansäure, wobei Essigsäure und Propionsäure bevorzugte protische Lösungsmittel sind.

Je nach Reaktivität der Edukte sind entweder die aprotischen oder die protischen Lösungsmittel bevorzugt. So sind die aprotischen Lösungsmittel für die Umsetzung nichthalogenierter Rylencarbonsäureanhydride (II) vorzuziehen, während die protischen Lösungsmittel bei der Umsetzung der reaktiveren halogenierten Rylencarbonsäureanhydride II bevorzugt sind.

In der Regel werden 1 bis 100 ml, insbesondere 3 bis 70 ml, Lösungsmittel pro g Rylencarbonsäureanhydrid (II), verwendet.

Bei der Imidierung kann auch eine Lewis-Säure als Katalysator eingesetzt werden.

Der Einsatz einer Lewis-Säure ist insbesondere bei der Umsetzung der reaktionsträgeren nichthalogenierten Rylencarbonsäureanhydride (II) und/oder reaktionsträgerem Rylendicarbonsäureimidderivat IIIc zu empfehlen.

Als Lewis-Säure eignen sich vor allem Zink-, Kupfer- und Eisensalze, wobei im Fall von Kupfer auch die Oxide verwendet werden können. Bevorzugt sind die Zink- und Kupferverbindungen, wobei die Zinkverbindungen besonders bevorzugt sind.

Beispiele für geeignete Lewis-Säuren sind Zinkacetat, Zinkpropionat, Kupfer(I)oxid, Kupfer(II)oxid, Kupfer(I)chlorid, Kupfer(I)acetat und Eisen(III)chlorid, wobei Zinkacetat ganz besonders bevorzugt ist.

Kommt eine Lewis-Säure zum Einsatz, so werden in der Regel 0,5 bis 3, vorzugsweise 0,5 bis 1,5, Äquivalente pro mol umzusetzender Anhydridgruppe im Rylencarbonsäureanhydrid (II) verwendet.

Die Reaktionstemperatur hängt ebenfalls von der Reaktivität der Edukte ab und liegt im allgemeinen im Bereich von 100 bis 250°C. Bei den reaktionsträgeren nichthalogenierten Rylencarbonsäureanhydriden (II) sind Temperaturen von 150 bis 230°C bevorzugt, die Umsetzung der reaktiveren halogenierten Rylencarbonsäureanhydride (II) wird vorzugsweise bei 110 bis 170°C vorgenommen.

Gewünschtenfalls kann man das sich bildende Reaktionswasser sowie das gegebenenfalls durch die Hilfsstoffe eingebrachte Wasser während der Umsetzung abdestillieren.

Es empfiehlt sich, unter Schutzgas, z.B. Stickstoff oder Argon, zu arbeiten.

Die Reaktionszeit beträgt üblicherweise 1 bis 48 h, insbesondere 6 bis 18 h.

Verfahrenstechnisch geht man bei der Imidierung zweckmäßigerweise wie folgt vor:
Man erhitzt eine Mischung von Rylencarbonsäureanhydrid (II), Rylendicarbonsäureimidderivat IIIc, Lösungsmittel und gegebenenfalls Lewis-Säure gewünschtenfalls unter Abdestillieren des sich bildenden Wassers 1 bis 48 h auf die gewünschte Reaktionstemperatur. Wird bei Abdestillieren des Wassers zu viel Lösungsmittel mitabdestilliert, so muß eine entsprechende weitere Menge ergänzt werden.

Die Isolierung der erhaltenen Mehrfachchromophore I kann wie folgt vorgenommen werden:
Die Mehrfachchromophore I werden durch Abkühlen und Zugabe eines protischen Lösungsmittels, wie Wasser oder eines niederen aliphatischen Alkohols, ausgefällt bzw. auskristallisiert, abfiltriert, mit einem der vorstehenden Lösungsmittel und gegebenenfalls einer verdünnten Mineralsäure zur Entfernung von Rückstanden von Rylendicarbonsäureimidderivat IIIc und/oder anorganischen Salzen gewaschen und getrocknet.

Wie bei der nucleophilen Substitution beschrieben, können die erhaltenen Mehrfachchromophore I gewünschtenfalls zur weiteren Reinigung einer Säulenchromatographie bzw. Säulenfiltration oder einer Umkristallisation bzw. fraktionierten Kristallisation unterzogen werden.

### Veresterung

Mehrfachchromophore I, bei denen die Chromophore X über Estergruppen an den Chromophor Rylen gebunden sind, können vorteilhaft ausgehend von den entsprechenden Rylendicarbonsäurechloriden IIf erhalten werden. Bei dem Chromophor Rylen handelt es sich hier insbesondere um Perylen.

Weiterer Gegenstand dieser Erfindung ist demgemäß ein Verfahren zur Herstellung von Mehrfachchromophoren auf Basis von Rylendicarbonsäureestern der allgemeinen Formel If 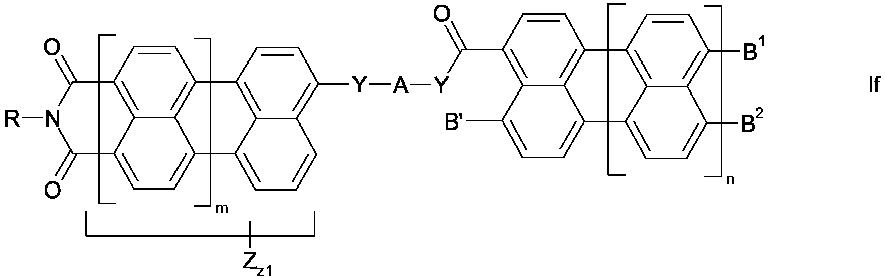 in der n = 1 ist, B' Wasserstoff, Halogen oder Cyano, einer der beiden Reste B¹ oder B² einen Rest der Formel 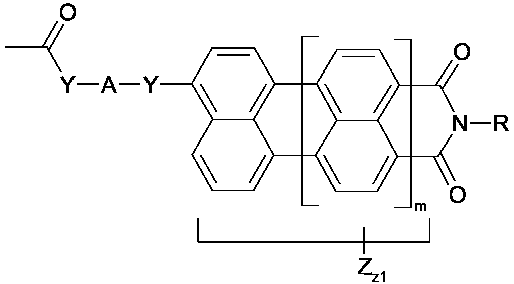 und der andere Rest Wasserstoff, Halogen oder Cyano bedeutet, welches dadurch gekennzeichnet ist, daß man ein Rylendicarbonsäureimidderivat der allgemeinen Formel IIIa 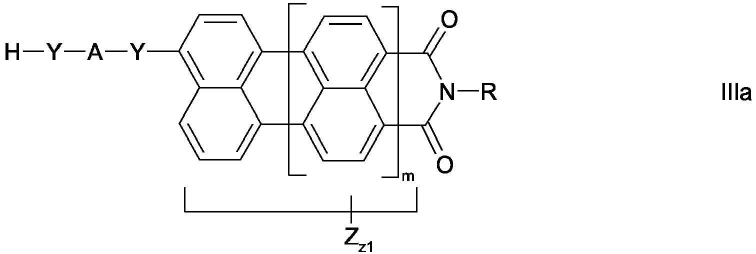 in Gegenwart einer Alkalimetallbase und eines aprotischen Lösungsmittels in das Alkalimetallsalz der allgemeinen Formel IIIf 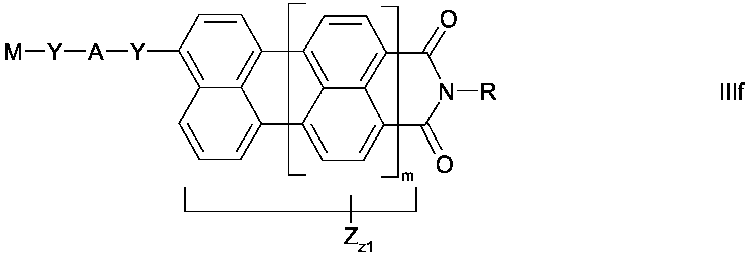 in der M ein Alkalimetallkation bedeutet, überführt und dieses dann mit einem Rylendicarbonsäurechlorid der allgemeinen Formel IIf 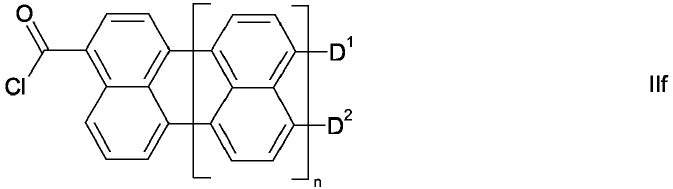 in der n = 1 ist und einer der beiden Reste D¹ oder D² -COCl oder der andere Rest Wasserstoff bedeutet, im Molverhältnis 0,8 : 1 bis 1,2 : 1 zu einem Rylendicarbonsäureester der Formel If, in der B' und der verbleibende Reste B¹ oder B² Wasserstoff bedeuten, umsetzt
und gewünschtenfalls den erhaltenen Rylendicarbonsäureester nach Zwischenisolierung in Gegenwart eines polaren organischen Lösungsmittels und einer Lewis-Säure mit N-Halogensuccinimid zum Rylendicarbonsäureester der Formel If, in der B' und der verbleibende Rest B¹ oder B² Halogen bedeuten, halogeniert
und gewünschtenfalls den erhaltenen halogenierten Rylendicarbonsäureester mit einem Metallcyanid in Gegenwart eines aprotischen Lösungsmittels und eines Übergangsmetallkatalysators in den entsprechenden cyanosubstituierten Rylendicarbonsäureester der Formel If, in der B' und der verbleibende Rest B¹ oder B² Cyano bedeuten, überführt.

Da die für die Veresterung als Edukt eingesetzten Perylendicarbonsäurechloride IIf üblicherweise in Form von Mischungen des 3,9- und 3,10-Isomers vorliegen, werden auch die Mehrfachchromophore If als entsprechende Isomerenmischung erhalten.

Die Veresterung wird in Gegenwart eines aprotischen Lösungsmittels und einer Alkalimetallbase vorgenommen.

Als aprotisches Lösungsmittel eignen sich vor allem polare aprotische Lösungsmittel, wie aprotische Ether und die vorstehend genannten N,N-disubstituierten aliphatischen Carbonsäureamide und N-Alkyllactame.

Beispiele für die bevorzugten Ether sind: Dialkylether, die zwei gleiche oder verschiedene Alkylreste aufweisen können und in der Regel 3 bis 8 C-Atome aufweisen, und Diarylether; Dialkylether, insbesondere Di(C₁-C₆-alkyl)ether und Diarylether (vor allem Diphenylether) von monomeren und oligomeren C₂-C₃-Alkylenglykolen, die bis zu sechs Alkylenoxideinheiten enthalten können, wobei Diethylenglykoldi(C₁-C₄-alkyl)-ether besonders bevorzugt sind, und cyclische Ether.

Als Beispiele seien im einzelnen genannt: tert.-Butylmethylether; Diphenylether; Diethylenglykoldimethyl-, -diethyl-, -dipropyl-, -diisopropyl-, -dibutyl-, -di-sec.-butyl- und -di-tert.-butylether, Diethylenglykolmethylethylether, Triethylenglykoldimethyl- und -diethylether und Triethylenglykolmethylethylether; Tetrahydrofuran und Dioxan. Ganz besonders bevorzugt sind Diethylenglykoldimethyl- und -diethylether.

Weiterhin eignen sich auch unpolare aprotische Lösungsmittel, wie Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl- und cycloalkylsubstituierte Aromaten (insbesondere Benzol, das durch ein bis drei C₁-C₆-Alkylgruppen oder einen C₅-C₈-Cycloalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist).

Als Beispiele seien im einzelnen genannt: Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methylcycloheptan und Methylcyclooctan; Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin und 1- und 2-Ethylnaphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exxsol^{®} Typ und Alkylbenzolgemische vom Solvesso^{®} Typ.

In der Regel werden 2 bis 75 ml, insbesondere 3 bis 50 ml, Lösungsmittel pro g Rylendicarbonsäureimidderivat IIIa eingesetzt.

Als Alkalimetallbase eignen sich vor allem Alkalimetallhydride, insbesondere Lithiumhydrid, Natriumhydrid und Kaliumhydrid.

Im allgemeinen werden 0,8 bis 1,2 mol, vorzugsweise 0,9 bis 1,1 mol, Alkalimetallbase pro mol Rylendicarbonsäureimidderivat IIIa verwendet.

In der Regel werden Rylendicarbonsäureimidderivat IIIf und Rylendicarbonsäurechlorid IIf im Molverhältnis 0,8 : 1 bis 1,2 : 1 umgesetzt.

Die Reaktionstemperatur liegt üblicherweise bei 0 bis 75°C, vor allem bei 20 bis 50°C.

Es empfiehlt sich, die Veresterung unter wasserfreien Bedingungen vorzunehmen.

Die Reaktionsdauer beträgt in der Regel 1 bis 48 h, bevorzugt 3 bis 10 h.

Verfahrenstechnisch geht man bei der Veresterung zweckmäßigerweise wie folgt vor:
Man legt Lösungsmittel, Alkalimetallbase und Rylendicarbonsäureimidderivat IIIa vor, erhitzt gegebenenfalls auf die gewünschte Reaktionstemperatur und überführt das Rylendicarbonsäureimidderivat IIIa auf diese Weise in sein Salz IIIf. Dann gibt man das Rylendicarbonsäurechlorid IIf zu und rührt die Mischung 3 bis 10 h bei der gewünschten Reaktionstemperatur.

Die Isolierung der Mehrfachchromophore If kann durch Verdünnen mit protischen Lösungsmitteln, wie Wasser und niederen aliphatischen Alkoholen, Abfiltrieren, wiederholtes Waschen mit einer wäßrigen Base, z.B. einer Alkalimetallhydrogencarbonatlösung, und abschließend mit Wasser und anschließendes Trocknen erfolgen.

Wie bei der nucleophilen Substitution beschrieben, können die erhaltenen Mehrfachchromophore If gewünschtenfalls zur weiteren Reinigung einer Säulenchromatographie bzw. Säulenfiltration oder einer Umkristallisation bzw. fraktionierten Kristallisation, wobei Schwefelsäure als Lösungsmittel ausgeschlossen ist, unterzogen werden.

Sollen Mehrfachchromophore If, die einen dihalogenierten, insbesondere dichlorierten oder dibromierten, Chromophor Rylen aufweisen (Formel If: B' = verbleibender Rest B¹ oder B² = Halogen), hergestellt werden, so kann man den bei der Veresterung erhaltenen Mehrfachchromophor If in Gegenwart eines polaren organischen Lösungsmittels und einer Lewis-Säure mit N-Halogensuccinimid umsetzen.

Als polare organische Lösungsmittel eignen sich insbesondere aprotische Lösungsmittel. Bevorzugte Beispiele für diese Lösungsmittel sind die vorstehend genannten N,N-disubstituierten aliphatischen Carbonsäureamide und halogenierte Kohlenwasserstoffe, wie Chloroform und Methylenchlorid. Besonders bevorzugt ist Dimethylformamid.

In der Regel kommen 10 bis 200 ml, vorzugsweise 10 bis 100 ml, Lösungsmittel pro g zu halogenierender Mehrfachchromophor If zum Einsatz.

Als Lewis-Säuren eignen sich vor allem Metallhalogenide, wobei Eisen(III)halogenide, Aluminiumtrihalogenide und Zinkhalogenide bevorzugt sind. Als Beispiele im einzelnen genannt seien Eisen(III)chlorid, Eisen(III)bromid, Eisen(III)iodid, Aluminiumtrichlorid, Aluminiumtribromid, Aluminiumtriiodid und Zinkchlorid, wobei die Eisenhalogenide besonders bevorzugt sind.

Im allgemeinen werden 0,01 bis 0,5 mol, vorzugsweise 0,05 bis 0,2 mol, Lewis-Säure pro mol zu halogenierender Mehrfachchromophor If eingesetzt.

Üblicherweise werden 2 bis 12 mol, vor allem 2 bis 8 mol, N-Halogensuccinimid pro mol If benötigt.

Die Halogenierungstemperatur liegt im allgemeinen bei 20 bis 100°C, bevorzugt bei 40 bis 80°C.

Es empfiehlt sich, unter Schutzgas, z.B. Stickstoff oder Argon, zu arbeiten.

Übliche Reaktionsdauern betragen 0,5 bis 24 h, vor allem 1 bis 2 h.

Verfahrenstechnisch geht man zweckmäßigerweise wie folgt vor:
Man erhitzt eine Mischung von zu halogenierendem Mehrfachchromophor If, Lewis-Säure, N-Halogensuccinimid und Lösungsmittel unter Rühren unter Schutzgas auf die gewünschte Reaktionstemperatur. Nach Abkühlen auf Raumtemperatur fällt man das Reaktionsprodukt mit verdünnter anorganischer Säure, z.B. mit verdünnter Salzsäure, aus. Das Reaktionsprodukt wird abfiltriert, mit heißem Wasser gewaschen und getrocknet.

Eine weitere Reinigung der erhaltenen Mehrfachchromophore If kann gewünschtenfalls wie oben beschrieben vorgenommen werden.

Gewünschtenfalls können die dihalogenierten Mehrfachchromophore If durch Umsetzung mit einem Metallcyanid in Gegenwart eines aprotischen Lösungsmittels und eines Übergangsmetallkatalysators in die entsprechenden dicyanosubstituierten Mehrfachchromophore If (B' = verbleibender Rest B¹ oder B² = Cyano) überführt werden.

Als Metallcyanide sind hierbei vor allem Alkalimetallcyanide, wie Natriumcyanid oder Kaliumcyanid, oder Zinkcyanid geeignet.

Als aprotisches Lösungsmittel eignen sich die vorstehend genannten Lösungsmittel, wobei die N,N-disubstituierten aliphatischen Carbonsäureamide und die N-Alkyllactame bevorzugt sind.

In der Regel werden 10 bis 200 ml, insbesondere 10 bis 100 ml, Lösungsmittel pro g dihalogenierter Mehrfachchromophor If eingesetzt.

Als Übergangsmetallkatalysator sind insbesondere Palladiumkomplexe geeignet, z.B. Tetrakis(triphenylphosphin)palladium(0), Tetrakis(tris-o-tolylphosphin)palladium(0), [1,2-Bis(diphenylphosphino)ethan]palladium(II)chlorid, [1,1'-Bis(diphenylphosphino)-ferrocen]palladium(II)chlorid, Bis(triethylphosphin)palladium(II)chlorid, Bis(tricyclohexylphosphin)palladium(II)acetat, (2,2'-Bipyridyl)palladium(II)chlorid, Bis(triphenylphosphin)palladium(II)chlorid, Tris(dibenzylidenaceton)dipalladium(0), 1,5-Cyclooctadienpalladium(II)chlorid, Bis(acetonitril)palladium(II)chlorid und Bis(benzonitril)palladium(II)chlorid, wobei [1,1'-Bis(diphenylphosphino)ferrocen]palladium(II)chlorid und Tetrakis(triphenylphosphin)palladium(0) bevorzugt sind.

Üblicherweise kommen 1 bis 10 mol-%, vor allem 2 bis 5 mol-%, Übergangsmetallkatalysator, bezogen auf den dihalogenierten Mehrfachchromophor If, zum Einsatz.

Im allgemeinen werden 2 bis 10, vorzugsweise 2 bis 6, Äquivalente Metallcyanid pro mol dihalogenierter Mehrfachchromophor If verwendet.

Die Reaktionstemperatur liegt in der Regel bei 20 bis 150°C, bevorzugt bei 50 bis 100°C.

Es empfiehlt sich, unter Schutzgas, z.B. Stickstoff oder Argon, zu arbeiten.

Die Umsetzung ist üblicherweise in 2 bis 40 h, vor allem 4 bis 20 h, beendet.

Verfahrenstechnisch geht man zweckmäßigerweise wie folgt vor:
Man erhitzt eine Mischung von halogeniertem Mehrfachchromophor If, Übergangsmetallkatalysator und Metallcyanid unter Rühren unter Schutzgas auf die gewünschte Reaktionstemperatur. Nach Abkühlen auf Raumtemperatur fällt man das Reaktionsprodukt mit niederen Alkoholen, denen verdünnter Ammoniak zugesetzt sein kann, aus. Das Reaktionsprodukt wird abfiltriert, mit heißem Wasser gewaschen und getrocknet.

Eine weitere Reinigung der erhaltenen Mehrfachchromophore If kann gewünschtenfalls wie oben beschrieben vorgenommen werden.

### Amidierung

Mehrfachchromophore I, bei denen die Chromophore X über Amidgruppen an den Chromophor Rylen gebunden sind, können vorteilhaft ebenfalls ausgehend von den entsprechenden Perylendicarbonsäurechloriden IIf erhalten werden. Bei dem Chromophor Rylen handelt es sich wiederum insbesondere um Perylen.

Demgemäß wurde ein Verfahren zur Herstellung von Mehrfachchromophoren auf Basis von Rylendicarbonsäureamiden der allgemeinen Formel Ig 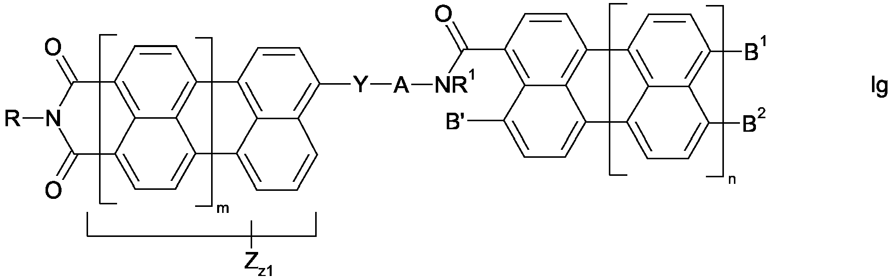 in der n = 1 und m = 0 ist, B' Wasserstoff, Halogen oder Cyano, einer der beiden Reste B¹ oder B² einen Rest der Formel 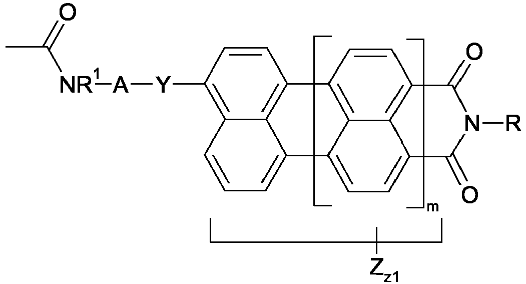 in der m = 0 ist, und der andere Rest Wasserstoff, Halogen oder Cyano bedeutet, gefunden, welches dadurch gekennzeichnet ist, daß man ein Rylendicarbonsäurechlorid der allgemeinen Formel IIf 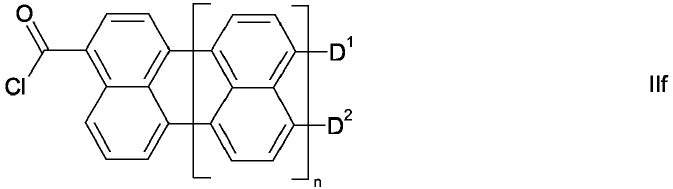 in der n = 1 ist und einer der beiden Reste D¹ oder D² -COCl oder der andere Rest Wasserstoff bedeutet, in Gegenwart einer nichtnucleophilen Base und gewünschtenfalls zusätzlich eines aprotischen Lösungsmittels mit 0,8 bis 1,2 mol pro mol eines Rylendicarbonsäureimidderivats der allgemeinen Formel IIIg 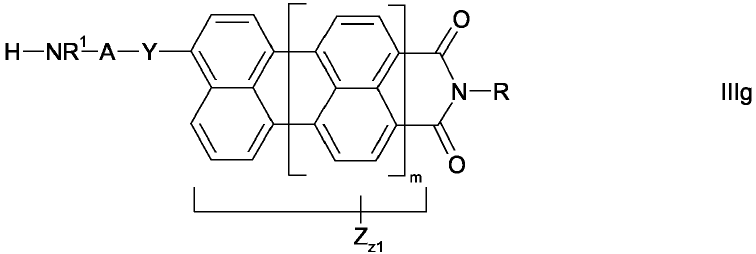 in der m = 0 ist, zu einem Rylendicarbonsäureamid der Formel lg, in der B' und der verbleibende Reste B¹ oder B² Wasserstoff bedeuten, umsetzt
und gewünschtenfalls das erhaltene Rylendicarbonsäureamid nach Zwischenisolierung in Gegenwart eines polaren organischen Lösungsmittels und einer Lewis-Säure mit N-Halogensuccinimid zum Rylendicarbonsäureamid der Formel lg, in der B' und der verbleibende Rest B¹ oder B² Halogen bedeuten, halogeniert
und gewünschtenfalls das erhaltene halogenierte Rylendicarbonsäureamid mit einem Metallcyanid in Gegenwart eines aprotischen Lösungsmittels und eines Übergangsmetallkatalysators in das entsprechende cyanosubstituierte Rylendicarbonsäureamid der Formel lg, in der B' und der verbleibende Rest B¹ oder B² Cyano bedeuten, überführt.

Da auch für die Amidierung die üblicherweise in Form von Mischungen des 3,9- und 3,10-Isomers vorliegenden Perylendicarbonsäurechloride IIIf eingesetzt werden, werden auch die Mehrfachchromophore lg als entsprechende Isomerenmischung erhalten.

Die Amidierung wird in Gegenwart einer nichtnucleophilen Base und gewünschtenfalls zusätzlich eines aprotischen Lösungsmittels vorgenommen.

Als nichtnucleophile Base werden vorzugsweise nichtnucleophile Stickstoffbasen eingesetzt. Geeignete Beispiele hierfür sind Alkylamine, insbesondere Tri(C₂-C₆-alkyl)-amine, wie Triethylamin, Tripropylamin und Tributylamin, und heterocyclische Basen, wie Pyridin, 4-(N,N-Dimethylamino)pyridin, (4-Pyrrolidino)pyridin, N-Methylpiperidin, N-Methylpiperidon, N-Methylmorpholin, N-Methyl-2-pyrrolidon, Pyrimidin, Chinolin, Isochinolin, Chinaldin, Diazabicyclooctan (DABCO), Diazabicyclononen (DBN) und Diazabicycloundecen (DBU).

Diese Stickstoffbasen können gleichzeitig als Lösungsmittel dienen. In diesem Fall werden üblicherweise 10 bis 100 g, bevorzugt 10 bis 50 g, Base pro g Rylendicarbonsäurechlorid IIf verwendet.

Wird die Amidierung in Gegenwart eines weiteren aprotischen Lösungsmittels, insbesondere eines N,N-disubstituierten aliphatischen Carbonsäureamids oder eines N-Alkyllactams, vorgenommen, so kommen in der Regel 3 bis 50 g, vor allem 10 bis 25 g, Stickstoffbase und etwa 3 bis 50 ml, insbesondere 10 bis 25 ml, des zusätzlichen Lösungsmittels, jeweils pro g Rylendicarbonsäurechlorid IIf zum Einsatz.

In der Regel werden Rylendicarbonsäureimidderivat IIIg und Rylendicarbonsäurechlorid IIf im Molverhältnis 0,8 : 1 bis 1,2 : 1 umgesetzt.

Die Reaktionstemperatur liegt üblicherweise bei 0 bis 75°C, vor allem bei 20 bis 50°C.

Es empfiehlt sich, die Amidierung unter wasserfreien Bedingungen vorzunehmen.

Die Reaktionsdauer beträgt in der Regel 1 bis 24 h, bevorzugt 1 bis 5 h.

Verfahrenstechnisch geht man bei der Amidierung zweckmäßigerweise wie folgt vor:
Man mischt Rylendicarbonsäurechlorid IIf, Rylendicarbonsäureimidderivat IIIg und nichtnucleophile Base sowie gegebenenfalls Lösungsmittel, erhitzt diese Mischung gegebenenfalls auf die gewünschte Reaktionstemperatur und rührt 1 bis 24 h bei der gewünschten Reaktionstemperatur.

Die Isolierung der Mehrfachchromophore Ig kann durch Verdünnen mit protischen Lösungsmitteln, wie Wasser und niederen aliphatischen Alkoholen, Abfiltrieren, wiederholtes Waschen mit einer wäßrigen Base, z.B. einer Alkalimetallhydrogencarbonatlösung, und abschließend mit Wasser und anschließendes Trocknen erfolgen.

Wie bei der nucleophilen Substitution beschrieben, können die erhaltenen Mehrfachchromophore Ig gewünschtenfalls zur weiteren Reinigung einer Säulenchromatographie bzw. Säulenfiltration oder einer Umkristallisation bzw. fraktionierten Kristallisation, wobei Schwefelsäure als Lösungsmittel ausgeschlossen ist, unterzogen werden.

Mehrfachchromophore Ig, die im Chromophor Rylen dihalogeniert, insbesondere dichloriert oder dibromiert, bzw. dicyanosubstituiert sind (B' = verbleibender Rest B¹ oder B² = Halogen bzw. Cyano), können analog zu den entsprechenden Mehrfachchromophoren If hergestellt werden. Zur näheren Beschreibung der jeweiligen Vorgehensweise sei daher auf die Ausführungen im Abschnitt Veresterung verwiesen.

### Herstellung der Rylendicarbonsäureimidderivate III

Schließlich wurde auch ein Verfahren zur Herstellung von Rylendicarbonsäureimidderivaten der eingangs definierten Formel III 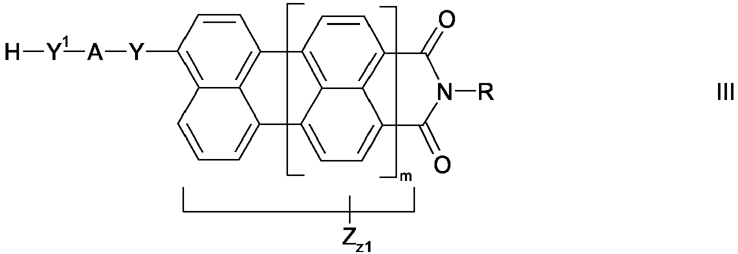 gefunden, welches dadurch gekennzeichnet ist, daß man ein peri-halogeniertes Rylendicarbonsäureimid der allgemeinen Formel V 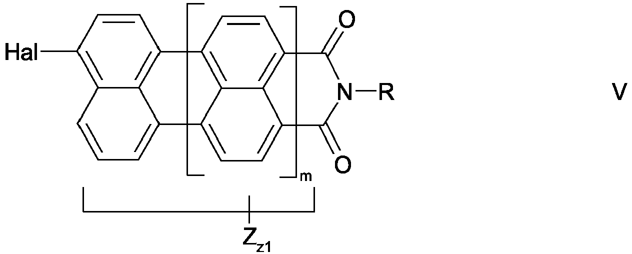 in der Hal Chlor oder Brom bedeutet, in Gegenwart einer Base und eines nichtnucleophilen Lösungsmittels mit 2 bis 5 mol pro mol auszutauschendes Halogenatom einer mindestens bifunktionellen aromatischen Verbindung der allgemeinen Formel VI

H-Y¹-A-Y-H VI

umsetzt.

Bei der das peri-Halogenatom ersetzenden, mindestens bifunktionellen Verbindung VI handelt es sich um einen Di(thio)alkohol oder einen Amino(thio)alkohol, der über Y (-O-bzw. -S-) an das Rylendicarbonsäureimid V gebunden wird.

Die mindestens bifunktionelle Verbindung VI enthält das oben eingehend beschriebene, mindestens einen (het)aromatischen Rest enthaltende Brückenglied A als die funktionellen Gruppen -YH und -Y¹H verknüpfende Einheit. Bevorzugte und besonders bevorzugte Verbindungen VI ergeben sich daher zwanglos aus den oben beschriebenen Brückengliedern A.

Als Beispiele für ganz besonders geeignete Verbindungen VI seien im einzelnen genannt:
1,4-Dihydroxybenzol (Hydrochinon), 1,3-Dihydroxybenzol (Resorcin), 1,2-Dihydroxybenzol (Brenzkatechin), 1,4-, 1,3- und 1,2-Dimercaptobenzol, 1,4-Dihydroxy-2,5-di(tert.-butyl)benzol, 1,4-Dihydroxy-2,5-dihexylbenzol, 1,4-Dihydroxy-2,5-di(tert.-octyl)-benzol, 1,4-Dihydroxy-2,5-didodecylbenzol, 1,4-Dihydroxy-2,5-di(2-dodecyl)benzol, p-, m- und o-Aminophenol, 1,4- und 1,8-Dihydroxynaphthalin, 1,4- und 1,8-Dimercaptonaphthalin, 1,4- und 1,8-Diaminonaphthalin, 4,4'-, 3,3'- und 2,2'-Dihydroxydiphenyl (4,4'-, 3,3'- und 2,2'-Dihydroxybiphenyl), 4,4'-Dihydroxy-2,2',6,6'-tetramethyldiphenyl, 4,4'-Dihydroxy-2,2',6,6'-tetraisopropyldiphenyl, 4,4'-Dihydroxy-2,2',6,6'-tetra(2-hexyl)-diphenyl, 4,4'-Dihydroxy-2,2',6,6'-tetramethyldiphenyl, (4,4'-Dihydroxydiphenyl)methan, (4,4'-Dihydroxydiphenyl)dimethylmethan (Bisphenol A), (4,4'-Dihydroxydiphenyl)diethylmethan und (4,4'-Dihydroxydiphenyl)diphenylmethan, wobei Hydrochinon, 1,4-Dihydroxy-2,5-di(tert.-octyl)benzol, p-Aminophenol, 4,4'-Di-hydroxy-2,2',6,6'-tetramethyldiphenyl, 4,4'-Dihydroxy-2,2',6,6'-tetraisopropyldiphenyl, 4,4'-Dihydroxy-2,2',6,6'-tetra-(2-hexyl)diphenyl und Bisphenol A ganz besonders bevorzugt sind.

Bei der Umsetzung zwischen dem peri-Halogenrylendicarbonsäureimid V und der mindestens bifunktionellen Verbindung VI handelt es sich ebenfalls um eine nucleophile Substitution.

Die oben im Abschnitt Nucleophile Substitution angegebenen Reaktionsbedingungen (Art und Menge des nichtnucleophilen Lösungsmittels, Art und Menge der Base, Reaktionstemperatur, verfahrenstechnische Vorgehensweise, Isolierung und gewünschtenfalls Reinigung des Reaktionsprodukts) können daher auf die vorliegende Umsetzung übertragen werden.

Das Molverhältnis von mindestens bifunktioneller Verbindung VI zu peri-Halogenrylendicarbonsäureimid V beträgt 2 : 1 bis 5 : 1, vorzugsweise 3 : 1 bis 4 : 1.

Durch den Überschuß an VI kann die im Fall der Diole und Dithiole zu erwartende Bildung von über die Gruppierung -Y-A-Y- verdoppelten Rylendicarbonsäureimiden wirkungsvoll unterdrückt werden. Das überschüssige VI kann zudem leicht durch Waschen mit einem protischen Lösungsmittel, wie Wasser, einem niederen aliphatischen Alkohol oder einem Alkohol/Wasser-Gemisch, aus dem Reaktionsprodukt, das in diesen Lösungsmitteln unlöslich ist, entfernt werden.

Die Rylendicarbonsäureimidderivate III sind daher vorteilhaft ohne Einsatz von aufwendiger Schutzgruppenchemie erhältlich.

Die erfindungsgemäßen Mehrfachchromophore I zeichnen sich durch die gezielte Anpaßbarkeit ihrer optischen Eigenschaften an den gewünschten Anwendungszweck aus. Ihre Emission ist langwellig gegenüber ihrer Absorption verschoben, wobei die jeweiligen Wellenlängenbereiche in einfacher Weise durch entsprechende Auswahl des Chromophors Rylen und des Chromophors X festgelegt werden kann. Auf diese Weise sind lösliche Farbmittel mit großem Stokes-Shift zugänglich.

Sie können problemlos in organische und anorganische Materialien eingearbeitet werden und eignen sich auch schon deshalb für eine ganze Reihe von Anwendungszwecken, von denen einige im folgenden beispielhaft aufgeführt werden.

Zur Charakterisierung der Mehrfachchromophore I wird dabei nur der Chromophor Rylen als Basis genannt. Wenn nichts anderes angegeben ist, stellt der Chromophor X das jeweils niedere Homologe dar.

Sie können generell zur Einfärbung von Lacken, Druckfarben und Kunststoffen eingesetzt werden.

Die Mehrfachchromophore I auf Perylen- und Terrylenbasis absorbieren im sichtbaren Bereich des elektromagnetischen Spektrums und zeichnen sich dabei insbesondere durch ihre Fluoreszenz aus, die im Fall der Mehrfachchromophore auf Perylenbasis im sichtbaren Bereich des elektromagnetischen Spektrums liegt.

Die Mehrfachchromophore I auf Quaterrylenbasis sind insbesondere aufgrund ihres Absorptionsvermögens im nahinfraroten Bereich des elektromagnetischen Spektrums von Interesse.

Die erfindungsgemäßen Mehrfachchromophore I können zur Herstellung elektromagnetische Strahlung absorbierender und/oder emittierender wäßriger Polymerisatdispersionen verwendet werden. Fluoreszierende Polymerisatdispersionen ergeben sich bei den Mehrfachchromophoren auf Terrylenbasis und insbesondere bei den Mehrfachchromophoren auf Perylenbasis, während bei den Mehrfachchromophoren auf Quaterrylenbasis im nahinfraroten Bereich des elektromagnetischen Spektrums absorbierende Polymerisatdispersionen resultieren.

Weiterhin eignen sich die Mehrfachchromophore zur Erzeugung von Markierungen und Beschriftungen. Die Mehrfachchromophore I auf Quaterrylenbasis ergeben hier aufgrund ihrer ausgeprägten Absorption im nahinfraroten Bereich des elektromagnetischen Spektrums für das menschliche Auge nicht sichtbare, Infrarotlicht absorbierende Markierungen und Beschriftungen, während die Mehrfachchromophore I auf Perylen- und Terrylenbasis aufgrund des großen Abstands zwischen (kurzwelliger) Absorption und (langwelliger) Emission von besonderem Interesse für Markierungs- und Beschriftungszwecke sind.

Die erfindungsgemäßen Mehrfachchromophore I sind schließlich auch als Filter oder Emitter für Display-Anwendungen einsetzbar. Dabei sind die im sichtbaren Bereich absorbierenden, fluoreszierenden Mehrfachchromophore I, vor allem also die Mehrfachchromophore auf Perylenbasis, als absorbierende Colorfilter oder als fluoreszierende Emitter für LCD- und OLED-Displays von Interesse.

Schließlich können die Mehrfachchromophore I auch als Emitter in Chemilumineszenzanwendungen Anwendung finden. Hier sind wiederum die fluoreszierenden Mehrfachchromophore auf Perylen- und Terrylenbasis besonders geeignet.

Nicht zuletzt können die erfindungsgemäßen Mehrfachchromophore I, insbesondere die Mehrfachchromophore auf Perylen- und Terrylenbasis, auch als Aktivkomponenten in der Photovoltaik zum Einsatz kommen.

### Beispiele

### Beispiel 1: Mehrfachchromophor Ia1

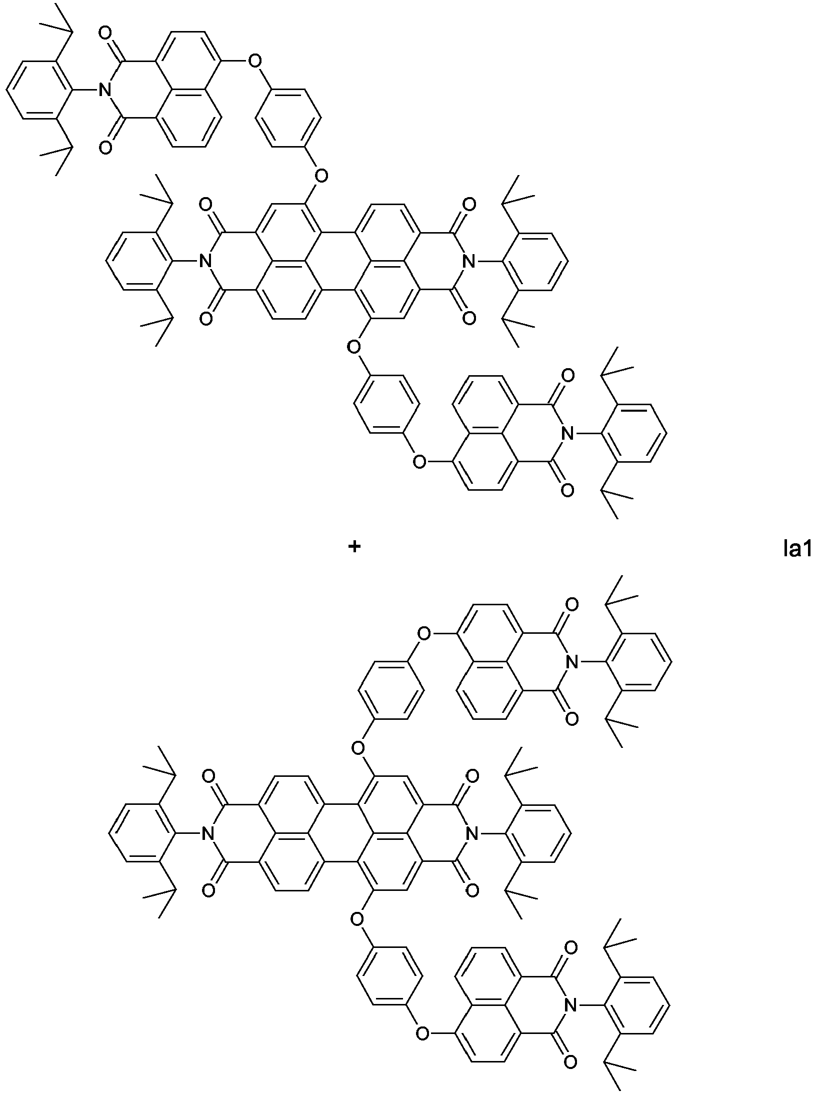

### a) Herstellung des Rylendicarbonsäureimidderivats 1111

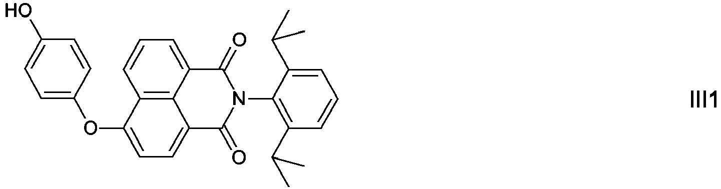

Eine Mischung aus 30 ml N-Methylpyrrolidon, 4,15 g (30 mmol) Kaliumcarbonat, 6,60 g (60 mmol) Hydrochinon und 4,15 g (15 mmol) N-(2,6-Diisopropylphenyl)-4-chlornaphthalin-1,8-dicarbonsäureimid wurde unter Stickstoff 24 h bei 80°C gerührt.

Nach Abkühlen auf Raumtemperatur wurde das Reaktionsgemisch auf 3 I Wasser gefällt. Die erhaltene Suspension wurde 30 min auf Rückflußtemperatur erhitzt, auf Raumtemperatur abgekühlt und filtriert. Der Filterrückstand wurde in einer Mischung aus 200 ml Wasser und 5 ml Ethanol 1 h bei 60°C gerührt, erneut abfiltriert, mit etwa 400 ml warmem Wasser gewaschen und im Vakuum bei 80°C getrocknet.

Es wurden 6,9 g (quantitative Umsetzung) des Rylendicarbonsäureimidderivats 1111 in Form eines gelblichen Feststoffs erhalten.

R_{f}-Wert (Toluol/Ethanol 7:1) = 0,40.

### b) Herstellung des Mehrfachchromophors Ia1

Eine Mischung aus 4,6 g (10 mmol) des Rylendicarbonsäureimidderivats 1111, 0,86 g (6 mmol) Kaliumcarbonat, 50 ml N-Methylpyrrolidon und 4,34 g (5 mmol) eines Gemisches von N,N'-Bis(2,6-diisopropylphenyl)-1,7- und -1,6-dibromperylen-3,4:9,10-tetracarbonsäurediimid (Isomerenverhältnis 75 : 25) wurde unter Stickstoff 28 h bei Raumtemperatur gerührt. Nach Zugabe von weiteren 0,86 g (6 mmol) Kaliumcarbonat wurde weitere 20 h bei Raumtemperatur gerührt.

Das Reaktionsgemisch wurde langsam in eine Mischung aus 1 I Wasser, 50 g 6 gew.-%iger Schwefelsäure und 50 ml Ethanol gegossen. Das auf diese Weise ausgefällte Produkt wurde abfiltriert, mehrfach mit Wasser und mit Ethanol gewaschen und im Vakuum bei 80°C getrocknet.

Es wurden 7,74 g (95%) des Mehrfachchromophors la1 in Form eines roten Feststoffs erhalten, der zur weiteren Reinigung einer Säulenchromatographie an Kieselgel mit einem Toluol/Essigester-Gemisch (20:1) als Eluens unterzogen wurde.

R_{f}-Wert (Toluol/Essigester 10:1) = 0,42;
Absorption: λₘₐₓ (CH₂Cl₂) = 351, 525 nm;
Emission: λₘₐₓ (CH₂Cl₂) = 575 nm.

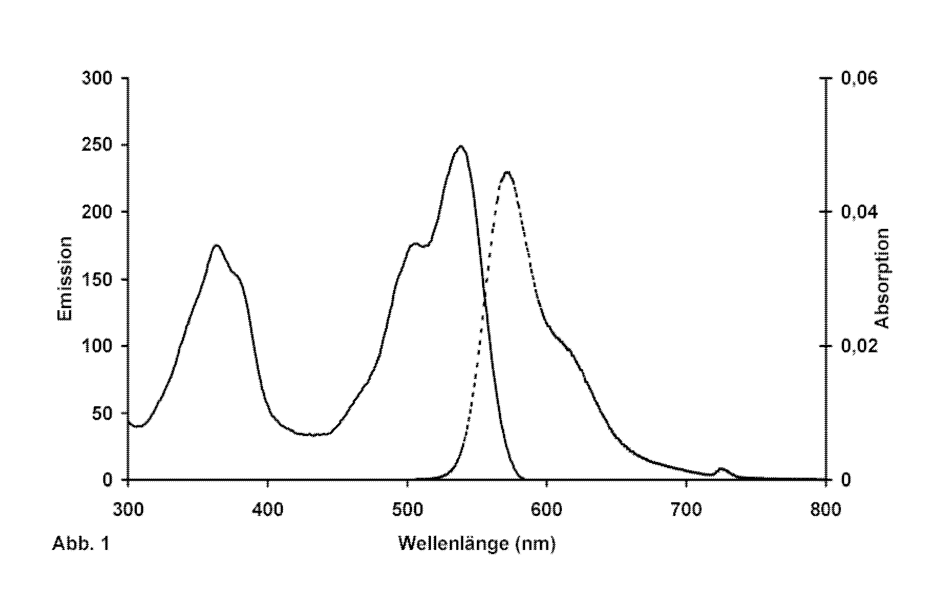

Abbildung 1 zeigt das Absorptionsspektrum (durchgezogene Linie) und Emissionsspektrum (unterbrochene Linie; Anregung bei 364 nm) des gereinigten Mehrfachchromophors la1 in Methylenchlorid.

### Beispiel 2: Mehrfachchromophor la2

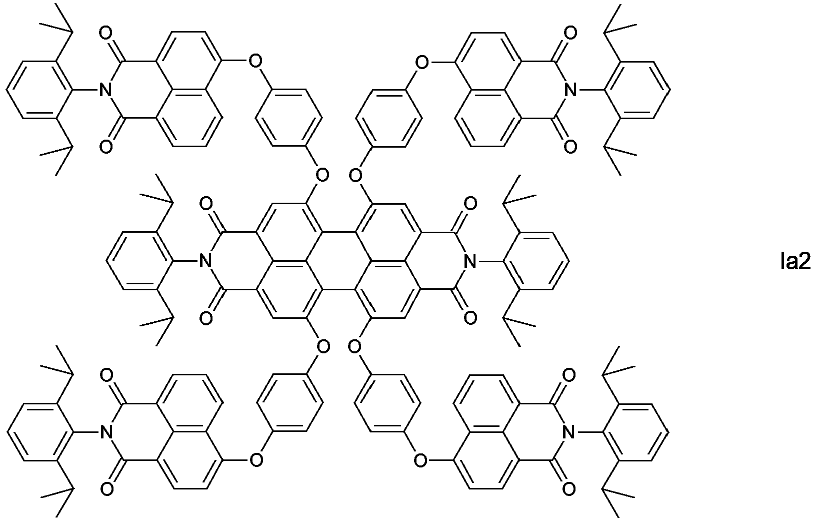

Eine Mischung aus 0,93 g (2 mmol) des Rylendicarbonsäureimidderivats 1111 aus Beispiel 1a), 0,17 g (1,25 mmol) Kaliumcarbonat, 10 ml N-Methylpyrrolidon und 0,42 g (0,5 mmol) N,N'-Bis(2,6-diisopropylphenyl)-1,6,7,12-tetrachlorperylen-3,4:9,10-tetracarbonsäurediimid wurde unter Stickstoff 30 h bei 80°C gerührt.

Nach Abkühlen auf Raumtemperatur wurde das Reaktionsgemisch langsam in eine Mischung aus 300 ml 5 gew.-%iger Schwefelsäure und 30 ml Ethanol gegossen. Nach einstündigem Rühren wurde das auf diese Weise ausgefällte Produkt abfiltriert, mit Wasser gewaschen und im Vakuum bei 80°C getrocknet. Das getrocknete Produkt wurde in Toluol unter Erwärmen gelöst und mit wenig Aktivkohle ausgerührt. Die Aktivkohle wurde anschließend abfiltriert, und das Filtrat wurde im Vakuum eingedampft.

Es wurden 0,8 g (62%) des Mehrfachchromophors la2 in Form eines violetten Feststoffs erhalten, der zur weiteren Reinigung einer Säulenfiltration an Kieselgel mit einem Toluol/Essigester-Gemisch (10:1) als Eluens unterzogen wurde.

R_{f}-Wert (Toluol/Essigester 10:1) = 0,42;
Absorption: λₘₐₓ (CH₂Cl₂) = 349, 551 nm;
Emission: λₘₐₓ (CH₂Cl₂ = 602 nm.

### Beispiel 3: Mehrfachchromophor Ia3

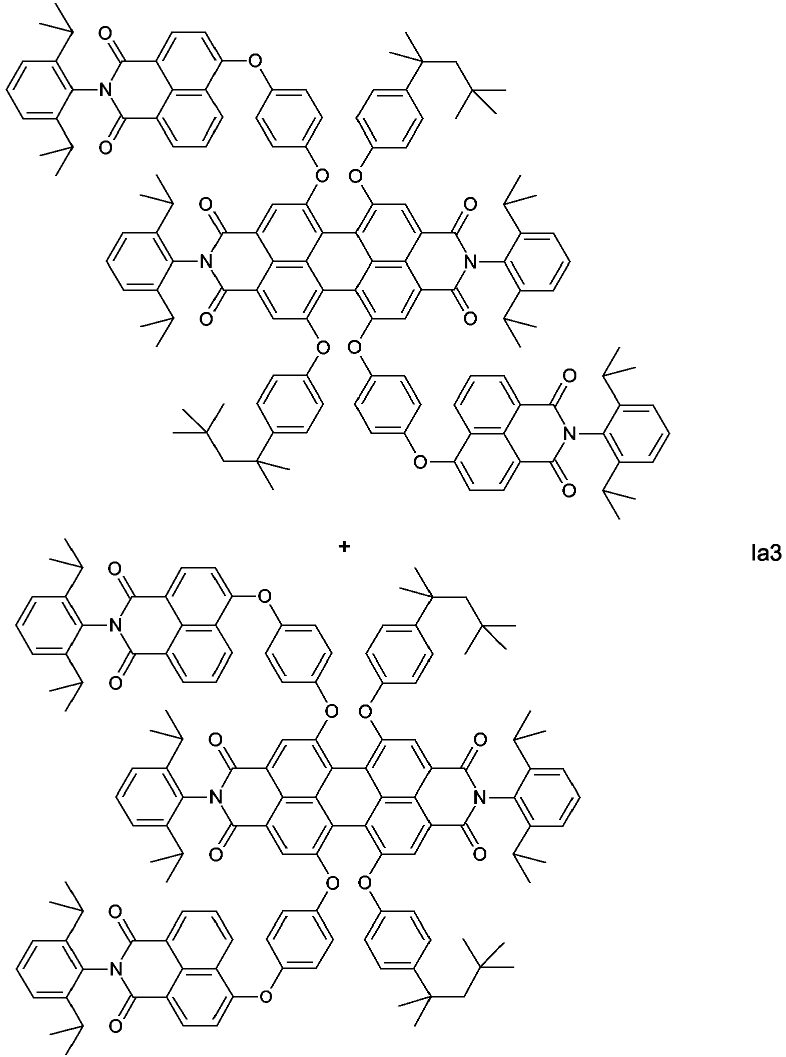

Die Formel Ia3 gibt die Hauptkomponenten des durch die beiden Phenoxyreste (X und Z) tetrasubstituierten Produkts wieder, das als Gemisch der die beiden Phenoxyreste im Verhältnis 4:0, 1:3, 2:2, 1:3 und 0:4 enthaltenden Produkte vorliegt.

Eine Mischung aus 3,72 g (8 mmol) des Rylendicarbonsäureimidderivats 1111 aus Beispiel 1a), 1,38 g (10 mmol) Kaliumcarbonat, 50 ml N-Methylpyrrolidon und 3,39 g (4 mmol) N,N'-Bis(2,6-diisopropylphenyl)-1,6,7,12-tetrachlorperylen-3,4:9,10-tetracarbonsäurediimid wurde unter Stickstoff 12 h bei 80°C gerührt. Nach Zugabe von 2,48 g (12 mmol) tert.-Octylphenol wurde weitere 6 h bei 80°C gerührt.

Nach Abkühlen auf Raumtemperatur wurde das Reaktionsgemisch langsam in 250 ml 5 gew.-%ige Schwefelsäure gegossen. Das auf diese Weise ausgefällte Produkt wurde abfiltriert, zunächst mit warmem Wasser, dann mit einem 1:1-Gemisch von Wasser und Methanol und abschließend mit reinem Methanol gewaschen und im Vakuum bei 80°C getrocknet. Das getrocknete Produkt wurde in Toluol unter Erwärmen gelöst und mit wenig Aktivkohle ausgerührt. Die Aktivkohle wurde anschließend abfiltriert, und das Filtrat wurde im Vakuum eingedampft.

Es wurden 7,18 g (87%) des Mehrfachchromophors Ia3 in Form eines roten Feststoffs als Isomerengemisch mit Ia3 als Hauptkomponenten erhalten.

R_{f}-Wert (Toluol/Essigester 10:1) = 0,57; 0,82; 0,43; 0,49; 0,96;
Absorption: λₘₐₓ (CH₂Cl₂) = 361, 570 nm;
Emission: λₘₐₓ (CH₂Cl₂) = 611 nm.

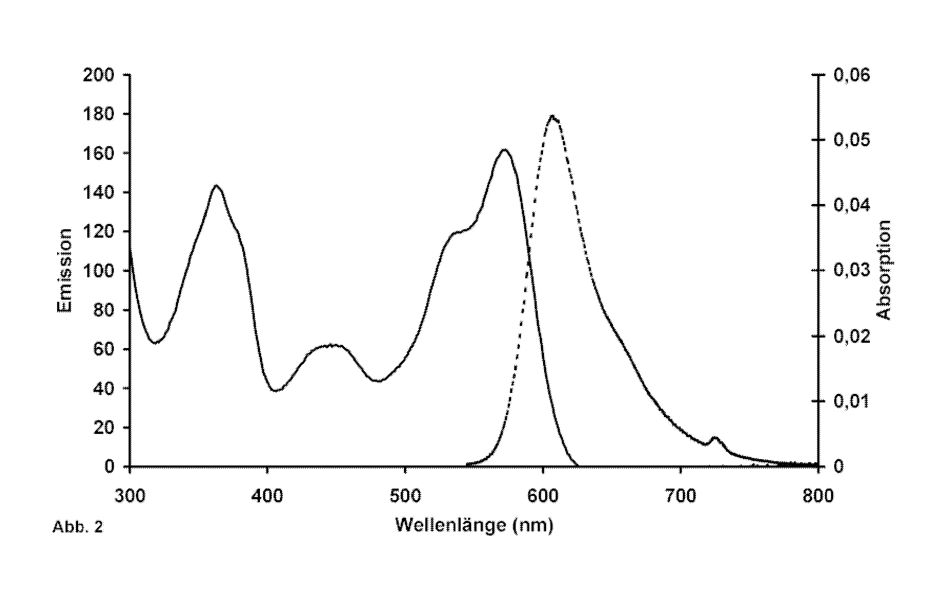

Abbildung 2 zeigt das Absorptionsspektrum (durchgezogene Linie) und Emissionsspektrum (unterbrochene Linie; Anregung bei 364 nm) des Mehrfachchromophors Ia3 in Methylenchlorid.

### Beispiel 4: Mehrfachchromophor Ib1

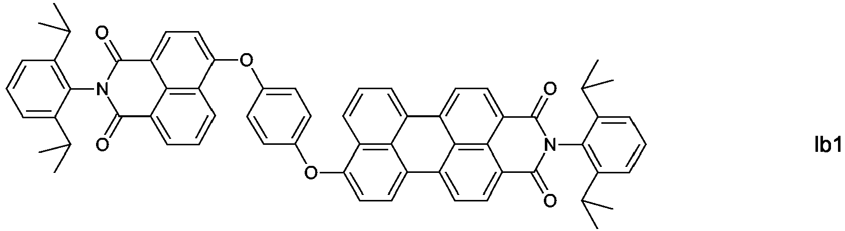

### a) Herstellung des Rylendicarbonsäureimidderivats III2

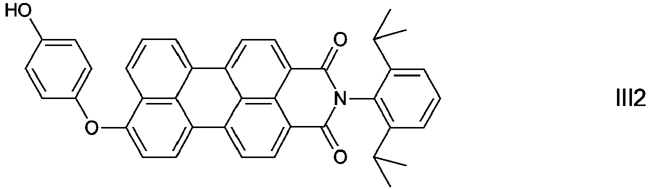

Eine Mischung aus 2,8 g (5 mmol) N,N'-Bis(2,6-diisopropylphenyl)-9-bromperylen-3,4:9,10-tetracarbonsäurediimid, 1,73 g (12,5 mmol) Kaliumcarbonat, 50 ml N-Methylpyrrolidon und 2,2 g (20 mmol) Hydrochinon wurde unter Stickstoff zunächst 22 h bei 80°C und dann weitere 22 h bei 110°C gerührt.

Nach Abkühlen auf Raumtemperatur wurde das Reaktionsgemisch langsam in 300 ml 5 gew.-%ige Schwefelsäure gegossen. Das auf diese Weise ausgefällte Produkt wurde abfiltriert, zunächst mit Wasser und dann mit einem 1:1-Gemisch von Wasser und Methanol gewaschen und im Vakuum bei 80°C getrocknet.

Es wurden 2,7 g (92%) des Rylendicarbonsäureimidderivats III2 in Form eines violetten Feststoffs erhalten.

R_{f}-Wert (Toluol/Essigester 10:1) = 0,12.

### b) Herstellung des Mehrfachchromophors Ib1

Eine Mischung aus 1,77 g (3 mmol) des Rylendicarbonsäureimidderivats III2, 1,18 g (3 mmol) N-(2,6-Diisopropylphenyl)-4-chlornaphthalin-1,8-dicarbonsäureimid, 1,04 g (7,5 mmol) Kaliumcarbonat und 50 ml N-Methylpyrrolidon wurde unter Stickstoff 34 h bei 80°C gerührt.

Nach Abkühlen auf Raumtemperatur wurde das Reaktionsgemisch langsam in 500 ml 5 gew.-%ige Schwefelsäure gegossen. Das auf diese Weise ausgefällte Produkt wurde abfiltriert, mit warmem Wasser gewaschen und im Vakuum bei 80°C getrocknet.

Es wurden 2,78 g (quantitative Umsetzung) des Mehrfachchromophors Ib1 in Form eines roten Feststoffs erhalten.

R_{f}-Wert (Toluol/Essigester 10:1) = 0,40;
Absorption: λₘₐₓ (CH₂Cl₂) = 353, 510 nm;
Emission: λₘₐₓ (CH₂Cl₂) = 584 nm.

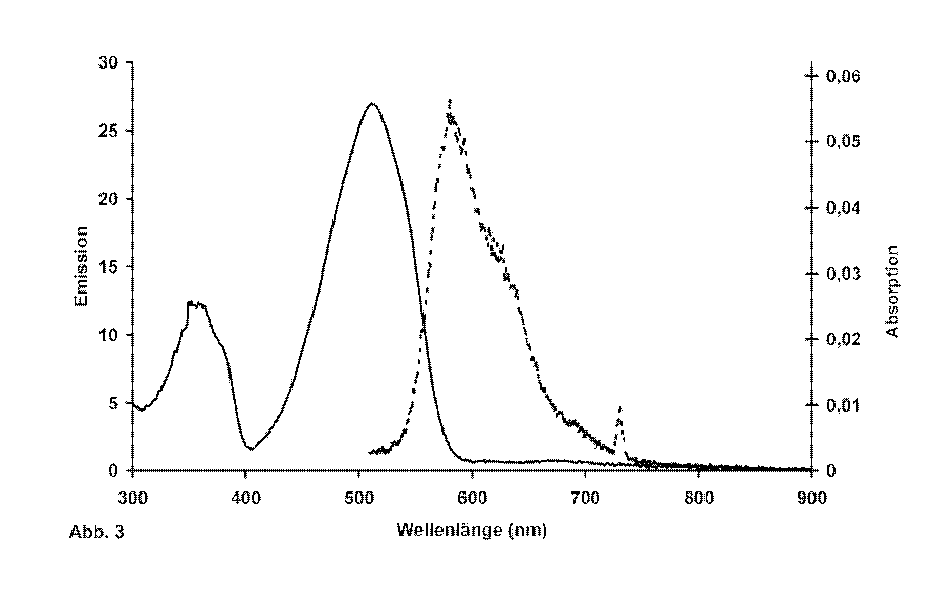

Abbildung 3 zeigt das Absorptionsspektrum (durchgezogene Linie) und Emissionsspektrum (unterbrochene Linie; Anregung bei 364 nm) des Mehrfachchromophors Ib1 in Methylenchlorid.

### Beispiel 5: Mehrfachchromophor Ia4

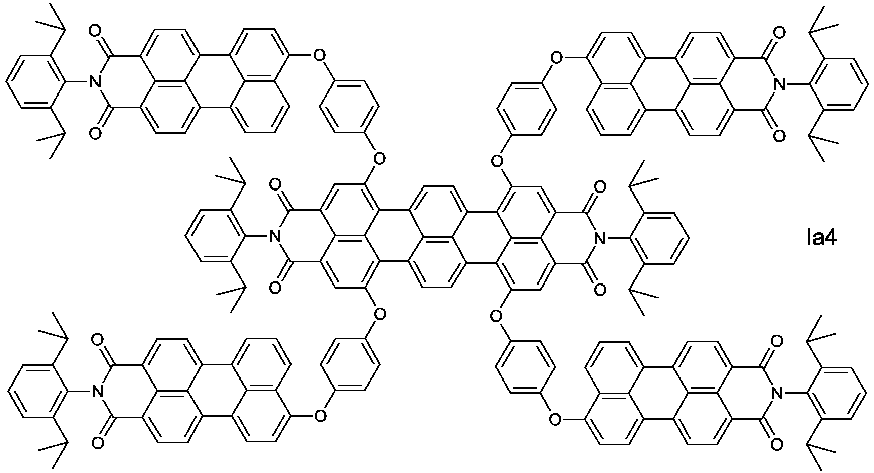

Eine Mischung aus 4,4 g (7,5 mmol) des Rylendicarbonsäureimidderivats 1112 aus Beispiel 4a), 1,24 g (9 mmol) Kaliumcarbonat, 100 ml N-Methylpyrrolidon und 1,73 g (1,5 mmol) N,N'-Bis(2,6-diisopropylphenyl)-1,6,9,14-tetrabromterrylen-3,4:11,12-tetracarbonsäurediimid wurde unter Stickstoff 3 h bei 80°C gerührt.

Nach Abkühlen auf Raumtemperatur wurde das Reaktionsgemisch langsam in 600 ml 5 gew.-%ige Schwefelsäure gegossen. Das auf diese Weise ausgefällte Produkt wurde abfiltriert, mehrfach mit Wasser und mit Ethanol gewaschen und im Vakuum bei 80°C getrocknet.

Es wurden 5,1 g (quantitative Umsetzung) des Mehrfachchromophors Ia4 in Form eines violetten Feststoffs erhalten, der zur weiteren Reinigung einer Säulenchromatographie an Kiesel mit einem Toluol/Ethanol-Gemisch (10:1) als Eluens unterzogen wurde.

R_{f}-Wert (Toluol/Ethanol 10:1) = 0,20;
Absorption: λₘₐₓ (CH₂Cl₂) = 510, 675 nm;
Emission: λₘₐₓ (CH₂Cl₂) = 709 nm.

### Beispiel 6: Mehrfachchromophor Ic1

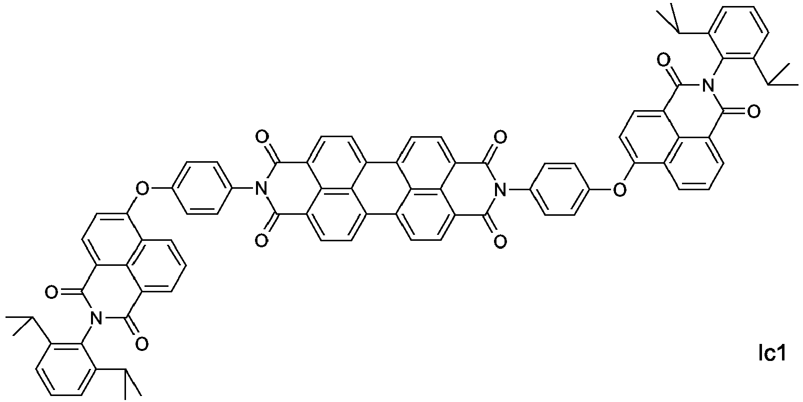

### a) Herstellung des Rylendicarbonsäureimidderivats III3

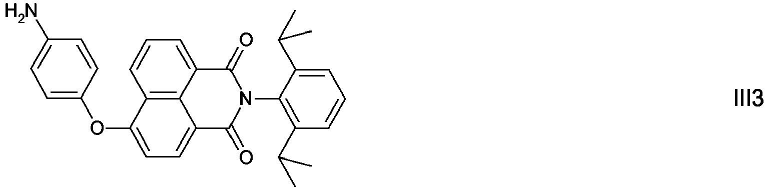

Eine Mischung aus 20,0 g (50 mmol) N-(2,6-Diisopropylphenyl)-4-chlornaphthalin-1,8-dicarbonsäureimid, 6,7 g (60 mmol) p-Aminophenol, 3,5 g (25 mmol) Kaliumcarbonat und 100 ml N-Methylpyrrolidon wurde unter Stickstoff 24 h bei 80°C gerührt. Nach Zugabe von weiteren 7,0 g (50 mmol) Kaliumcarbonat wurde weitere 6 h bei 80°C gerührt.

Nach Abkühlen auf Raumtemperatur wurde das Reaktionsgemisch auf 500 ml Wasser gefällt. Die erhaltene Suspension wurde 24 h auf 80°C erhitzt, auf Raumtemperatur abgekühlt und filtriert. Der Filterrückstand wurde mehrfach mit heißem Wasser gewaschen und im Vakuum bei 80°C getrocknet.

Es wurden 21,5 g (92%) des Rylendicarbonsäureimidderivats III3 in Form eines bräunlichen Feststoffs erhalten.

R_{f}-Wert (Toluol/'Ethanol 7:2) = 0,60.

### b) Herstellung des Mehrfachchromophors Ic1

Eine Mischung aus 1,04 g (2,5 mmol) Perylen-3,4:9,10-tetracarbonsäuredianhydrid,

50 ml Chinolin, 2,4 g des Rylendicarbonsäureimidderivats 1113 und 0,5 g (2,5 mmol) Zinkacetatdihydrat wurde unter Stickstoff 6 h bei 160°C, 6 h bei 180°C und 2 h bei 200°C gerührt.

Nach Abkühlen auf Raumtemperatur wurde das Produkt durch Zugabe von 250 ml Methanol ausgefällt, abfiltriert, in 250 ml Methanol erneut auf Rückflußtemperatur erhitzt, nach dem Abkühlen abfiltriert und im Vakuum bei 80°C getrocknet.

Es wurden 2,95 g (92%) des Mehrfachchromophors Ic1 in Form eines orangefarbenen Feststoffs erhalten, der zur weiteren Reinigung einer fraktionierten Kristallisation in einem N-Methylpyrrolidon/Ethanol-Gemisch (1:3) unterzogen wurde.

R_{f}-Wert (Toluol/Ethanol 7:2) = 0,80;
Absorption: λₘₐₓ (CH₂Cl₂) = 361, 489, 525 nm;
Emission: λₘₐₓ (CH₂Cl₂) = 535, 578 nm.

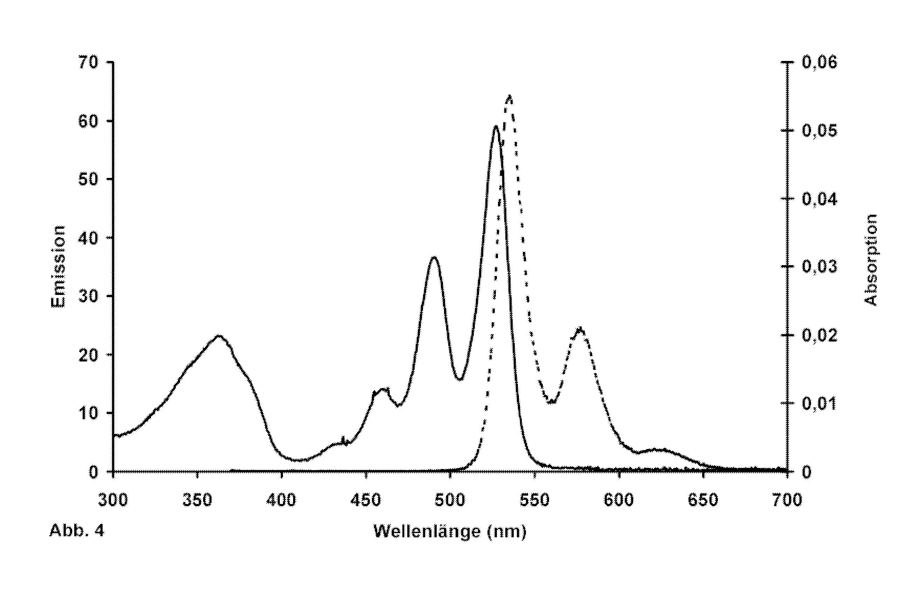

Abbildung 4 zeigt das Absorptionsspektrum (durchgezogene Linie) und Emissionsspektrum (unterbrochene Linie; Anregung bei 361 nm) des gereinigten Mehrfachchromophors Ic1 in Methylenchlorid.

### Beispiel 7: Mehrfachchromophor If1

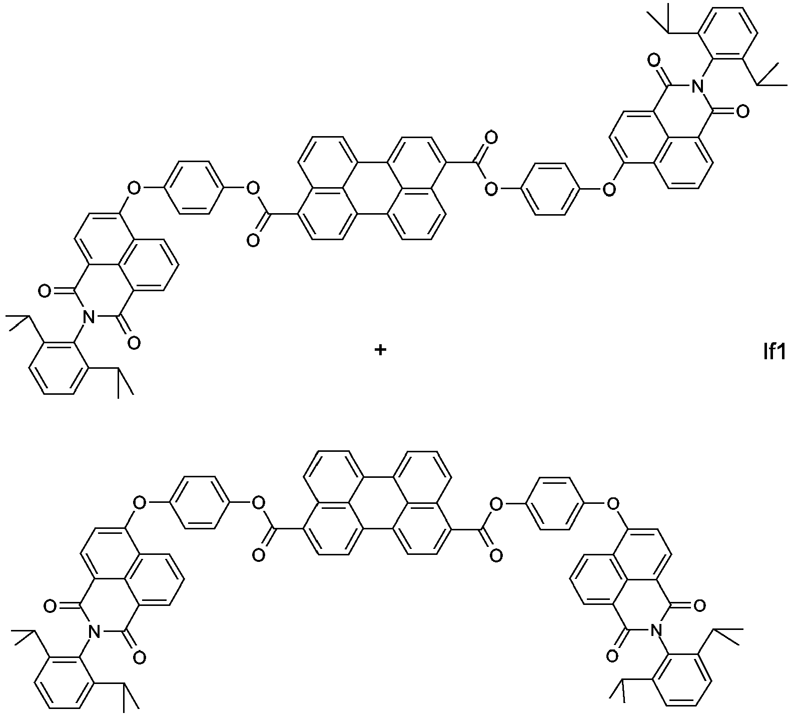

### a) Herstellung des Gemischs von Perylen-3,9- und -3,10-dicarbonsäurechlorid (Isomerenverhältnis etwa 1 : 1) IIf1

Zu einer Mischung aus 17,0 g (50 mmol) eines Gemischs aus Perylen-3,9- und -3,10-dicarbonsäure (Isomerenverhältnis etwa 1 : 1), 125 mol Toluol und 5 Tropfen Dimethylformamid wurden unter Stickstoff 13,7 g (120 mmol) Thionylchlorid bei Raumtemperatur in 15 min zugetropft. Dann wurde die Mischung 5 h bei 110°C gerührt.

Nach Abkühlen des Reaktionsgemischs auf Raumtemperatur wurde das Produkt abfiltriert, mit Petrolether bis zum farblosen Ablauf gewaschen und im Ölpumpenvakuum getrocknet.

Es wurden 17,2 g (91%) des Isomerengemischs IIf1 in Form eines roten Feststoffs erhalten.

### b) Herstellung des Mehrfachchromophors If1

Eine Suspension von 1,16 g (29 mmol) Natriumhydrid (60 gew.-%ig in Mineralöl), 100 ml Diethylenglykoldiethylether und 11,1 g (24 mmol) des Rylendicarbonsäureimidderivats 1111 aus Beispiel 1a) wurde unter Stickstoff 1 h bei Raumtemperatur gerührt. Nach Zugabe von 5,0 g (13 mmol) des Isomerengemischs IIf1 wurde nochmals 1 h bei Raumtemperatur gerührt.

Das Reaktionsgemisch wurde dann in 1 I Wasser gegeben. Das auf diese Weise ausgefällte Produkt wurde abfiltriert, viermal mit je 100 ml gesättigter Natriumhydrogencarbonatlösung und dann mit Wasser gewaschen und im Vakuum bei 80°C getrocknet.

Es wurden 15,0 g (91%) des Mehrfachchromophors If1 in Form eines roten Feststoffs erhalten, der zur weiteren Reinigung einer fraktionierten Kristallisation in einem N-Methylpyrrolidon/Wasser-Gemisch (1:1) unterzogen wurde.

R_{f}-Wert (Toluol/Methylenchlorid 1:10) = 0,16;
Absorption: λₘₐₓ (CH₂Cl₂) = 361, 445, 472 nm;
Emission: λₘₐₓ (CH₂Cl₂) = 490, 522 nm.

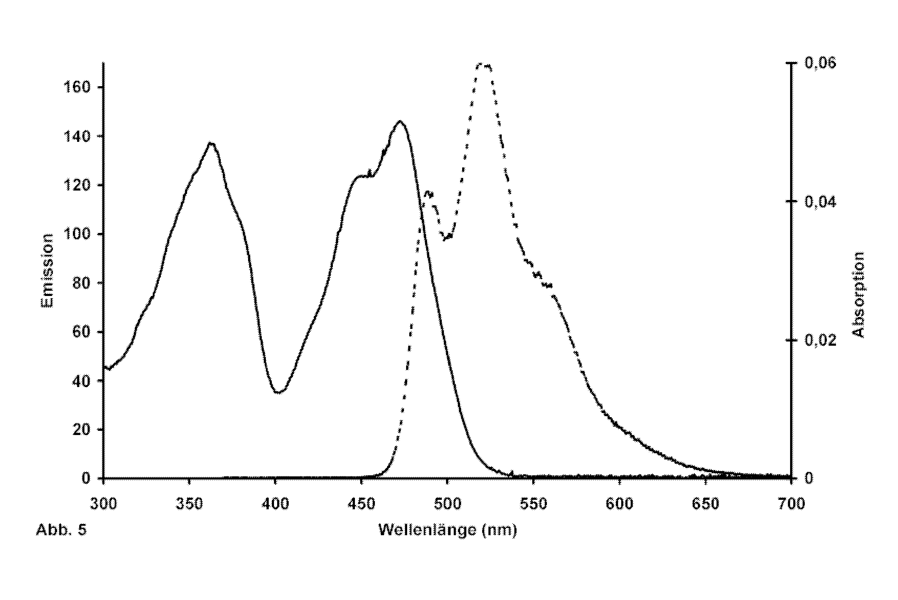

Abbildung 5 zeigt das Absorptionsspektrum (durchgezogene Linie) und Emissionsspektrum (unterbrochene Linie; Anregung bei 360 nm) des gereinigten Mehrfachchromophors If1 in Methylenchlorid.

### Beispiel 8: Mehrfachchromophor Ia5

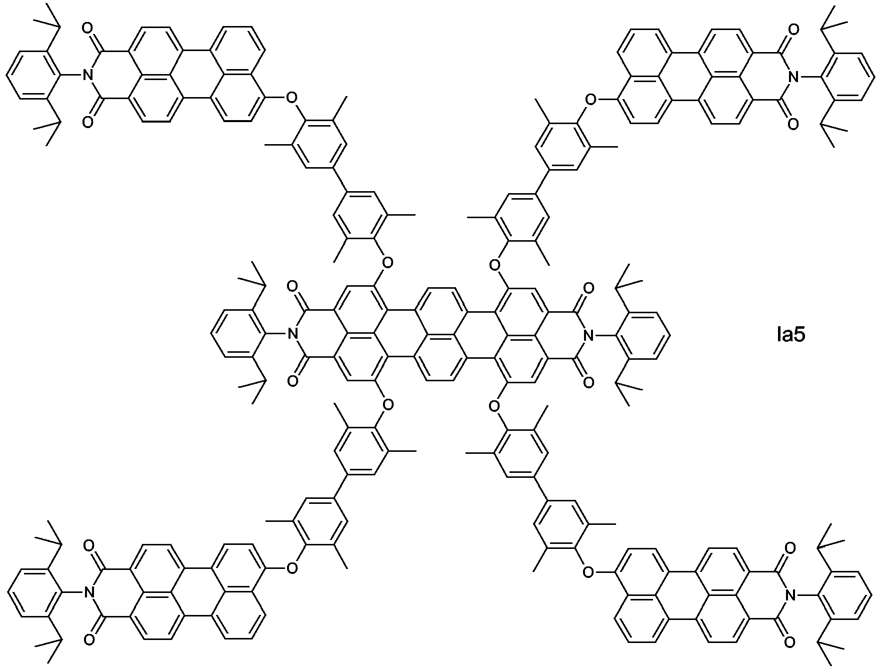

### a) Herstellung des Rylendicarbonsäureimidderivats 1114

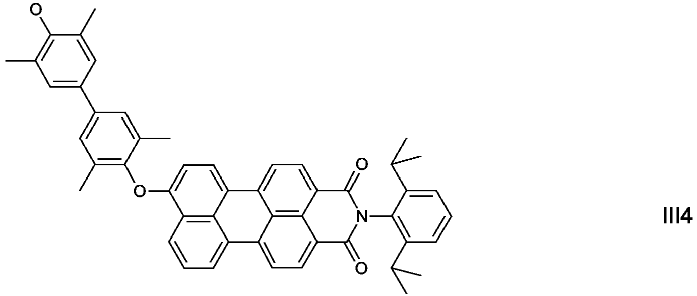

Eine Mischung aus 14,0 g (25 mmol) N-(2,6-Diisopropylphenyl)-9-bromperylen-3,4-dicarbonsäureimid, 8,65 g (62 mmol) Kaliumcarbonat, 24,9 g (100 mmol) 4,4'-Dihydroxy-3,3',5,5'-tetramethyldiphenyl und 200 ml N-Methylpyrrolidon wurde unter Stickstoff 14 h bei 110°C gerührt. Nach Zugabe von weiteren 3,0 g (22 mmol) Kaliumcarbonat wurde noch 1 h bei 140°C gerührt.

Nach Abkühlen auf Raumtemperatur wurde das Reaktionsgemisch auf 1 I 5 gew.-%ige Schwefelsäure gefällt. Das ausgefällte Produkt wurde abfiltriert, mit 80°C warmem Wasser gewaschen und im Vakuum bei 80°C getrocknet. Das getrocknete Produkt wurde in 400 ml Ethanol 1 h auf Rückflußtemperatur erhitzt und dann langsam auf Raumtemperatur abgekühlt.

Es wurden 11,5 g (64%) des Rylendicarbonsäureimidderivats III4 in Form eines violetten Feststoffs erhalten, der zur weiteren Reinigung einer Säulenchromatographie an Kieselgel mit Methylenchlorid als Eluens unterzogen wurde.

R_{f}-Wert (Methylenchlorid) = 0,18.

### b) Herstellung des Mehrfachchromophors Ia5

Eine Mischung aus 0,8 g (0,7 mmol) N,N'-Bis(2,6-diisopropylphenyl)-1,6,9,14-terrylen-3,4:11,12-tetracarbonsäuredümid, 2,0 g (2,8 mmol) des Rylendicarbonsäureimidderivats III4, 0,57 g (4 mmol) Kaliumcarbonat und 50 ml N-Methylpyrrolidon wurde unter Stickstoff 16 h bei 75°C gerührt. Nach weiterer Zugabe von 0,14 g (1 mmol) Kaliumcarbonat wurde weitere 16 h bei 75°C gerührt.

Nach Abkühlen auf Raumtemperatur wurde das Reaktionsgemisch auf 80 ml 5 gew.-%ige Schwefelsäure gefällt. Das ausgefällte Produkt wurde abfiltriert, nacheinander mit etwa 500 ml Wasser und 100 ml Ethanol gewaschen und im Vakuum bei 80°C getrocknet.

Es wurden 2,3 g (90%) des Mehrfachchromophors Ia5 in Form eines violetten Feststoffs erhalten, der zur weiteren Reinigung einer Säulenchromatographie an Kieselgel mit einem Toluol/Essigester-Gemisch (30:1) unterzogen wurde.

R_{f}-Wert (Toluol/Essigester 30:1) = 0,06;
Absorption:λₘₐₓ (Methylisobutyrat) = 520, 683 nm;
Emission: λₘₐₓ (Methylisobutyrat) = 702 nm.

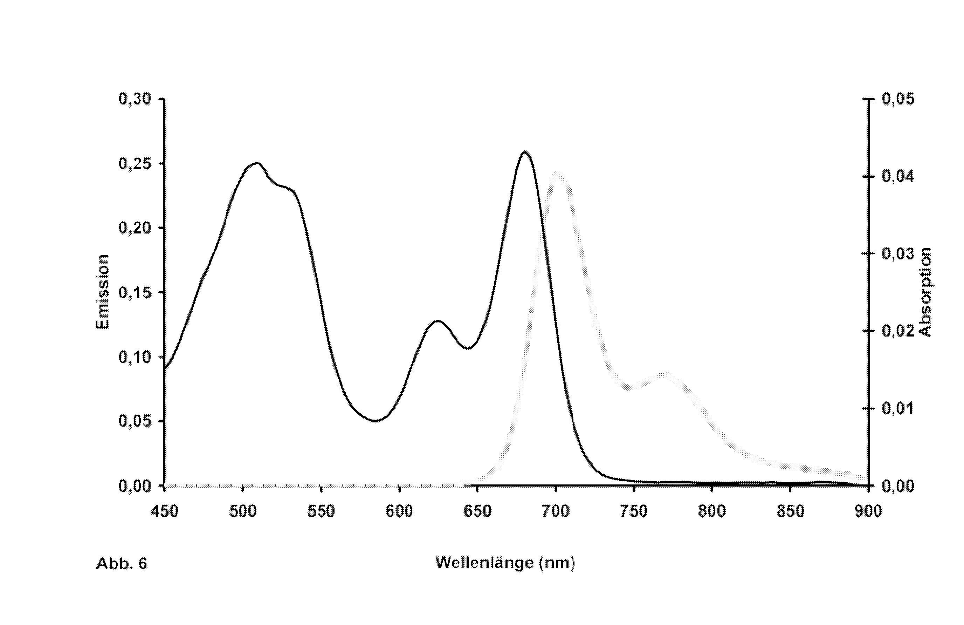

Abbildung 6 zeigt das Absorptionsspektrum (durchgezogene Linie) und Emissionsspektrum (graue Linie; Anregung bei 500 nm) des gereinigten Mehrfachchromophors Ia5 in Methylisobutyrat.

## Patentansprüche

1. Mehrfachchromophore auf Rylenbasis der allgemeinen Formel I' 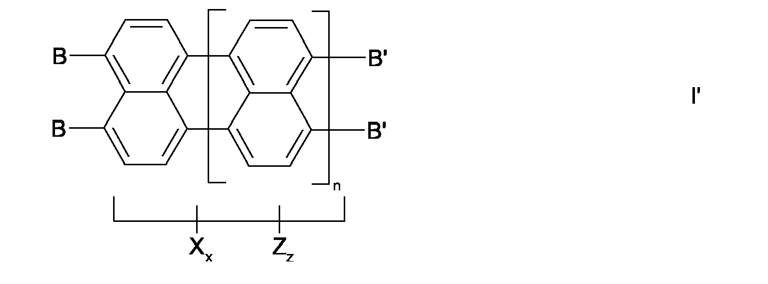 in der die Variablen folgende Bedeutung haben:
X ein Rylendicarbonsäureimidrest der Formel 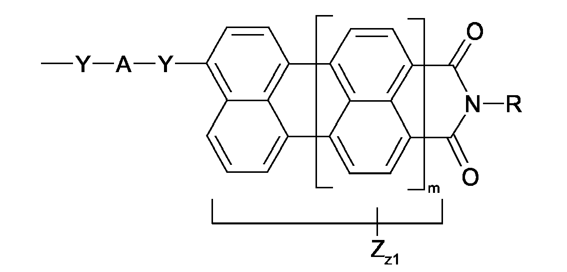 wobei die Reste X gleich oder verschieden sein können;
Z Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder -SO₂- unterbrochen sein kann, anneliert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO-und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² und/oder -SO₃R²;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder Hetaryl, das jeweils ein- oder mehrfach durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR² und/oder -SO₃R² substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂-bedeutet;
(v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² oder -SO₃R²,
wobei die Reste Z für z > 1 und/oder z1 > 1 gleich oder verschieden sein können;
B für n = 1, 2 oder 3:
miteinander unter Ausbildung eines Sechsrings verbunden zu einem Rest der Formel (a) oder (b) 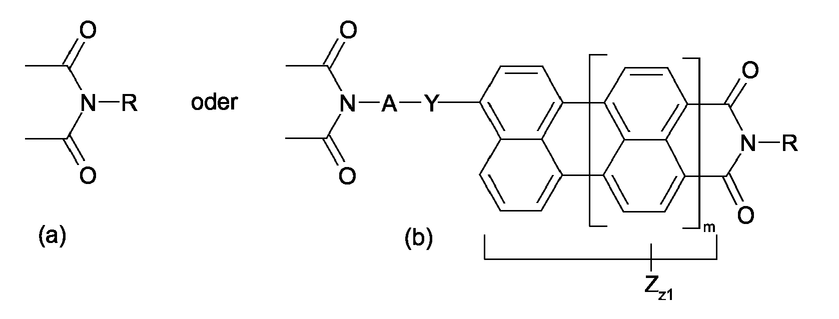 oder
ein Rest B Wasserstoff und der andere Rest einen Rest X; für n = 1 zusätzlich:
beide Wasserstoff, ein Rest B Wasserstoff und der andere Rest Z oder ein Rest B Wasserstoff, Halogen oder Cyano und der andere Rest einen Rest der Formel (c) 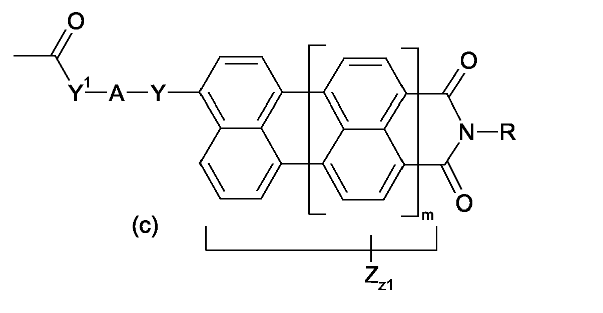
B' für n = 1, 2 oder 3:
miteinander unter Ausbildung eines Sechsrings verbunden zu einem Rest der Formel (a), wenn die Reste B zusammen einen Rest der Formel (a) bedeuten;
miteinander unter Ausbildung eines Sechsrings verbunden zu einem Rest der Formel (a), wenn ein Rest B Wasserstoff und der andere Rest einen Rest X bedeutet, wobei für n = 2 oder 3 gilt: x = 0 und z ≠ 0;
miteinander unter Ausbildung eines Sechsrings verbunden zu einem Rest der Formel (b), wenn die Reste B zusammen einen Rest der Formel (a)
oder (b) bedeuten oder ein Rest B Wasserstoff und der andere Rest einen Rest X oder Z bedeutet, wobei für n = 2 oder 3 gilt: x = 0 und z ≠ 0;
für n = 1 zusätzlich:
miteinander unter Ausbildung eines Sechsrings verbunden zu einem Rest der Formel (a) oder (b), wenn ein Rest B Wasserstoff und der andere Rest einen Rest Z bedeutet;
ein Rest B' Wasserstoff, Halogen oder Cyano und der andere Rest einen Rest der Formel (c), wenn ein Rest B Wasserstoff, Halogen oder Cyano und der andere Rest einen Rest der Formel (c) bedeutet;
A ein mindestens einen aromatischen oder hetaromatischen Rest aufweisendes Brückenglied, wobei die Gruppen Y oder Y und Y¹ an den aromatischen oder hetaromatischen Rest gebunden sind;
Y -O- oder -S-;
Y¹ -O-, -S- oder -NR¹-;
R Wasserstoff;
C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach durch die als Substituenten für die Reste Z genannten Reste (ii), (iii), (iv) und/oder (v) substituiert sein kann;
C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach durch die als Substituenten für die Reste Z genannten Reste (i), (ii), (iii), (iv) und/oder (v) substituiert sein kann; Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch die als Substituenten für die Reste Z genannten Reste (i), (ii), (iii), (iv), (v) und/oder Aryl- und/oder Hetarylazo, das jeweils ein- oder mehrfach durch C₁-C₁₀-Alkyl, C₁-C₆-Alkoxy und/oder Cyano substituiert sein kann, wobei die Reste R gleich oder verschieden sein können, wenn sie mehrfach in Formel I auftreten;
R¹ Wasserstoff oder C₁-C₁₈-Alkyl, wobei die Reste R¹ gleich oder verschieden sein können, wenn sie mehrfach auftreten;
R², R³ unabhängig voneinander Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR¹ substituiert sein kann; Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann;
n 1, 2 oder 3;
m 0, 1 oder 2;
x für n = 1:
2 bis 4 oder auch 0, wenn die Reste B und die Reste B' jeweils einen Rest der Formel (b) oder ein Rest B und ein Rest B' jeweils einen Rest der Formel (c) bedeuten;
für n = 2:
2 bis 6, wenn die Reste B und die Reste B' jeweils einen Rest der Formel (a) bedeuten;
0 für alle weiteren Bedeutungen der Reste B und B';
für n = 3:
2 bis 4, wenn die Reste B und die Reste B' jeweils einen Rest der Formel (a) bedeuten;
0 für alle weiteren Bedeutungen der Reste B und B';
z 0 bis 8, wobei x + z ≤ 8 ist und für n = 2 oder 3 gilt: z ≠0, wenn x = 0 ist;
z1 für m = 0: 0;
für m = 1: 0 bis 2;
für m = 2: 2 bis 4.

2. Mehrfachchromophore der allgemeinen Formel I' nach Anspruch 1, in der die Variablen folgende Bedeutung haben:
Z Phenoxy oder Thiophenoxy, das jeweils ein- oder mehrfach durch gleiche oder verschiedene Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -C≡C-, -CR¹=CR¹- und/oder -CO- und/oder -SO₂- unterbrochen sein kann und das ein- oder mehrfach substituiert sein kann durch: C₁-C₁₂-Alkoxy, Hydroxy, Halogen, Cyano und/oder Aryl, das ein- oder mehrfach durch C₁-C₁₈-Alkyl und/oder C₁-C₆-Alkoxy substituiert sein kann;
(ii) C₃-C₈-Cycloalkyl, dessen Kohlenstoffgerüst durch eine oder mehrere Gruppierungen -O-, -S-, -NR¹-, -CR¹=CR¹- und/oder -CO- unterbrochen sein kann und das ein- oder mehrfach durch C₁-C₁₈-Alkyl, C₁-C₁₂-Alkoxy und/oder C₁-C₆-Alkylthio substituiert sein kann;
(iii) Aryl oder Hetaryl, an das jeweils weitere 5- bis 7-gliedrige gesättigte oder ungesättigte Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, -C=CR¹-, -CR¹=CR¹-, Hydroxy, Halogen, Cyano, -NR²R³, -NR²COR³, -CONR²R³, -SO₂NR²R³, -COOR², -SO₃R², Aryl und/oder Hetaryl, das jeweils ein- oder mehrfach durch C₁-C₁₈-Alkyl, C₁-C₁₈-Alkoxy und/oder Cyano substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂-bedeutet;
(v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² oder -SO₃R²,
wobei die Reste Z für z > 1 und/oder z1 > 1 gleich oder verschieden sein können;
A ein Arylen- oder Hetarylenrest der Formeln 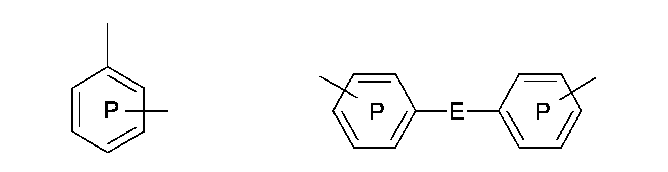 in denen die Ringe P gleich oder verschieden sein können, Heteroatome als Ringatome enthalten können und/oder annelierte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, aufweisen können, wobei das gesamte Ringsystem ein- oder mehrfach durch die als Substituenten für die Reste Z genannten Reste (i), (ii), (iii) und/oder (v) substituiert sein kann,
wobei die Reste A gleich oder verschieden sein können, wenn sie mehrfach in Formel I auftreten;
E eine chemische Bindung, eine Gruppierung -O-, -S-, -NR¹-, -N=CR¹-, -C≡C-, -CR¹=CR¹-, -CO-, -SO- oder -SO₂- oder C₁-C₁₂-Alkylen oder C₄-C₇-Cycloalkylen, dessen Kohlenstoffkette jeweils ein- oder mehrfach durch diese Gruppierungen unterbrochen sein kann und das jeweils ein- oder mehrfach durch die als Substituenten für die Reste Z genannten Reste (i), (ii), (iii) und/oder (v) substituiert sein kann;
Arylen oder Hetarylen, das jeweils ein- oder mehrfach durch die als Substituenten für die Reste Z genannten Reste (i), (ii), (iii) und/oder (v) substituiert sein kann, wobei Hydroxy und Mercapto als Reste (v) ausgeschlossen sind;
Y -O-;
Y¹ -O- oder -NR¹-;
R C₁-C₃₀-Alkyl, dessen Kohlenstoffkette durch eine oder mehrere Gruppierungen -O- und/oder -CO- unterbrochen sein kann und das ein- oder mehrfach substituiert sein kann durch: C₁-C₆-Alkoxy, Cyano und/oder Aryl, das ein- oder mehrfach durch C₁-C₁₈-Alkyl und/oder C₁-C₆-Alkoxy substituiert sein kann;
Phenyl, Naphthyl, Pyridyl oder Pyrimidyl, das jeweils ein- oder mehrfach substituiert sein kann durch: C₁-C₁₈-Alkyl, C₁-C₆-Alkoxy, Halogen, Cyano, Nitro, -CONR²R³, -SO₂NR²R³ und/oder Phenyl- und/oder Naphthylazo, das jeweils ein- oder mehrfach durch C₁-C₁₀-Alkyl, C₁-C₆-Alkoxy und/oder Cyano substituiert sein kann;
C₅-C₈-Cycloalkyl, das ein- oder mehrfach durch C₁-C₆-Alkyl substituiert sein kann,
wobei die Reste R gleich oder verschieden sein können, wenn sie mehrfach in Formel I auftreten;
R¹ Wasserstoff oder C₁-C₆-Alkyl;
R², R³ unabhängig voneinander Wasserstoff;
C₁-C₁₈-Alkyl, das ein- oder mehrfach durch C₁-C₆-Alkoxy, Hydroxy, Halogen und/oder Cyano substituiert sein kann;
Aryl oder Hetaryl, das jeweils ein- oder mehrfach durch C₁-C₆-Alkyl
und/oder die vorstehenden, als Substituenten für Alkyl genannten Reste substituiert sein kann;
n 1 oder 2;
m 0 oder 1, wobei m = n - 1 ist;
x für n = 1:
2 bis 4 oder auch 0, wenn die Reste B und die Reste B' jeweils einen Rest der Formel (b) oder ein Rest B und ein Rest B' jeweils einen Rest der Formel (c) bedeuten;
für n = 2:
2 bis 4, wenn die Reste B und die Reste B' jeweils einen Rest der Formel (a) bedeuten;
0 für alle weiteren Bedeutungen der Reste B und B';
z 0 bis 4, wobei x + z ≤ 4 ist, wobei für n = 2 gilt: z ≠ 0, wenn x = 0 ist;
z1 für m = 0: 0;
für m = 1: 0 bis 2.

3. Mehrfachchromophore der allgemeinen Formel I' nach Anspruch 1, in der die Variablen folgende Bedeutung haben:
Z Phenoxy, das ein- oder mehrfach durch gleiche oder verschiedene Reste (i), (ii), (iii), (iv) und/oder (v) substituiert sein kann:
(i) C₁-C₁₈-Alkyl, dessen Kohlenstoffkette durch eine oder mehrere Gruppierungen -O-, -S-, -NR¹- und/oder -CO- unterbrochen sein kann und das ein- oder mehrfach durch C₁-C₁₂-Alkoxy, Hydroxy und/oder Halogen substituiert sein kann;
(ii) C₃-C₈-Cycloalkyl, das ein- oder mehrfach durch C₁-C₁₈-Alkyl und/oder C₁-C₁₂-Alkoxy substituiert sein kann;
(iii) Aryl oder Hetaryl, das ein- oder mehrfach durch C₁-C₁₈-Alkyl, C₁-C₁₂-Alkoxy, Hydroxy und/oder Halogen substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S- oder -NR¹- bedeutet;
(v) C₁-C₁₂-Alkoxy, Hydroxy, Halogen oder Cyano;
B, B' jeweils miteinander verbunden zu einem Rest der Formel (a) oder (b);
A ein Arylenrest der Formeln 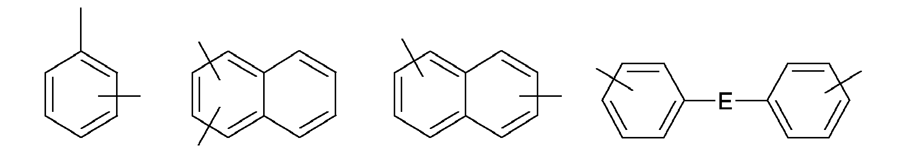 in denen die Phenylen- oder Naphthylenringe ein- oder mehrfach durch C₁-C₁₈-Alkyl substituiert sein können,
wobei die Reste A gleich oder verschieden sein können, wenn sie mehrfach in Formel I auftreten;
E eine chemische Bindung, Methylen oder Isopropylen;
Y, Y¹ -O-;
R gleiche Reste:
C₁-C₃₀-Alkyl, dessen Kohlenstoffkette durch eine oder mehrere Gruppierungen -O- und/oder -CO- unterbrochen sein kann und das ein- oder mehrfach substituiert sein kann durch: C₁-C₆-Alkoxy, Cyano und/oder Aryl, das ein- oder mehrfach durch C₁-C₁₈-Alkyl und/oder C₁-C₆-Alkoxy substituiert sein kann;
Phenyl, Naphthyl, Pyridyl oder Pyrimidyl, das jeweils ein- oder mehrfach substituiert sein kann durch: C₁-C₁₈-Alkyl, C₁-C₆-Alkoxy, Halogen, Cyano, Nitro, -CONR²R³, -SO₂NR²R³ und/oder Phenyl- und/oder Naphthylazo,
das jeweils ein- oder mehrfach durch C₁-C₁₀-Alkyl, C₁-C₆-Alkoxy und/oder Cyano substituiert sein kann;
C₅-C₈-Cycloalkyl, das ein- oder mehrfach durch C₁-C₆-Alkyl substituiert sein kann;
R¹ Wasserstoff oder C₁-C₆-Alkyl;
R², R³ unabhängig voneinander Wasserstoff;
C₁-C₁₈-Alkyl, das ein- oder mehrfach durch C₁-C₆-Alkoxy, Hydroxy, Halogen und/oder Cyano substituiert sein kann;
Aryl oder Hetaryl, das jeweils ein- oder mehrfach durch C₁-C₆-Alkyl
und/oder die vorstehenden, als Substituenten für Alkyl genannten Reste substituiert sein kann;
n 1 oder 2;
m 0 oder 1, wobei m = n - 1 ist;
x für n = 1:
2 bis 4 oder auch 0, wenn die Reste B und die Reste B' jeweils einen Rest der Formel (b) bedeuten;
für n = 2:
2 bis 4, wenn die Reste B und die Reste B' jeweils einen Rest der Formel (a) bedeuten;
0, wenn die Reste B und die Reste B' jeweils einen Rest der Formel (b) bedeuten;
z 0 bis 4, wobei x + z ≤ 4 ist;
z1 0.

4. Verfahren zur Herstellung von Mehrfachchromophoren auf Basis von Rylentetracarbonsäurediimiden der allgemeinen Formel la 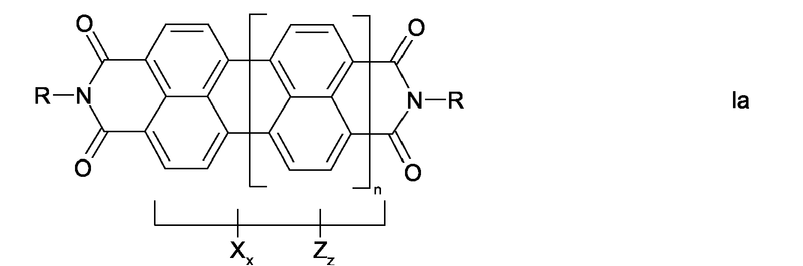 in der die Variablen die in Aspruch 1 genannte Bedeutung haben, **dadurch gekennzeichnet, daß** man ein halogeniertes der allgemeinen Formel IIa 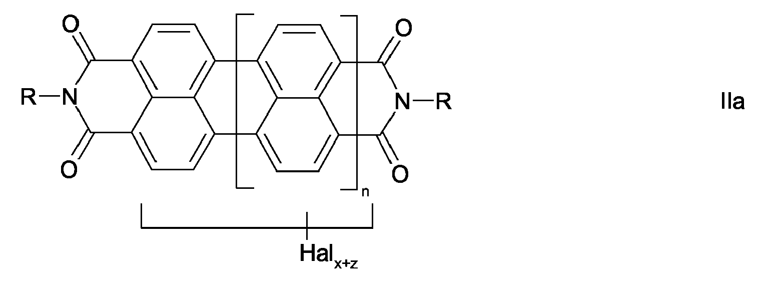 in der Hal Chlor oder Brom bedeutet, in Gegenwart einer Base und eines nichtnucleophilen Lösungsmittels
a) mit 0,8 bis 1,2 mol pro mol auszutauschendes Halogenatom eines Rylendicarbonsäureimidderivats der allgemeinen Formel IIIa 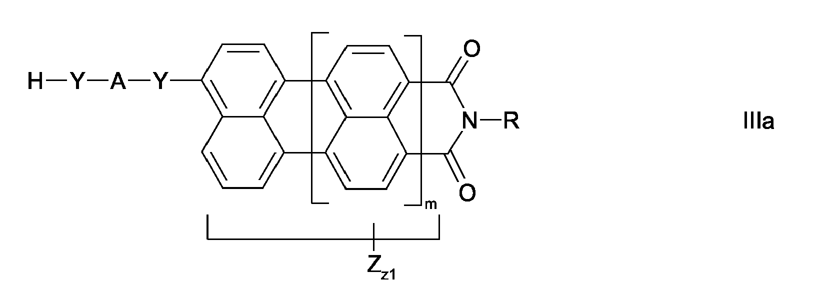 in der die Variablen die in Anspruch 2 genannte Bedeutung haben, zu einem Rylentetracarbonsäurediimid der Formel Ia, in der z = 0 ist, umsetzt oder
b) zunächst mit 0,8 bis 1,2 mol pro mol auszutauschendes Halogenatom eines Rylendicarbonsäureimidderivats der Formel IIIa und dann mit 0,8 bis 3 mol pro mol weiteres auszutauschendes Halogenatom einer Verbindung der allgemeinen Formel IV
H-Z IV
oder zunächst mit 0,8 bis 1,2 mol pro mol auszutauschendes Halogenatom einer Verbindung der Formel IV und dann mit 0,8 bis 1,2 mol pro mol weiteres auszutauschendes Halogenatom eines Rylendicarbonsäureimidderivats der Formel IIIa
oder gleichzeitig mit jeweils 0,8 bis 1,2 mol pro mol auszutauschendes Halogenatom des Rylendicarbonsäureimidderivats der Formel IIIa und der Verbindung der Formel IV zu einem Rylentetracarbonsäurediimid der Formel la, in der z ≠ 0 ist, umsetzt.

5. Verfahren zur Herstellung von Mehrfachchromophoren auf Basis von Rylendicarbonsäureimiden der allgemeinen Formel Ib 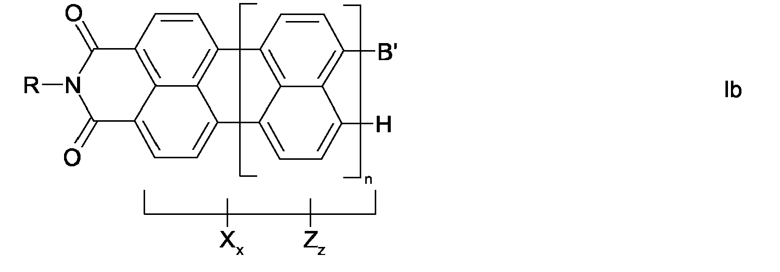 in der n = 1 ist, B' Wasserstoff, einen Rest X oder einen Rest Z bedeutet und die weiteren Variablen die in Anspruch 1 genannte Bedeutung haben, **dadurch gekennzeichnet, daß** man ein halogeniertes Rylendicarbonsäureimid der allgemeinen Formel IIb 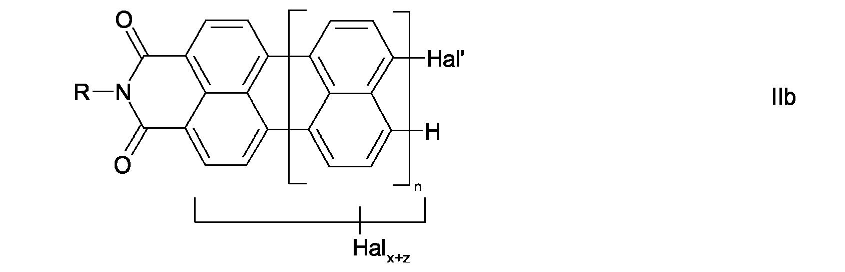 in der n = 1 ist und Hal' Wasserstoff oder Hal bedeutet, wobei Hal für Chlor oder Brom steht, in Gegenwart einer Base und eines nichtnucleophilen Lösungsmittels
a) mit 0,8 bis 1,2 mol pro mol auszutauschendes Halogenatom eines Rylendicarbonsäureimidderivats der allgemeinen Formel IIIa 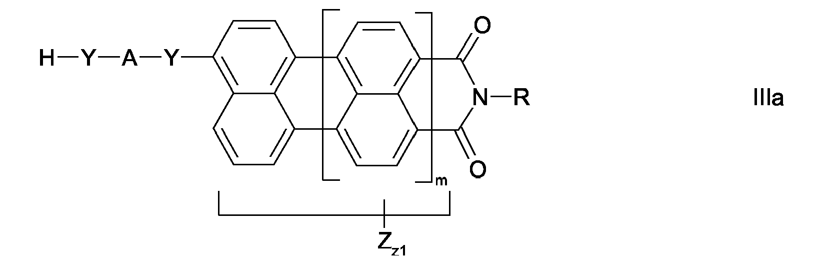 in der die Variablen die in Anspruch 2 genannte Bedeutung haben, zu einem Rylendicarbonsäureimid der Formel Ib, in der z = 0 ist, umsetzt
oder
b) zunächst mit 0,8 bis 1,2 mol pro mol auszutauschendes Halogenatom eines Rylendicarbonsäureimidderivats der Formel IIIa und dann mit 0,8 bis 3 mol pro mol weiteres auszutauschendes Halogenatom einer Verbindung der allgemeinen Formel IV
H-Z IV
oder zunächst mit 0,8 bis 1,2 mol pro mol auszutauschendes Halogenatom einer Verbindung der Formel IV und dann mit 0,8 bis 1,2 mol pro mol weiteres auszutauschendes Halogenatom eines Rylendicarbonsäureimidderivats der Formel IIIa
oder gleichzeitig mit jeweils 0,8 bis 1,2 mol pro mol auszutauschendes Halogenatom des Rylendicarbonsäureimidderivats der Formel IIIa und der Verbindung der Formel IV
zu einem Rylendicarbonsäureimid der Formel Ib, in der z # 0 ist, umsetzt.

6. Verfahren zur Herstellung von Mehrfachchromophoren auf Basis von Rylendicarbonsäureimiden der allgemeinen Formel Ib' 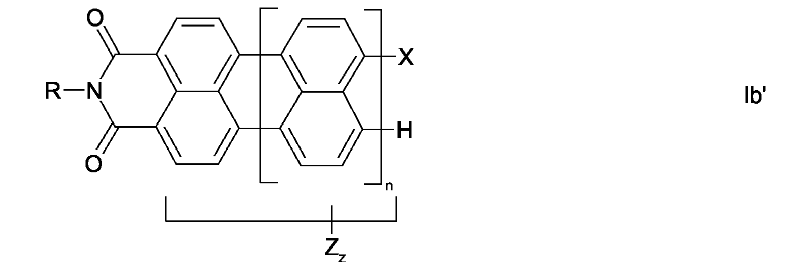 in der n = 2 oder 3 und z = 2 bis 8 ist und die weiteren Variablen die in Anspruch 1 genannte Bedeutung haben, **dadurch gekennzeichnet, daß** man ein peri-Halogenrylendicarbonsäureimid der allgemeinen Formel IIb' 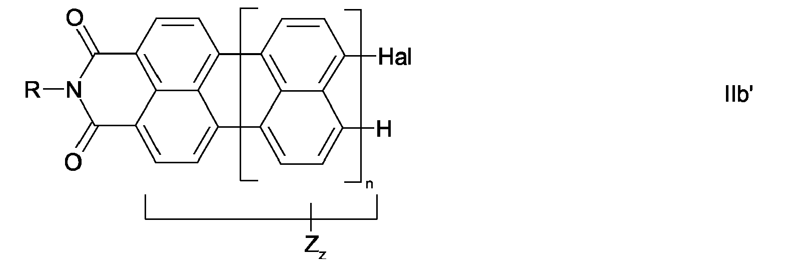 in der n = 2 oder 3 und z = 2 bis 8 ist und Hal Chlor oder Brom bedeutet, in Gegenwart einer Base und eines nichtnucleophilen Lösungsmittels mit 0,8 bis 1,2 mol pro mol eines Rylendicarbonsäureimidderivats der allgemeinen Formel IIIa 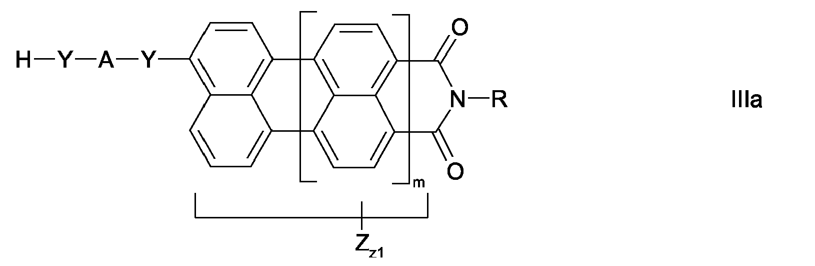 umsetzt.

7. Verfahren zur Herstellung von Mehrfachchromophoren auf Basis von Rylentetracarbonsäurediimiden der allgemeinen Formel Ic 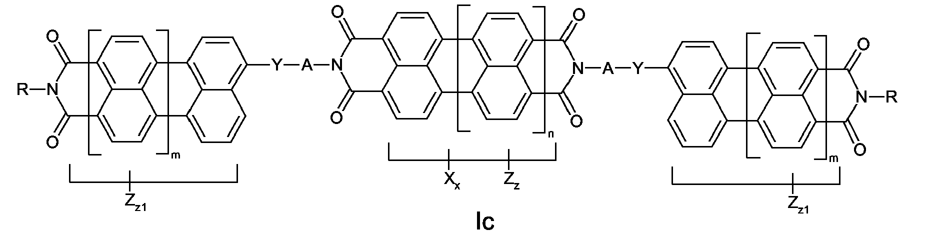 in der n = 1 ist, x 0 oder 2 bis 4 bedeutet und die weiteren Variablen die in Anspruch 1 genannte Bedeutung haben, **dadurch gekennzeichnet, daß** man ein gegebenenfalls halogeniertes Rylentetracarbonsäuredianhydrid der allgemeinen Formel IIc 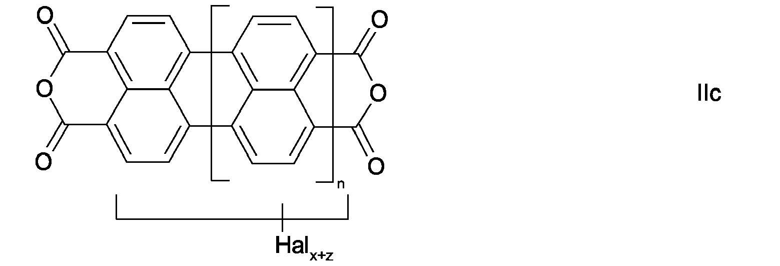 in der n = 1 ist und Hal Chlor oder Brom bedeutet, in Gegenwart eines hochsiedenden Lösungsmittels und gewünschtenfalls einer Lewis-Säure mit 1,8 bis 3 mol pro mol eines Rylendicarbonsäureimidderivats der allgemeinen Formel IIIc 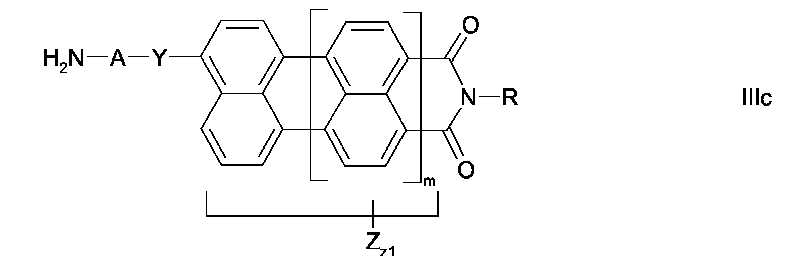 in der die Variablen die in Anspruch 2 genannte Bedeutung haben, zu einem Rylentetracarbonsäurediimid der Formel Ic, in der x = z = 0 ist, umsetzt
und gegebenenfalls das erhaltene im Rylenkern halogenierte Rylentetracarbonsäurediimid Ic nach Zwischenisolierung in Gegenwart einer Base und eines nichtnucleophilen Lösungsmittels
a) mit 0,8 bis 1,2 mol pro mol auszutauschendes Halogenatom eines Rylendicarbonsäureimidderivats der allgemeinen Formel IIIa 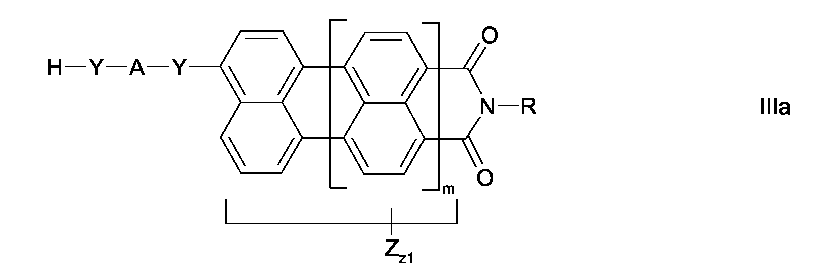 zu einem Rylentetracarbonsäurediimid der Formel Ic, in der x # 0 und z = 0 ist, umsetzt
oder
b) mit 0,8 bis 3 mol pro mol auszutauschendes Halogenatom einer Verbindung der allgemeinen Formel IV
H-Z IV
zu einem Rylentetracarbonsäurediimid der Formel Ic, in der z # 0 und x = 0 ist, umsetzt
oder
c) zunächst mit 0,8 bis 1,2 mol pro mol auszutauschendes Halogenatom eines Rylendicarbonsäureimidderivats der Formel IIIa und dann mit 0,8 bis 3 mol pro mol weiteres auszutauschendes Halogenatom einer Verbindung der Formel IV
oder zunächst mit 0,8 bis 1,2 mol pro mol auszutauschendes Halogenatom einer Verbindung der Formel Iv und dann mit 0,8 bis 1,2 mol pro mol weiteres auszutauschendes Halogenatom eines Rylendicarbonsäureimidderivats der Formel IIIa
oder gleichzeitig mit jeweils 0,8 bis 1,2 mol pro mol auszutauschendes Halogenatom des Rylendicarbonsäureimidderivats der Formel IIIa und der Verbindung der Formel IV
zu einem Rylentetracarbonsäurediimid der Formel Ic, in der x ≠ 0 und z ≠ 0 ist, umsetzt.

8. Verfahren zur Herstellung von Mehrfachchromophoren auf Basis von Rylendicarbonsäureimiden der allgemeinen Formel Id 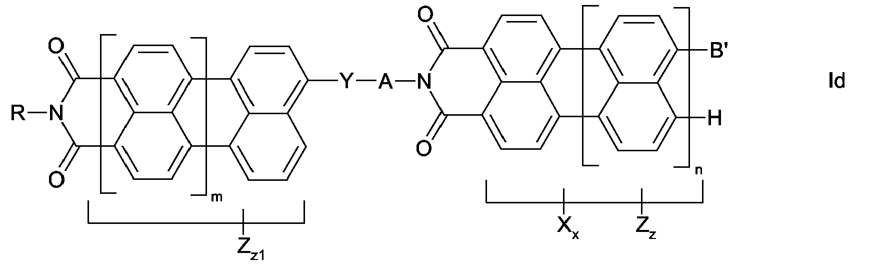 in der n = 1 ist, B' Wasserstoff, einen Rest X oder einen Rest Z bedeutet, x für 0 oder 2 bis 4 steht und die weiteren Variablen die in Anspruch 1 angegebene Bedeutung haben, **dadurch gekennzeichnet, daß** man ein gegebenenfalls halogeniertes Rylendicarbonsäureanhydrid der allgemeinen Formel IId 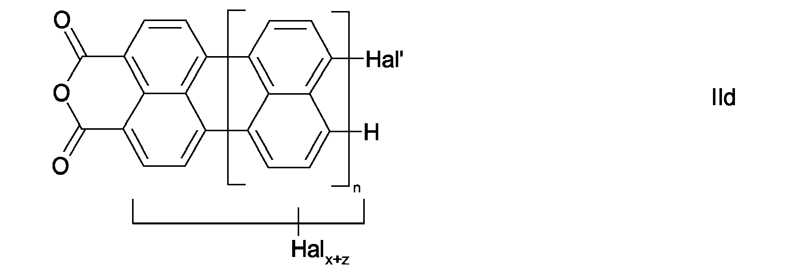 in der n = 1 ist und Hal' Wasserstoff oder Hal bedeutet, wobei Hal für Chlor oder Brom steht, in Gegenwart eines hochsiedenden Lösungsmittels und gewünschtenfalls einer Lewis-Säure mit 0,8 bis 1,2 mol pro mol eines Rylendicarbonsäureimidderivats der allgemeinen Formel IIIc 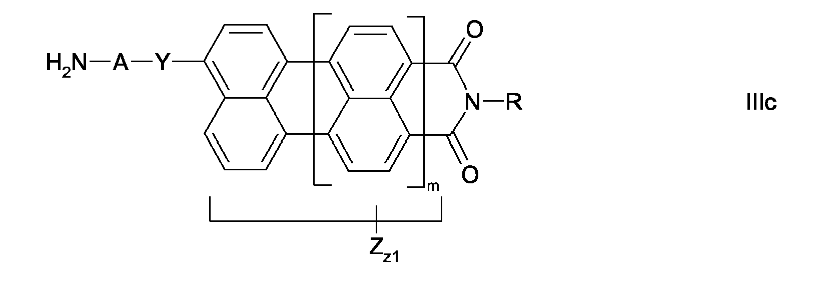 zu einem Rylendicarbonsäureimid der Formel Id, in der x = z = 0 ist, umsetzt
und gegebenenfalls das erhaltene im Rylenkern halogenierte Rylendicarbonsäureimid Id nach Zwischenisolierung in Gegenwart einer Base und eines nichtnucleophilen Lösungsmittels
a) mit 0,8 bis 1,2 mol pro mol auszutauschendes Halogenatom eines Rylendicarbonsäureimidderivats der allgemeinen Formel IIIa 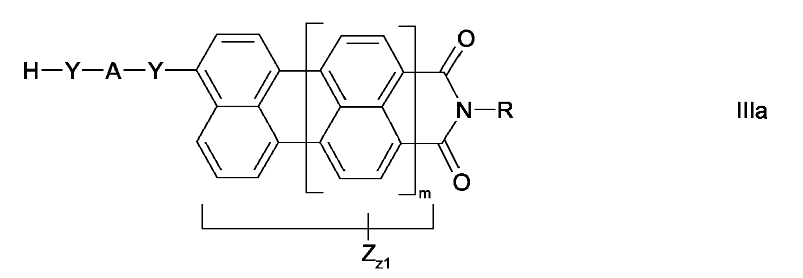 zu einem Rylendicarbonsäureimid der Formel Id, in der x ≠ 0 und z = 0 ist, umsetzt
oder
b) mit 0,8 bis 3 mol pro mol auszutauschendes Halogenatom einer Verbindung der allgemeinen Formel IV
H-Z IV
zu einem Rylendicarbonsäureimid der Formel Id, in der z ≠ 0 und x = 0 ist, umsetzt
oder
c) zunächst mit 0,8 bis 1,2 mol pro mol auszutauschendes Halogenatom eines Rylendicarbonsäureimidderivats der Formel IIIa und dann mit 0,8 bis 3 mol pro mol weiteres auszutauschendes Halogenatom einer Verbindung der Formel IV
oder zunächst mit 0,8 bis 1,2 mol pro mol auszutauschendes Halogenatom einer Verbindung der Formel IV und dann mit 0,8 bis 1,2 mol pro mol weiteres auszutauschendes Halogenatom eines Rylendicarbonsäureimidderivats der Formel IIIa
oder gleichzeitig mit jeweils 0,8 bis 1,2 mol pro mol auszutauschendes Halogenatom des Rylendicarbonsäureimidderivats der Formel IIIa und der Verbindung der Formel IV
zu einem Rylendicarbonsäureimid der Formel Id, in der x ≠ 0 und z ≠ 0 ist, umsetzt.

9. Verfahren zur Herstellung von Mehrfachchromophoren auf Basis von Rylentetracarbonsäurediimiden der allgemeinen Formel Ie 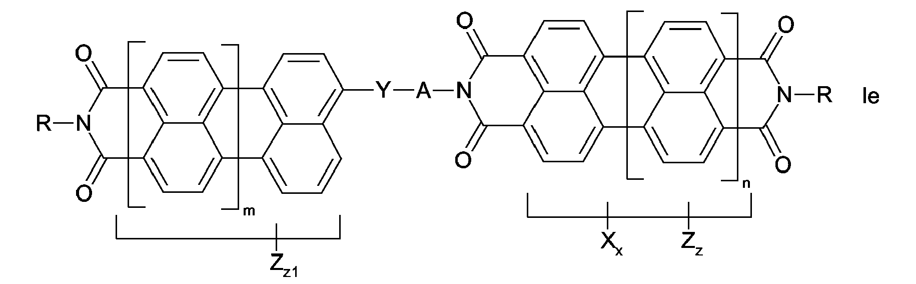 in der n = 1 ist und die weiteren Variablen die in Anspruch 1 genannte Bedeutung haben, **dadurch gekennzeichnet, daß** man ein gegebenenfalls halogeniertes Rylentetracarbonsäuremonoanhydridmonoimid der allgemeinen Formel IIe 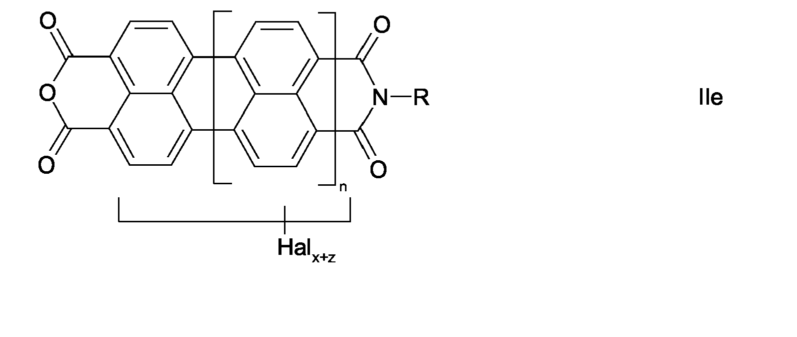 in der n = 1 ist und Hal Chlor oder Brom bedeutet, in Gegenwart eines hochsiedenden Lösungsmittels und gewünschtenfalls einer Lewis-Säure mit 0,8 bis 1,2 mol pro mol eines Rylendicarbonsäureimidderivats der allgemeinen Formel IIIc 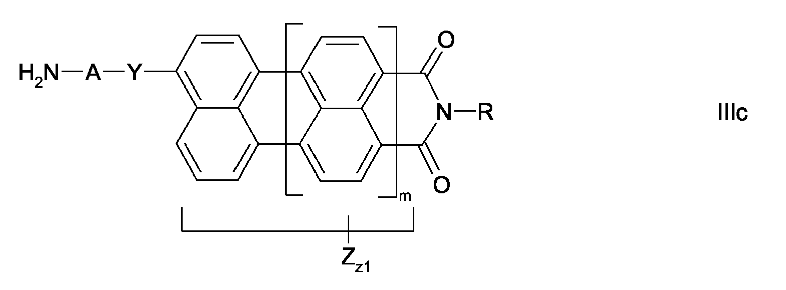 zu einem Rylentetracarbonsäurediimid der Formel le, in der x = z = 0 ist, umsetzt
und gegebenenfalls das erhaltene im Rylenkern halogenierte Rylendicarbonsäureimid le nach Zwischenisolierung in Gegenwart einer Base und eines nichtnucleophilen Lösungsmittels
a) mit 0,8 bis 1,2 mol pro mol auszutauschendes Halogenatom eines Rylendicarbonsäureimidderivats der allgemeinen Formel IIIa 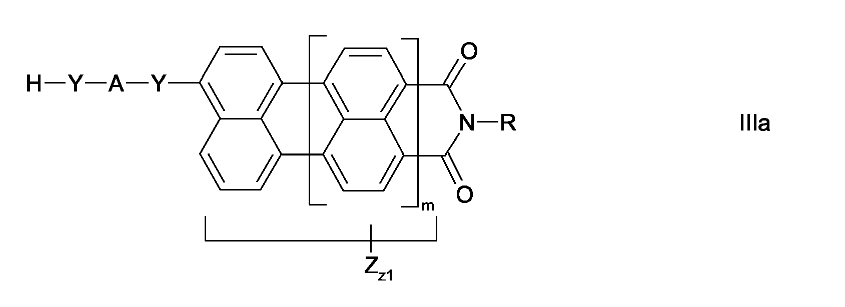 zu einem Rylentetracarbonsäurediimid der Formel le, in der x ≠ 0 und z = 0 ist, umsetzt
oder
b) mit 0,8 bis 3 mol pro mol auszutauschendes Halogenatom einer Verbindung der allgemeinen Formel IV
H-Z IV
zu einem Rylentetracarbonsäuredümid der Formel le, in der z ≠ 0 und x = 0 ist, umsetzt
oder
c) zunächst mit 0,8 bis 1,2 mol pro mol auszutauschendes Halogenatom eines Rylendicarbonsäureimidderivats der Formel IIIa und dann mit 0,8 bis 3 mol pro mol weiteres auszutauschendes Halogenatom einer Verbindung der Formel IV
oder zunächst mit 0,8 bis 1,2 mol pro mol auszutauschendes Halogenatom einer Verbindung der Formel IV und dann mit 0,8 bis 1,2 mol pro mol weiteres auszutauschendes Halogenatom eines Rylendicarbonsäureimidderivats der Formel IIIa
oder gleichzeitig mit jeweils 0,8 bis 1,2 mol pro mol auszutauschendes Halogenatom des Rylendicarbonsäureimidderivats der Formel IIIa und der Verbindung der Formel IV
zu einem Rylentetracarbonsäurediimid der Formel le, in der x # 0 und z # 0 ist, umsetzt.

10. Verfahren zur Herstellung von Mehrfachchromophoren auf Basis von Rylentetracarbonsäurediimiden der allgemeinen Formel Ic' 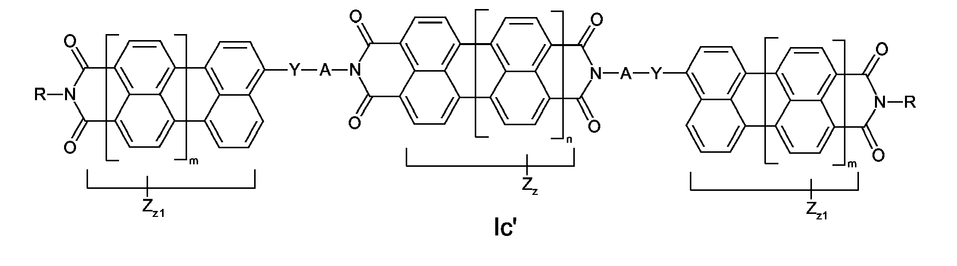 in der n = 2 oder 3 und z = 2 bis 8 ist und die weiteren Variablen die in Anspruch 1 genannte Bedeutung haben, **dadurch gekennzeichnet, daß** man ein Rylentetracarbonsäuredianhydrid der allgemeinen Formel IIc' 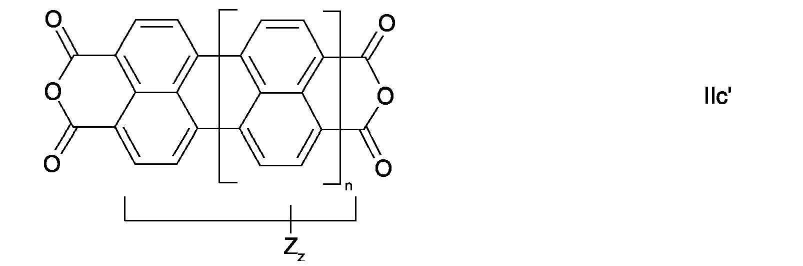 in der n = 2 oder 3 und z = 2 bis 8 ist, in Gegenwart eines hochsiedenden Lösungsmittels und einer Lewis-Säure mit 0,8 bis 1,2 mol pro mol eines Rylendicarbonsäureimidderivats der allgemeinen Formel IIIc 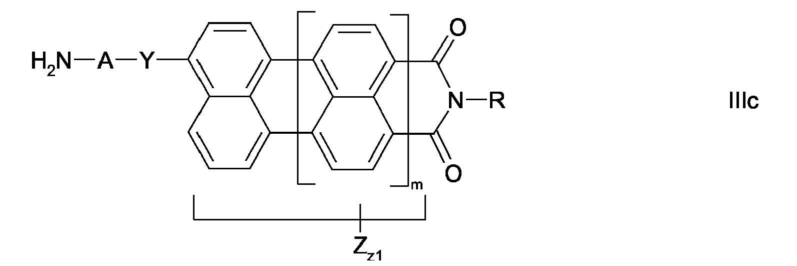 umsetzt.

11. Verfahren zur Herstellung von Mehrfachchromophoren auf Basis von Rylendicarbonsäureimiden der allgemeinen Formel Id' 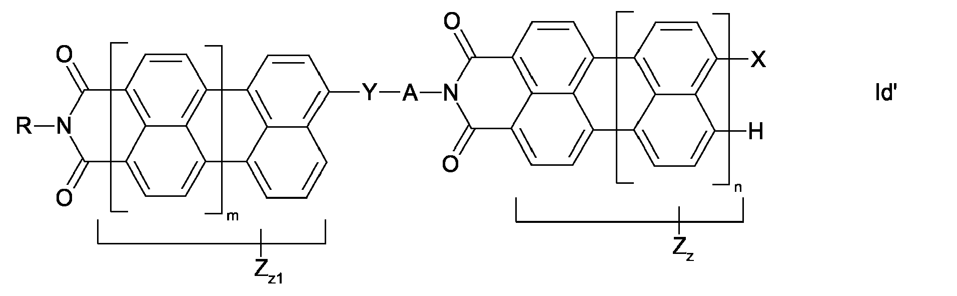 in der n = 2 oder 3 und z = 2 bis 8 ist und die weiteren Variablen die in Anspruch 1 genannte Bedeutung haben, **dadurch gekennzeichnet, daß** man ein peri-Halogenrylendicarbonsäureanhydrid der allgemeinen Formel IId' 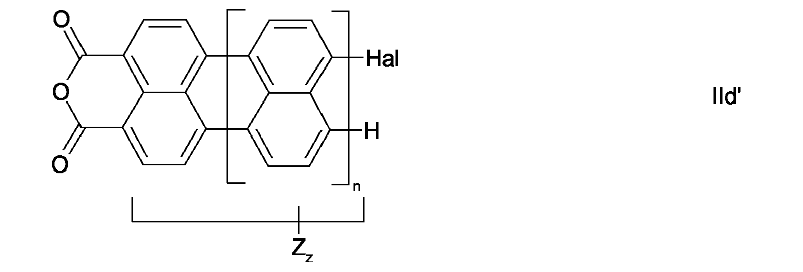 in der n = 2 oder 3 und z = 2 bis 8 ist und Hal Chlor oder Brom bedeutet, in Gegenwart eines hochsiedenden Lösungsmittels und einer Lewis-Säure mit 0,8 bis 1,2 mol pro mol eines Rylendicarbonsäureimidderivats der allgemeinen Formel IIIc 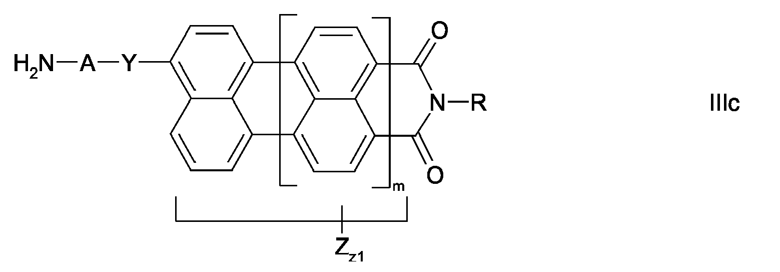 umsetzt.

12. Verfahren zur Herstellung von Mehrfachchromophoren auf Basis von Rylentetracarbonsäurediimiden der allgemeinen Formel le' 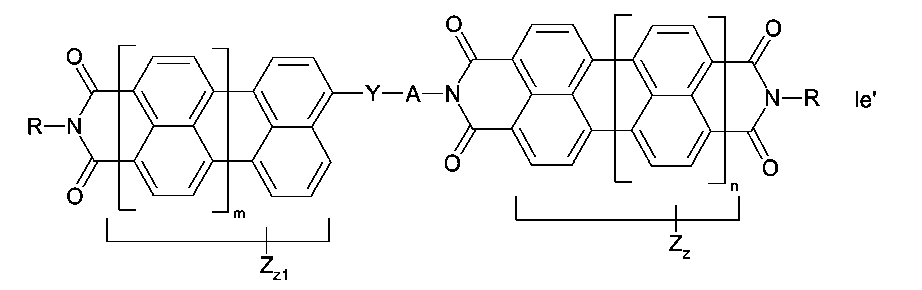 in der n = 2 oder 3 und z = 2 bis 8 ist und die weiteren Variablen die in Anspruch 1 genannte Bedeutung haben, **dadurch gekennzeichnet, daß** man ein Rylentetracarbonsäuremonoanhydridmonoimid der allgemeinen Formel Ile' 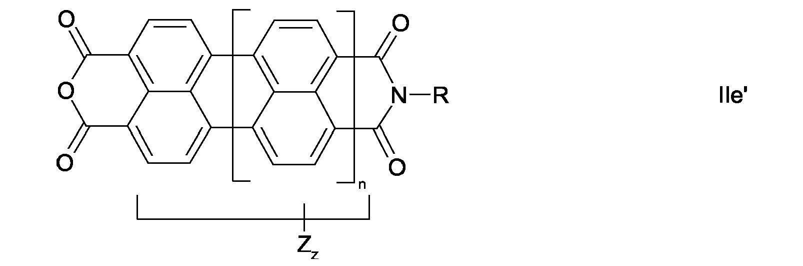 in der n = 2 bis 8 ist, in Gegenwart eines hochsiedenden Lösungsmittels und einer Lewis-Säure mit 0,8 bis 1,2 mol pro mol eines Rylendicarbonsäureimidderivats der allgemeinen Formel IIIc 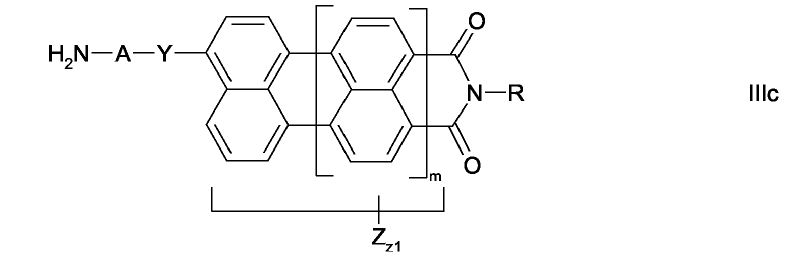 zu einem Rylentetracarbonsäurediimid der Formel le' umsetzt.

13. Verfahren zur Herstellung von Mehrfachchromophoren auf Basis von Rylendicarbonsäureestern der allgemeinen Formel If 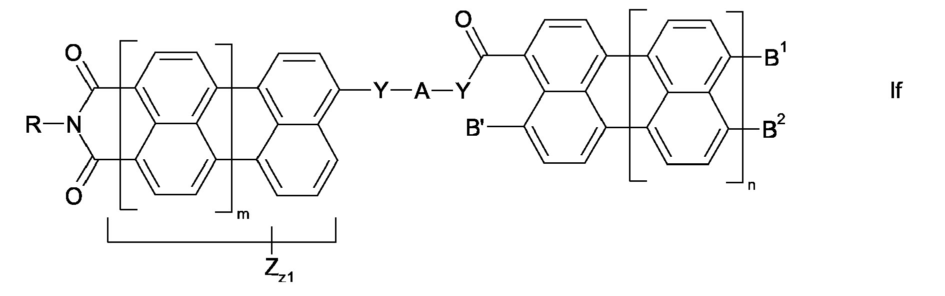 in der n = 1 ist, B' Wasserstoff, Halogen oder Cyano, einer der beiden Reste B¹ oder B² einen Rest der Formel 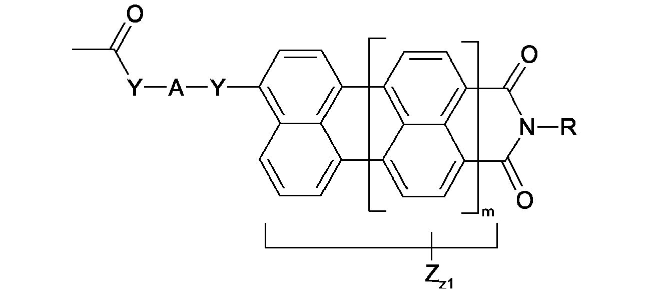 und der andere Rest Wasserstoff, Halogen oder Cyano bedeutet und die weiteren Variablen die in Anspruch 1 genannte Bedeutung haben, **dadurch gekennzeichnet, daß** man ein Rylendicarbonsäureimidderivat der allgemeinen Formel IIIa 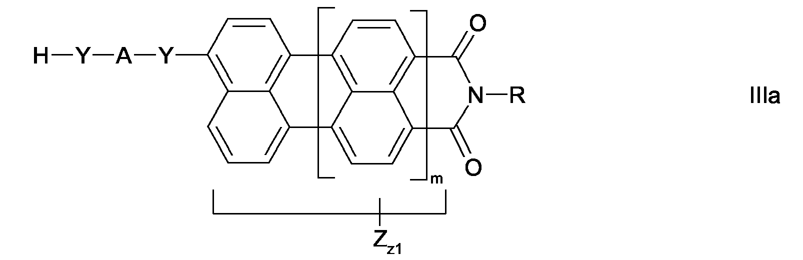 in Gegenwart einer Alkalimetallbase und eines aprotischen Lösungsmittels in das Alkalimetallsalz der allgemeinen Formel IIIf 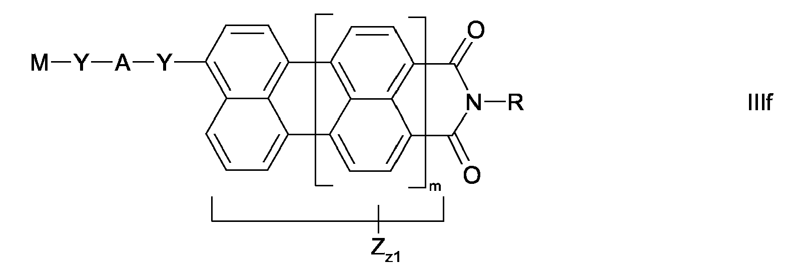 in der M ein Alkalimetallkation bedeutet, überführt und dieses dann mit einem Rylendicarbonsäurechlorid der allgemeinen Formel IIf 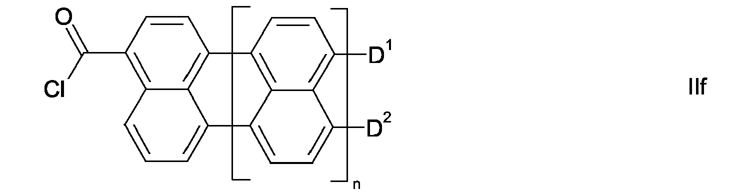 in der n = 1 ist und einer der beiden Reste D¹ oder D² -COCl oder der andere Rest Wasserstoff bedeutet, im Molverhältnis 0,8 : 1 bis 1,2 : 1 zu einem Rylendicarbonsäureester der Formel If, in der B' und der verbleibende Reste B¹ oder B² Wasserstoff bedeuten, umsetzt
und gewünschtenfalls den erhaltenen Rylendicarbonsäureester nach Zwischenisolierung in Gegenwart eines polaren organischen Lösungsmittels und einer Lewis-Säure mit N-Halogensuccinimid zum Rylendicarbonsäureester der Formel If, in der B' und der verbleibende Rest B¹ oder B² Halogen bedeuten, halogeniert
und gewünschtenfalls den erhaltenen halogenierten Rylendicarbonsäureester mit einem Metallcyanid in Gegenwart eines aprotischen Lösungsmittels und eines Übergangsmetallkatalysators in den entsprechenden cyanosubstituierten Rylendicarbonsäureester der Formel If, in der B' und der verbleibende Rest B¹ oder B² Cyano bedeuten, überführt.

14. Verfahren zur Herstellung von Mehrfachchromophoren auf Basis von Rylendicarbonsäureamiden der allgemeinen Formel Ig 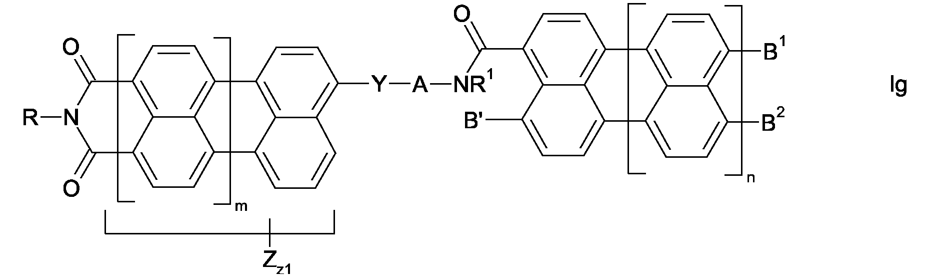 in der n = 1 ist, B' Wasserstoff, Halogen oder Cyano, einer der beiden Reste B¹ oder B² einen Rest der Formel 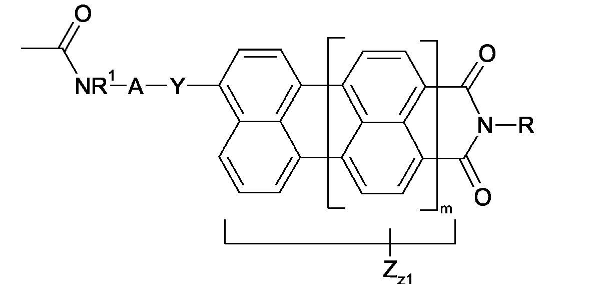 und der andere Rest Wasserstoff, Halogen oder Cyano bedeutet und die weiteren Variablen die in Anspruch 1 genannte Bedeutung haben, **dadurch gekennzeichnet, daß** man ein Rylendicarbonsäurechlorid der allgemeinen Formel IIf 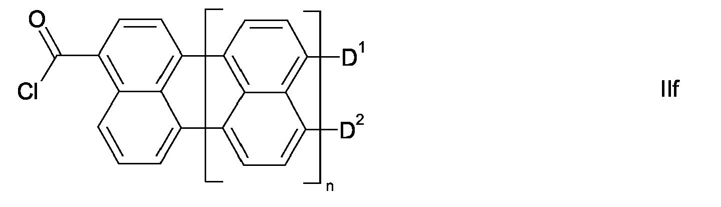 in der n = 1 ist und einer der beiden Reste D¹ oder D² -COCl oder der andere Rest Wasserstoff bedeutet, in Gegenwart einer nichtnucleophilen Base und gewünschtenfalls zusätzlich eines aprotischen Lösungsmittels mit 0,8 bis 1,2 mol pro mol eines Rylendicarbonsäureimidderivats der allgemeinen Formel IIIg 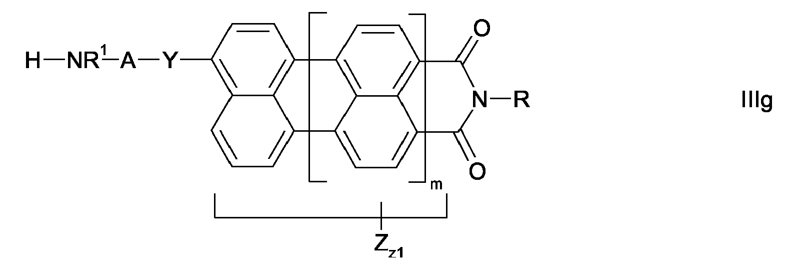 zu einem Rylendicarbonsäureamid der Formel Ig, in der B' und der verbleibende Rest B¹ oder B² Wasserstoff bedeuten, umsetzt
und gewünschtenfalls das erhaltene Rylendicarbonsäureamid nach Zwischenisolierung in Gegenwart eines polaren organischen Lösungsmittels und einer LewisSäure mit N-Halogensuccinimid zum Rylendicarbonsäureamid der Formel Ig, in der B' und der verbleibende Rest B¹ oder B² Halogen bedeuten, halogeniert und gewünschtenfalls das erhaltene halogenierte Rylendicarbonsäureamid mit einem Metallcyanid in Gegenwart eines aprotischen Lösungsmittels und eines Übergangsmetallkatalysators in das entsprechende cyanosubstituierte Rylendicarbonsäureamid der Formel Ig, in der B' und der verbleibende Rest B¹ oder B² Cyano bedeuten, überführt.

15. Rylendicarbonsäureimidderivate der allgemeinen Formel III 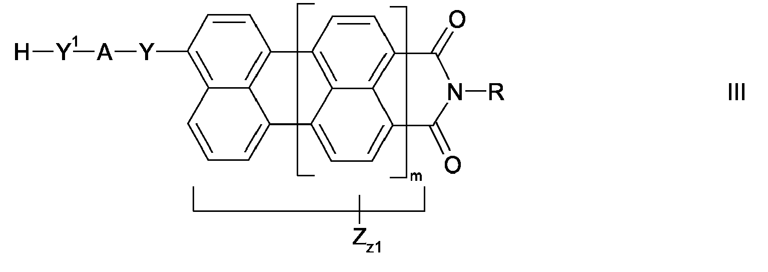 in der die Variablen folgende Bedeutung haben:
Z Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder -SO₂- unterbrochen sein kann, anneliert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -N=CR¹-, -C≡C-, -CR¹=CR1-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² und/oder -SO₃R²;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder Hetaryl, das jeweils ein- oder mehrfach durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR² und/oder -SO₃R² substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂-bedeutet;
(v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² oder -SO₃R²,
wobei die Reste Z für z > 1 und/oder z1 > 1 gleich oder verschieden sein können;
A ein mindestens einen aromatischen oder hetaromatischen Rest aufweisendes Brückenglied, wobei die Gruppen Y oder Y und Y¹ an den aromatischen oder hetaromatischen Rest gebunden sind;
Y -O- oder -S-;
Y¹ -O-, -S- oder -NR¹-;
R Wasserstoff;
C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach durch die als Substituenten für die Reste Z genannten Reste (ii), (iii), (iv) und/oder (v) substituiert sein kann;
C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach durch die als Substituenten für die Reste Z genannten Reste (i), (ii), (iii), (iv) und/oder (v) substituiert sein kann; Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch die als Substituenten für die Reste Z genannten Reste (i), (ii), (iii), (iv), (v) und/oder Aryl- und/oder Hetarylazo, das jeweils ein- oder mehrfach durch C₁-C₁₀-Alkyl, C₁-C₆-Alkoxy und/oder Cyano substituiert sein kann, wobei die Reste R gleich oder verschieden sein können, wenn sie mehrfach in Formel I auftreten;
R¹ Wasserstoff oder C₁-C₁₈-Alkyl, wobei die Reste R¹ gleich oder verschieden sein können, wenn sie mehrfach auftreten;
R², R³ unabhängig voneinander Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR¹ substituiert sein kann; Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann;
m 0, 1 oder 2;
z1 für m = 0: 0;
für m = 1: 0 bis 2;
für m = 2: 2 bis 4.

16. Verfahren zur Herstellung von Rylenderivaten der Formel III gemäß Anspruch 15,
**dadurch gekennzeichnet, daß** man ein peri-halogeniertes Rylendicarbonsäureimid der allgemeinen Formel V 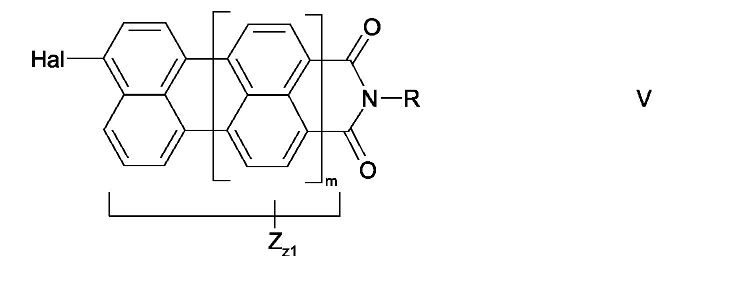 in der Hal Chlor oder Brom bedeutet, in Gegenwart einer Base und eines nichtnucleophilen Lösungsmittels mit 2 bis 5 mol pro mol auszutauschendes Halogenatom einer mindestens bifunktionellen aromatischen Verbindung der allgemeinen Formel VI
H-Y¹-A-Y-H VI
umsetzt.

17. Verwendung von Mehrfachchromophoren gemäß den Ansprüchen 1 bis 3 zur Einfärbung von organischen und anorganischen Materialien.

18. Verwendung nach Anspruch 17, **dadurch gekennzeichnet, daß** die organischen Materialien Lacke, Druckfarben oder Kunststoffe sind.

19. Verwendung von Mehrfachchromophoren gemäß den Ansprüchen 1 bis 3 zur Herstellung elektromagnetische Strahlung absorbierender und/oder emittierender wäßriger Polymerisatdispersionen.

20. Verwendung von Mehrfachchromophoren gemäß den Ansprüchen 1 bis 3 zur Erzeugung für das menschliche Auge nicht sichtbarer Markierungen und Beschriftungen.

21. Verwendung von Mehrfachchromophoren gemäß den Ansprüchen 1 bis 3 als Filter oder Emitter in Display-Anwendungen.

22. Verwendung von Mehrfachchromophoren gemäß den Ansprüchen 1 bis 3 als Emitter in Chemilumineszenzanwendungen.

23. Verwendung von Mehrfachchromophoren gemäß den Ansprüchen 1 bis 3 als Aktivkomponenten in der Photovoltaik.

## Claims

1. A rylene-based polychromophore of the general formula I' 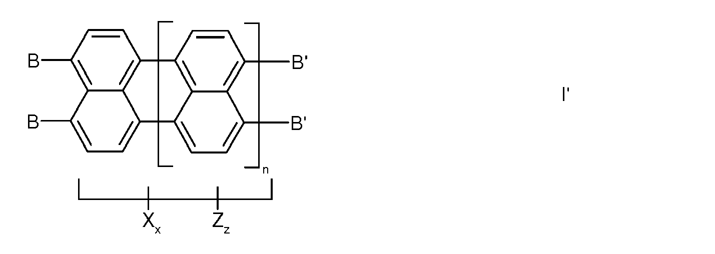 in which the variables are each defined as follows:
X is a rylenedicarboximide radical of the formula 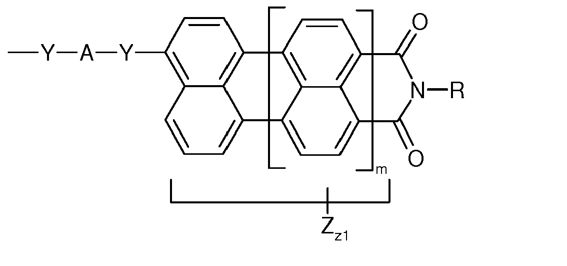 where the X radicals may be the same or different;
Z is aryloxy, arylthio, hetaryloxy or hetarylthio, to each of which may be fused further saturated or unsaturated 5- to 7-membered rings whose carbon skeleton may be interrupted by one or more -O-, -S-, -NR¹-, -N=CR¹-, -CO-, -SO- and/or -SO₂- moieties, where the entire ring system may be mono- or polysubstituted by the (i), (ii), (iii), (iv) and/or (v) radicals:
(i) C₁-C₃₀-alkyl whose carbon chain may be interrupted by one or more -O-, -S-, -NR¹-, -N=CR¹-, -C≡C-, -CR¹=CR¹-, -CO-, -SO- and/or -SO₂- moieties and which may be mono- or polysubstituted by: C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², aryl and/or saturated or unsaturated C₄-C₇-cycloalkyl whose carbon skeleton may be interrupted by one or more -O-, -S-, -NR¹-, -N=CR¹-, -CR¹=CR¹-, -CO-, -SO- and/or -SO₂-moieties, where the aryl and cycloalkyl radicals may each be mono- or polysubstituted by C₁-C₁₈-alkyl and/or the above radicals specified as substituents for alkyl;
(ii) C₃-C₈-cycloalkyl whose carbon skeleton may be interrupted by one or more -O-, -S-, -NR¹-, -N=CR¹-, -CR¹=CR¹-, -CO-, -SO- and/or -SO₂- moieties and to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR² and/or -SO₃R²;
(iii) aryl or hetaryl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², aryl and/or hetaryl, each of which may be mono- or polysubstituted by C₁-C₁₈-alkyl, C₁-C₁₂-alkoxy, hydroxyl, mercapto, halogen, cyano, nitro, -NR²R³, -NR²COR³, -CONR²R³, -SO₂NR²R³, -COOR² and/or -SO₃R²;
(iv) a -U-aryl radical which may be mono- or polysubstituted by the above radicals specified as substituents for the aryl radicals (iii), where U is an -O-, -S-, -NR¹-, -CO-, -SO- or -SO₂- moiety;
(v) C₁-C₁₂-alkoxy, C₁-C₆-alkylthio, -C≡CR¹, -CR¹=CR¹², hydroxyl, mercapto, halogen, cyano, nitro, -NR²R³, -NR²COR³, -CONR²R³, -SO₂NR²R³, -COOR² or -SO₃R²,
where the Z radicals may be the same or different when z > 1 and/or z1 > 1;
B' when n = 1, 2 or 3:
are joined together with formation of a six-membered ring to give a radical of the formula (a) or (b) 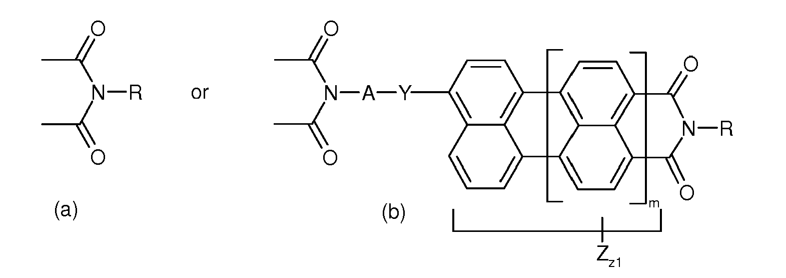 or
one B radical is hydrogen and the other radical is an X radical;
when n = 1, additionally:
both are hydrogen, one B radical is hydrogen and the other radical is Z, or
one B radical is hydrogen, halogen or cyano and the other radical is a radical of the formula (c) 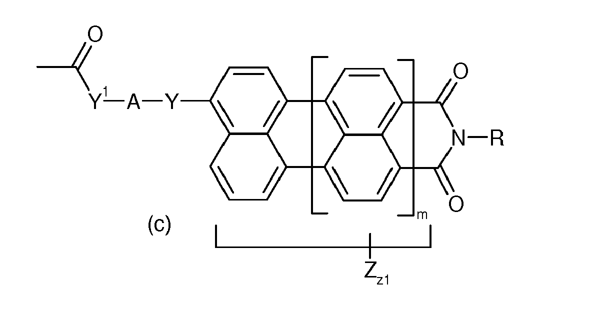
B', when n = 1, 2 or 3:
are joined to one another with formation of a six-membered ring to give a radical of the formula (a) when the B radicals together are a radical of the formula (a);
are joined together with formation of a six-membered ring to give a radical of the formula (a) when one B radical is hydrogen and the other radical is an X radical, where, when n = 2 or 3, x = 0 and z ≠ 0;
are joined together with formation of a six-membered ring to give a radical of the formula (b) when the B radicals together are a radical of the formula (a) or (b), or one B radical is hydrogen and the other radical is an X or Z radical, where, when n = 2 or 3, x = 0 and z ≠ 0;
when n = 1, additionally:
are joined together with formation of a six-membered ring to give a radical of the formula (a) or (b) when one B radical is hydrogen and the other radical is a Z radical;
one B' radical is hydrogen, halogen or cyano and the other radical is a radical of the formula (c) when one B radical is hydrogen, halogen or cyano and the other radical is a radical of the formula (c);
A is a bridging member having at least one aromatic or heteroaromatic radical, where the Y or Y and Y¹ groups are bonded to the aromatic or heteroaromatic radical;
Y is -O- or -S-;
Y¹ is -O-, -S- or -NR¹-;
R is hydrogen;
C₁-C₁₀-alkyl whose carbon chain may be interrupted by one or more -O-, -S-, -NR¹-, -N=CR¹-, -C≡C-, -CR¹=CR¹-, -CO-, -SO- and/or -SO₂- moieties and which may be mono- or polysubstituted by the (ii), (iii), (iv) and/or (v) radicals specified as substituents for the Z radicals;
C₃-C₈-cycloalkyl to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by the (i), (ii), (iii), (iv) and/or (v) radicals specified as substituents for the Z radicals;
aryl or hetaryl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by the (i), (ii), (iii), (iv), (v) radicals specified as substituents for the Z radicals, and/or aryl- and/or hetarylazo, each of which may be mono- or polysubstituted by C₁-C₁₀-alkyl, C₁-C₆- alkoxy and/or cyano;
where the R radicals may be the same or different when they occur repeatedly in formula I;
R¹ is hydrogen or C₁-C₁₈-alkyl, where the R¹ radicals may be the same or different when they occur more than once;
R¹, R³ are each independently hydrogen;
C₁-C₁₈-alkyl whose carbon chain may be interrupted by one or more -0-, -S-, -CO-, -SO- and/or -SO₂- moieties and which may be mono- or polysubstituted by C₁-C₁₂-alkoxy, C₁-C₆-alkylthio, hydroxyl, mercapto, halogen, cyano, nitro and/or -COOR¹;
aryl or hetaryl, to each of which may be fused further saturated or unsaturated 5- to 7-membered rings whose carbon skeleton may be interrupted by one or more -O-, -S-, -CO-and/or -SO₂- moieties, where the entire ring system may be mono- or polysubstituted by C₁-C₁₂-alkyl and/or the above radicals specified as substituents for alkyl;
n is 1, 2 or 3;
m is 0, 1 or 2;
x, when n = 1:
is from 2 to 4 or else 0 when the B radicals and the B' radicals are each a radical of the formula (b) or one B radical and one B' radical are each a radical of the formula (c) ;
when n = 2:
is from 2 to 6 when the B radicals and the B' radicals are each a radical of the formula (a) ;
is 0 for all further definitions of the B and B' radicals;
when n = 3:
is from 2 to 4 when the B radicals and the B' radicals are each a radical of the formula (a) ;
is 0 for all further definitions of the B and B' radicals;
z is from 0 to 8, where x + z ≤ 8, and, when n = 2 or 3: z ≠ 0 when x = 0;
z1 when m = 0: 0;
when m = 1: from 0 to 2;
when m = 2: from 2 to 4.

2. The polychromophore of the general formula I' according to claim 1, in which the variables are each defined as follows:
Z is phenoxy or thiophenoxy, each of which may be mono- or polysubstituted by identical or different (i), (ii), (iii), (iv) and/or (v) radicals:
(i) C₁-C₃₀-alkyl whose carbon chain may be interrupted by one or more -O-, -S-, -NR¹-, -C≡C-, -CR¹=_{C}R¹- and/or -CO- and/or -SO₂-moieties and which may be mono- or polysubstituted by: C₁-C₁₂-alkoxy, hydroxyl, halogen, cyano, and/or aryl which may be mono- or polysubstituted by C₁-C₁₈-alkyl or C₁-C₆-alkoxy;
(ii) C₃-C₈-cycloalkyl whose carbon skeleton may be interrupted by one or more -O-, -S-, -NR¹-, -CR¹=CR¹- and/or -CO- moieties and which may be mono- or polysubstituted by C₁-C₁₈-alkyl, C₁-C₁₂-alkoxy and/or C₁-C₆-alkylthio;
(iii) aryl or hetaryl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by: C₁-C₁₈-alkyl, C₁-C₁₂-alkoxy, -C=CR¹-, -CR¹=CR¹, hydroxyl, halogen, cyano, -NR²R³, -NR²COR³, -CONR²R³, -SO₂NR²R³, -COOR², -SO₃R², aryl and/or hetaryl, each of which may be mono- or polysubstituted by C₁-C₁₈-alkyl, C₁-C₁₈-alkoxy and/or cyano;
(iv) a -U-aryl radical which may be mono- or polysubstituted by the above radicals specified as substituents for the aryl radicals (iii), where U is an -O-, -S-, -NR¹-, -CO-, -SO- or -SO₂- moiety;
(v) C₁-Cₗ₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR² or -SO₃R²,
where the Z radicals may be the same or different when z > 1 and/or z1 > 1;
A is an arylene or hetarylene radical of the formulae 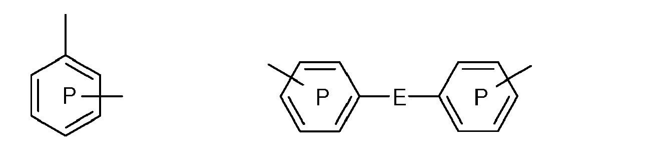 in which the rings P may be the same or different, may comprise heteroatoms as ring atoms and/or may have fused 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by (i), (ii), (iii) and/or (v) radicals specified as substituents for the Z radicals,
where the A radicals may be the same or different when they occur repeatedly in formula I;
E is a chemical bond, an -O-, -S-, -NR¹-, -N=CR¹-, -C≡C-, -CR¹=CR¹-, -CO-, -SO- or -SO₂-moiety or C₁-C₁₂-alkylene or C₄-C₇-cycloalkylene, whose carbon chain may in each case be interrupted once or more than once by these moieties and which may each be mono- or polysubstituted by the (i), (ii), (iii) and/or (v) radicals specified as substituents for the Z radicals;
arylene or hetarylene, each of which may be mono- or polysubstituted by the (i), (ii), (iii) and/or (v) radicals specified as substituents for the Z radicals, where hydroxyl and mercapto are excluded as (v) radicals;
Y is -O-;
Y¹ is -O- or -NR¹-;
R is C₁-C₃₀-alkyl whose carbon chain may be interrupted by one or more -O- and/or -CO-moieties and which may be mono- or polysubstituted by: C₁-C₆-alkoxy, cyano and/or aryl which may be mono- or polysubstituted by C₁-C₁₈-alkyl and/or C₁-C₆-alkoxy;
phenyl, naphthyl, pyridyl or pyrimidyl, each of which may be mono- or polysubstituted by: C₁-C₁₈-alkyl, C₁-C₆-alkoxy, halogen, cyano, nitro, -CONR²R³, -SO₂NR²R³ and/or phenyl- and/or naphthylazo, each of which may be mono- or polysubstituted by C₁-C₁₀-alkyl, C₁-C₆-alkoxy and/or cyano;
C₅-C₈-cycloalkyl which may be mono- or polysubstituted by C₁-C₆-alkyl,
where the R radicals may be the same or different when they occur more than once in formula I;
R¹ is hydrogen or C₁-C₆-alkyl;
R², R³ are each independently hydrogen;
C₁-C₁₈-alkyl which may be mono- or polysubstituted by C₁-C₆-alkoxy, hydroxyl, halogen and/or cyano;
aryl or hetaryl, each of which may be mono- or polysubstituted by C₁-C₆-alkyl and/or the above radicals specified as substituents for alkyl;
n is 1 or 2;
m is 0 or 1 where m = n - 1;
x, when n = 1:
is from 2 to 4 or else 0 when the B radicals and the B' radicals are each a radical of the formula (b), or one B radical and one B' radical are each a radical of the formula (c) ;
when n = 2:
is from 2 to 4 when the B radicals and the B' radicals are each a radical of the formula (a) ;
is 0 for all further definitions of the B and B' radicals;
z is from 0 to 4, where x + z ≤ 4, where, when n = 2: z ≠ 0 when x = 0;
z1 when m = 0: 0;
when m = 1: from 0 to 2.

3. The polychromophore of the general formula I' according to claim 1, in which the variables are each defined as follows:
Z is phenoxy which may be mono- or polysubstituted by identical or different (i), (ii), (iii), (iv) and/or (v) radicals:
(i) C₁-C₁₈-alkyl whose carbon chain may be interrupted by one or more -O-, -S-, -NR¹- and/or -CO- moieties and which may be mono- or polysubstituted by: C₁-C₁₂-alkoxy, hydroxyl and/or halogen;
(ii) C₃-C₈-cycloalkyl which may be mono- or polysubstituted by C₁-C₁₈-alkyl and/or C₁-C₁₂-alkoxy;
(iii) aryl or hetaryl which may be mono- or polysubstituted by C₁-C₁₈-alkyl, C₁-C₁₂-alkoxy, hydroxyl and/or halogen;
(iv) a -U-aryl radical which may be mono- or polysubstituted by the above radicals specified as substituents for the aryl radicals (iii), where U is an -O-, -S- or -NR¹- moiety;
(v) C₁-C₁₂-alkoxy, hydroxyl, halogen or cyano;
B, B' are each independently joined to give a radical of the formula (a) or (b);
A is an arylene radical of the formulae 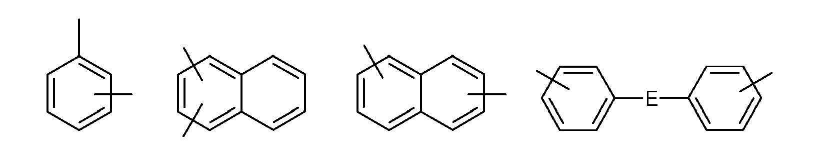 in which the phenylene or naphthylene rings may be mono- or polysubstituted by C₁-C₁₈-alkyl,
where the A radicals may be the same or different when they occur more than once in formula I;
E is a chemical bond, methylene or isopropylene;
Y, Y¹ are each -0-;
R are identical radicals:
C₁-C₃₀-alkyl whose carbon chain may be interrupted by one or more -O- and/or -CO-moieties and which may be mono- or polysubstituted by: C₁-C₆-alkoxy, cyano and/or aryl which may be mono- or polysubstituted by C₁-C₁₈-alkyl or C₁-C₆-alkoxy;
phenyl, naphthyl, pyridyl or pyrimidyl, each of which may be mono- or polysubstituted by: C₁-C₁₈-alkyl, C₁-C₆-alkoxy, halogen, cyano, nitro, -CONR²R³, -SO₂NR²R³, phenyl- and/or naphthylazo, each of which may be mono- or polysubstituted by C₁-C₁₀-alkyl, C₁-C₆-alkoxy and/or cyano;
C₅-C₈-cycloalkyl which may be mono- or polysubstituted by C₁-C₆-alkyl,
R¹ is hydrogen or C₁-C₆-alkyl;
R², R³ are each independently hydrogen;
C₁-C₁₈-alkyl which may be mono- or polysubstituted by C₁-C₆-alkoxy, hydroxyl, halogen and/or cyano;
aryl or hetaryl, each of which may be mono- or polysubstituted by C₁-C₆-alkyl and/or the above radicals specified as substituents for alkyl;
n is 1 or 2;
m is 0 or 1 where m = n - 1;
x, when n = 1:
is from 2 to 4 or else 0 when the B radicals and the B' radicals are each a radical of the formula (b);
when n = 2:
is from 2 to 4 when the B radicals and the B' radicals are each a radical of the formula (a) ;
is 0 when the B and B' radicals are each a radical of the formula b);
z is from 0 to 4, where x + z ≤ 4;
z1 is 0.

4. A process for preparing polychromophores based on rylenetetracarboximides of the general formula Ia 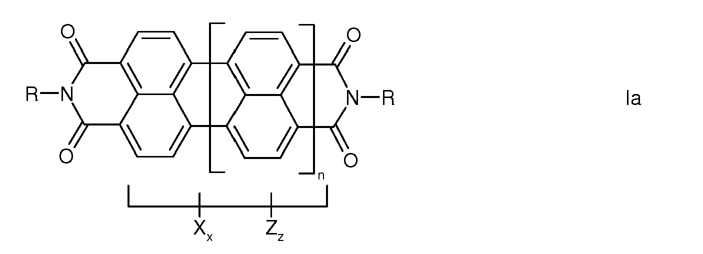 in which the variables are each as defined in claim 1, which comprises reacting a halogenated rylenetetracarboximide of the general formula IIa 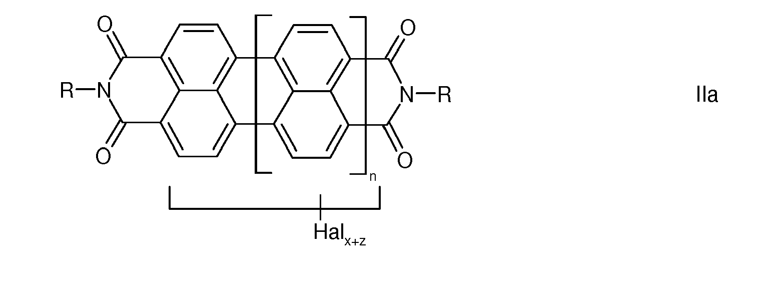 in which Hal is chlorine or bromine, in the presence of a base and of a non-nucleophilic solvent,
a) with from 0.8 to 1.2 mol per mole of halogen atom to be exchanged of a rylenedicarboximide derivative of the general formula IIIa 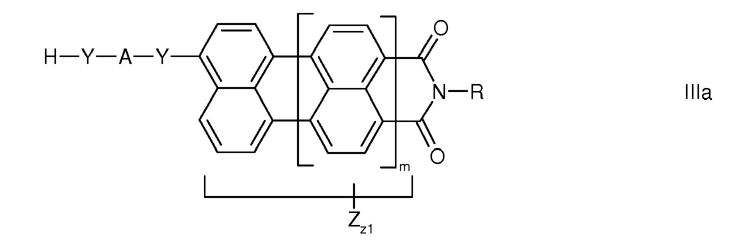 in which the variables are each as defined in claim 2 to give a rylenetetracarboximide of the general formula Ia in which z = 0
or
b) reacting it first with from 0.8 to 1.2 mol per mole of halogen atom to be exchanged of a rylenedicarboximide derivative of the formula IIIa and then with from 0.8 to 3 mol per mole of further halogen atom to be exchanged of a compound of the general formula IV
H-Z IV
or first with from 0.8 to 1.2 mol per mole of halogen atom to be exchanged of a compound of the formula IV and then with from 0.8 to 1.2 mol per mole of further halogen atom to be exchanged of a rylenedicarboximide derivative of the formula IIIa or simultaneously with in each case from 0.8 to 1.2 mol per mole of halogen atom to be exchanged of the rylenedicarboximide derivative of the formula IIIa and of the compound of the formula IV
to give a rylenetetracarboximide of the formula Ia in which z ≠ 0.

5. A process for preparing polychromophores based on rylenedicarboximides of the general formula Ib 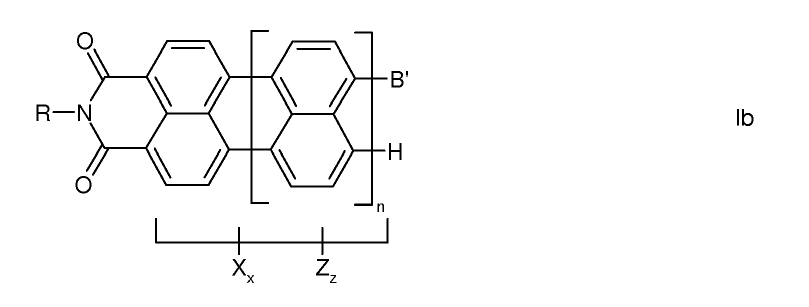 in which n = 1, B' is hydrogen, an X radical or a Z radical and the further variables are each as defined in claim 1, which comprises reacting a halogenated rylenedicarboximide of the general formula IIb 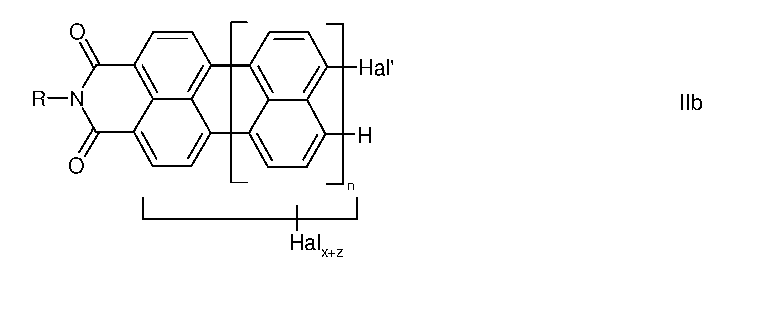 in which n = 1 and Hal' is hydrogen or Hal where Hal is chlorine or bromine, in the presence of a base and of a non-nucleophilic solvent,
a) with from 0.8 to 1.2 mol per mole of halogen atom to be exchanged of a rylenedicarboximide derivative of the general formula IIIa 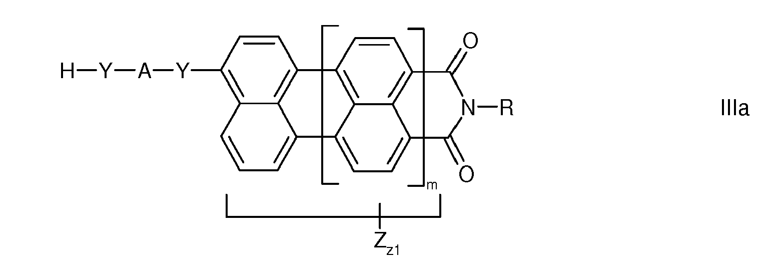 in which the variables are each as defined in claim 2 to give a rylenedicarboximide of the general formula Ib in which z = 0
or
b) reacting it first with from 0.8 to 1.2 mol per mole of halogen atom to be exchanged of a rylenedicarboximide derivative of the formula IIIa and then with from 0.8 to 3 mol per mole of further halogen atom to be exchanged of a compound of the general formula IV
H-Z IV
or first with from 0.8 to 1.2 mol per mole of halogen atom to be exchanged of a compound of the formula IV and then with from 0.8 to 1.2 mol per mole of further halogen atom to be exchanged of a rylenedicarboximide derivative of the formula IIIa or simultaneously with in each case from 0.8 to 1.2 mol per mole of halogen atom to be exchanged of the rylenedicarboximide derivative of the formula IIIa and of the compound of the formula IV
to give a rylenedicarboximide of the formula Ib in which z ≠ 0.

6. A process for preparing polychromophores based on rylenedicarboximides of the general formula Ib' 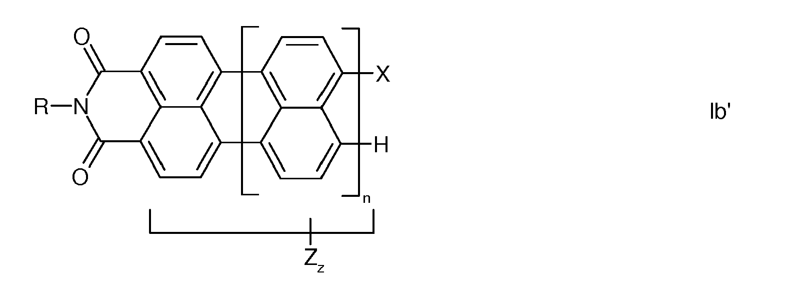 in which n = 2 or 3 and z = from 2 to 8 and the further variables are each as defined in claim 1, which comprises reacting a peri-halorylene-dicarboximide of the general formula IIb' 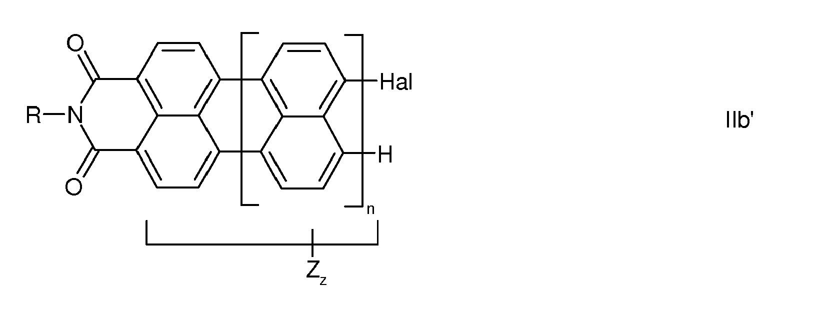 in which n = 2 or 3 and z = from 2 to 8 and Hal is chlorine or bromine, in the presence of a base and of a non-nucleophilic solvent, with from 0.8 to 1.2 mol per mole of a rylenedicarboximide derivative of the general formula IIIa

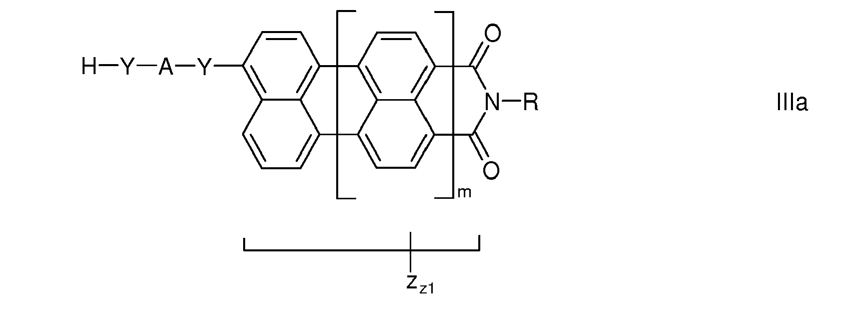

7. A process for preparing polychromophores based on rylenetetracarboximides of the general formula Ic 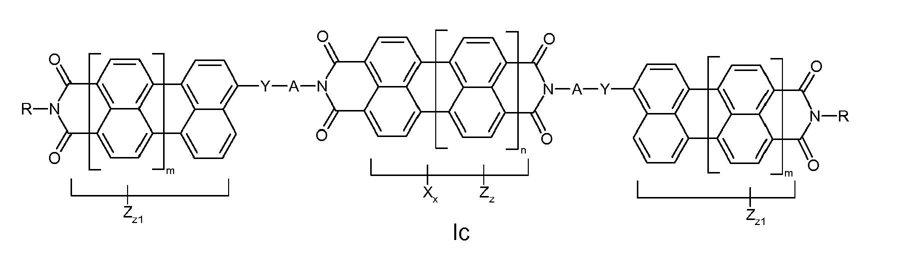 in which n = 1, x is 0 or from 2 to 4 and the further variables are each as defined in claim 1, which comprises reacting an optionally halogenated rylenetetracarboxylic dianhydride of the general formula IIc 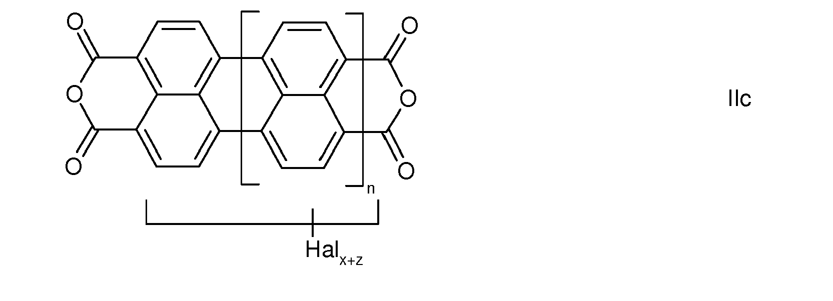 in which n = 1 and Hal is chlorine or bromine, in the presence of a high-boiling solvent and, if desired, of a Lewis acid, with from 1.8 to 3 mol per mole of a rylenedicarboximide derivative of the general formula IIIc 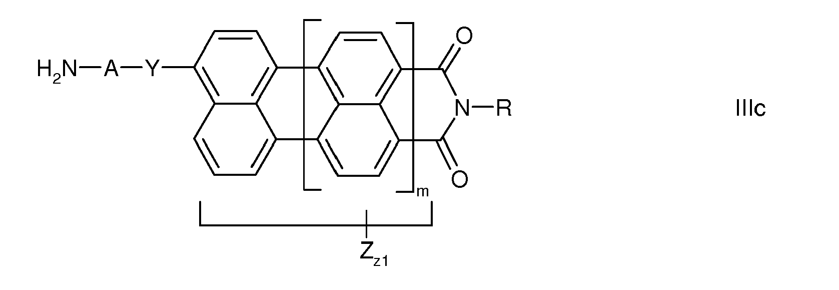 in which the variables are each as defined in claim 2 to give a rylenetetracarboximide of the formula Ic in which x = z = 0,
and optionally reacting the resulting rylenetetracarboximide Ic halogenated in the rylene ring, after intermediate isolation, in the presence of a base and of a non-nucleophilic solvent,
a) with from 0.8 to 1.2 mol per mole of halogen atom to be exchanged of a rylenedicarboximide derivative of the general formula IIIa 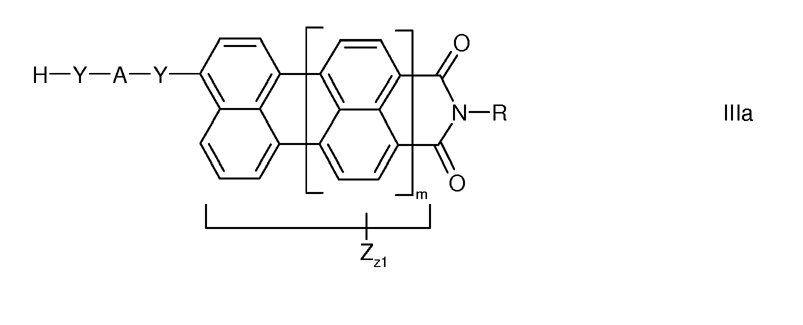 to give a rylenetetracarboximide of the general formula Ic in which x ≠ 0 and z = 0 or
b) reacting it with from 0.8 to 3 mol per mole of halogen atom to be exchanged of a compound of the general formula IV
H-Z IV
to give a rylenetetracarboximide of the formula Ic in which z ≠ 0 and x = 0,
or
c) reacting it first with from 0.8 to 1.2 mol per mole of halogen atom to be exchanged of a rylenedicarboximide derivative of the formula IIIa and then with from 0.8 to 3 mol per mole of further halogen atom to be exchanged of a compound of the general formula IV
or first with from 0.8 to 1.2 mol per mole of halogen atom to be exchanged of a compound of the formula IV and then with from 0.8 to 1.2 mol per mole of further halogen atom to be exchanged of a rylenedicarboximide derivative of the formula IIIa
or simultaneously with in each case from 0.8 to 1.2 mol per mole of halogen atom to be exchanged of the rylenedicarboximide derivative of the formula IIIa and of the compound of the formula IV
to give a rylenetetracarboximide of the formula Ic in which x ≠ 0 and z ≠ 0.

8. A process for preparing polychromophores based on rylenedicarboximides of the general formula Id 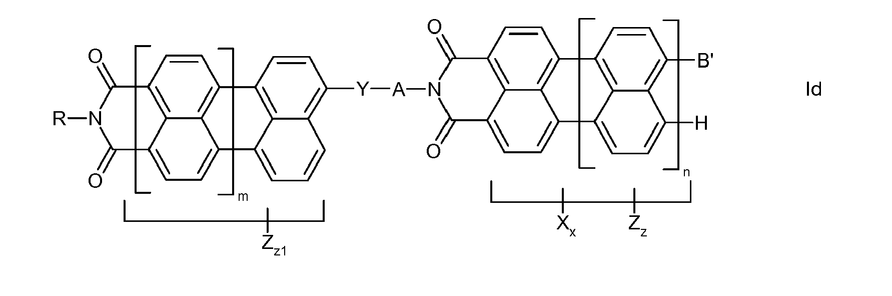 in which n = 1, B' is hydrogen, an X radical or a Z radical, x is 0 or from 2 to 4 and the further variables are each as defined in claim 1, which comprises reacting an optionally halogenated rylenedicarboxylic anhydride of the general formula IId 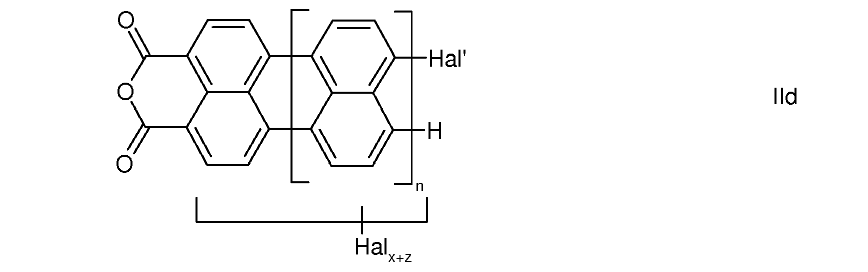 in which n = 1 and Hal' is hydrogen or Hal where Hal is chlorine or bromine, in the presence of a high-boiling solvent and, if desired, of a Lewis acid, with from 0.8 to 1.2 mol per mole of a rylenedicarboximide derivative of the general formula IIIc 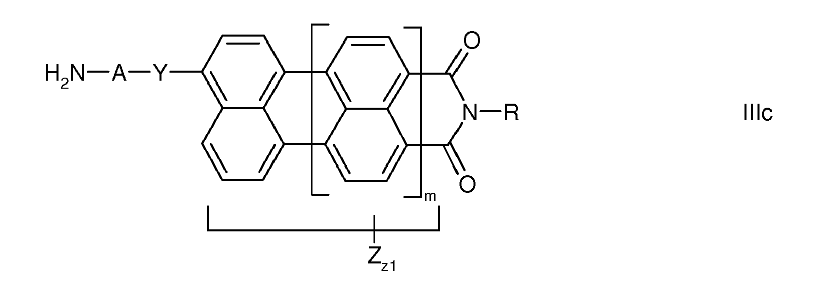 to give a rylenedicarboximide of the formula Id in which x = z = 0,
and optionally reacting the resulting rylenedicarboximide Id halogenated in the rylene ring, after intermediate isolation, in the presence of a base and of a non-nucleophilic solvent,
a) with from 0.8 to 1.2 mol per mole of halogen atom to be exchanged of a rylenedicarboximide derivative of the general formula IIIa 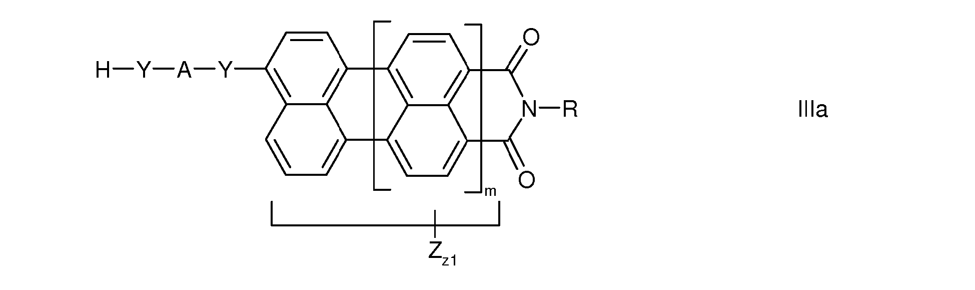 to give a rylenedicarboximide of the general formula Id in which x ≠ 0 and z = 0
or
b) reacting it with from 0.8 to 3 mol per mole of halogen atom to be exchanged of a compound of the general formula IV
H-Z IV
to give a rylened-dicarboximide of the formula Id in which z ≠ 0 and x = 0,
or
c) reacting it first with from 0.8 to 1.2 mol per mole of halogen atom to be exchanged of a rylenedicarboximide derivative of the formula IIIa and then with from 0.8 to 3 mol per mole of further halogen atom to be exchanged of a compound of the general formula IV
or first with from 0.8 to 1.2 mol per mole of halogen atom to be exchanged of a compound of the formula IV and then with from 0.8 to 1.2 mol per mole of further halogen atom to be exchanged of a rylenedicarboximide derivative of the formula IIIa
or simultaneously with in each case from 0.8 to 1.2 mol per mole of halogen atom to be exchanged of the rylenedicarboximide derivative of the formula IIIa and of the compound of the formula IV
to give a rylenedicarboximide of the formula Id in which x ≠ 0 and z ≠ 0.

9. A process for preparing polychromophores based on rylenetetracarboximides of the general formula Ie 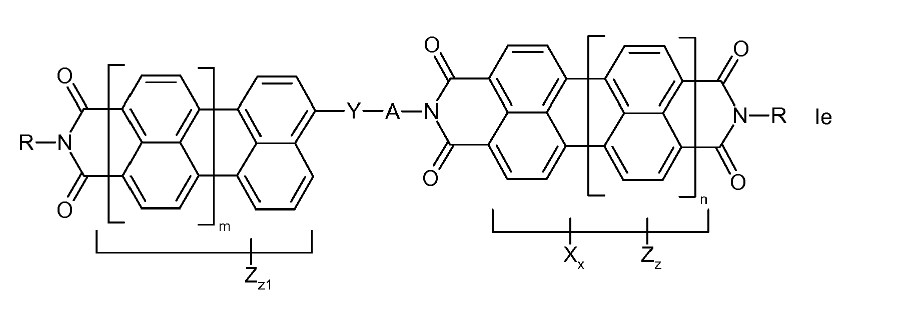 in which n = 1 and the further variables are each as defined in claim 1, which comprises reacting an optionally halogenated rylenetetracarboxylic monoanhydride monoimide of the general formula IIe 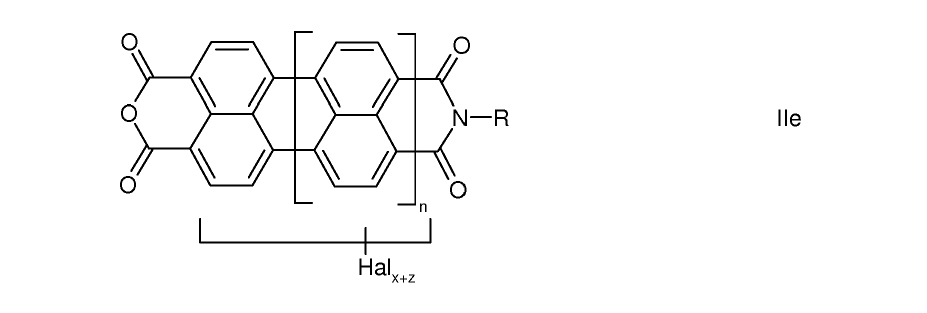 in which n = 1 and Hal is chlorine or bromine, in the presence of a high-boiling solvent and, if desired, of a Lewis acid, with from 0.8 to 1.2 mol per mole of a rylenedicarboximide derivative of the general formula IIIc 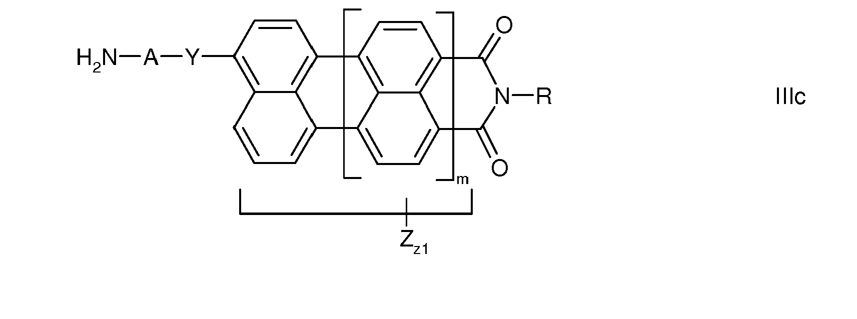 to give a rylenetetracarboximide of the formula Ie in which x = z = 0,
and optionally reacting the resulting rylenedicarboximide Ie halogenated in the rylene ring, after intermediate isolation, in the presence of a base and of a non-nucleophilic solvent,
a) with from 0.8 to 1.2 mol per mole of halogen atom to be exchanged of a rylenedicarboximide derivative of the general formula IIIa 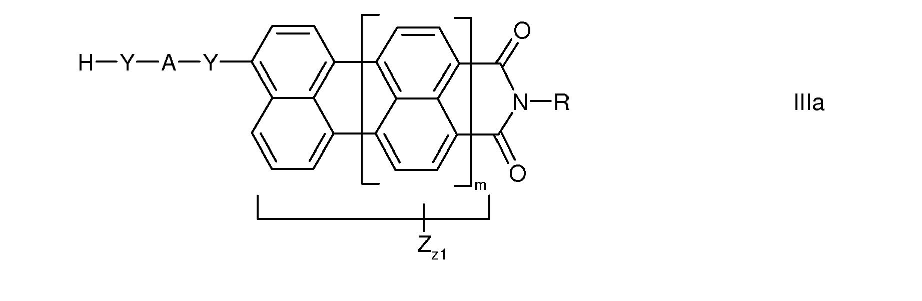 to give a rylenetetracarboximide of the general formula Ie in which x ≠ 0 and z = 0
or
b) reacting it with from 0.8 to 3 mol per mole of halogen atom to be exchanged of a compound of the general formula IV
H-Z IV
to give a rylenetetracarboximide of the formula Ie in which z ≠ 0 and x = 0,
or
c) reacting it first with from 0.8 to 1.2 mol per mole of halogen atom to be exchanged of a rylenedicarboximide derivative of the formula IIIa and then with from 0.8 to 3 mol per mole of further halogen atom to be exchanged of a compound of the general formula IV
or first with from 0.8 to 1.2 mol per mole of halogen atom to be exchanged of a compound of the formula IV and then with from 0.8 to 1.2 mol per mole of further halogen atom to be exchanged of a rylenedicarboximide derivative of the formula IIIa
or simultaneously with in each case from 0.8 to 1.2 mol per mole of halogen atom to be exchanged of the rylenedicarboximide derivative of the formula IIIa and of the compound of the formula IV
to give a rylenetetracarboximide of the formula Ie in which x ≠ 0 and z ≠ 0.

10. A process for preparing polychromophores based on rylenetetracarboximides of the general formula Ic' 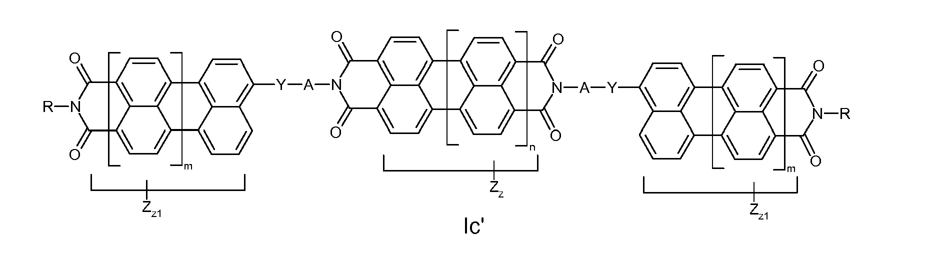 in which n = 2 or 3 and z = from 2 to 8 and the further variables are each as defined in claim 1, which comprises reacting a rylenetetracarboxylic dianhydride of the general formula IIc' 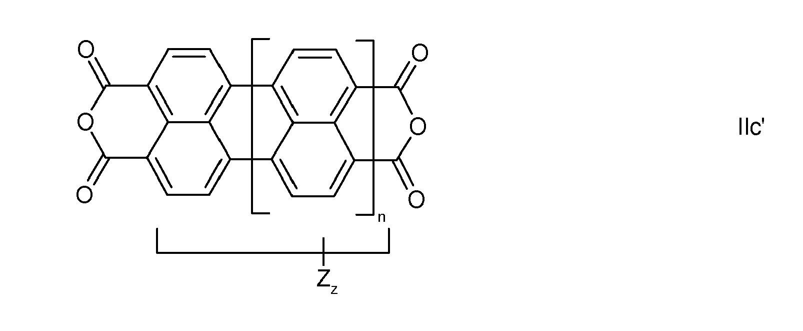 in which n = 2 or 3 and z = from 2 to 8, in the presence of a high-boiling solvent and of a Lewis acid, with from 0.8 to 1.2 mol per mole of a rylenedicarboximide derivative of the general formula IIIc

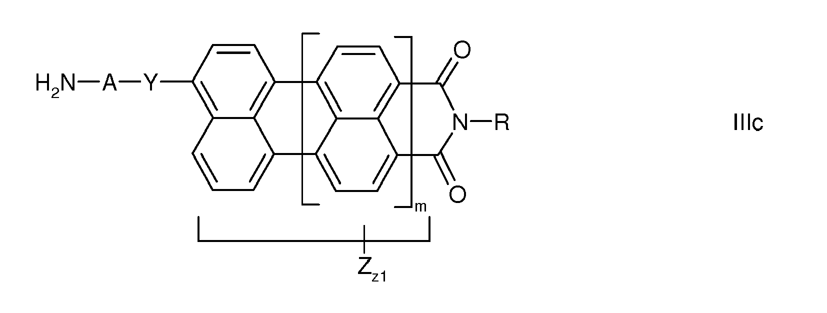

11. A process for preparing polychromophores based on rylenedicarboximides of the general formula Id' 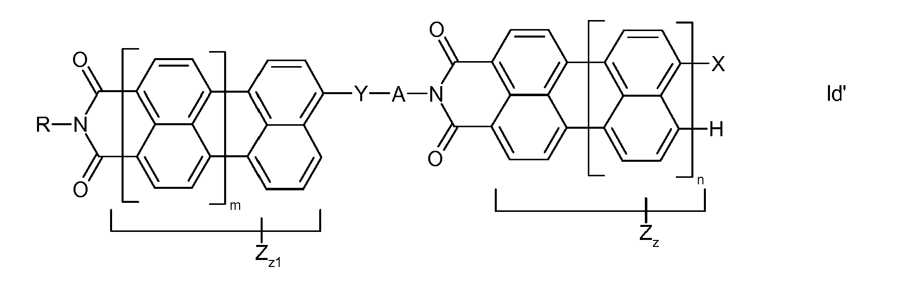 in which n = 2 or 3 and z = from 2 to 8 and the further variables are each as defined in claim 1, which comprises reacting a peri-halorylene-dicarboxylic anhydride of the general formula IId' 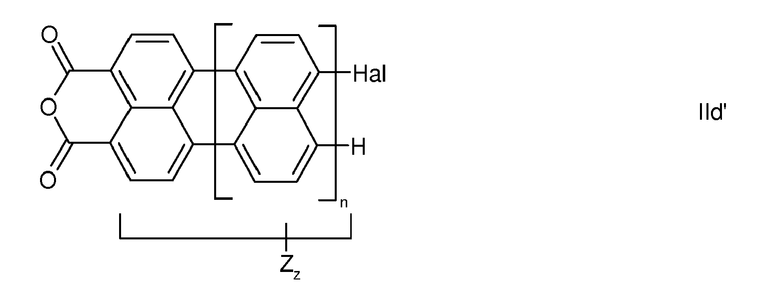 in which n = 2 or 3 and z = from 2 to 8 and Hal is chlorine or bromine, in the presence of a high-boiling solvent and of a Lewis acid, with from 0.8 to 1.2 mol per mole of a rylenedicarboximide derivative of the general formula IIIc

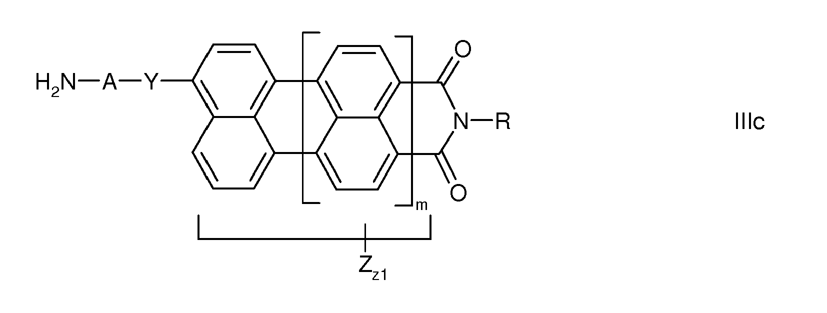

12. A process for preparing polychromophores based on rylenetetracarboximides of the general formula Ie' 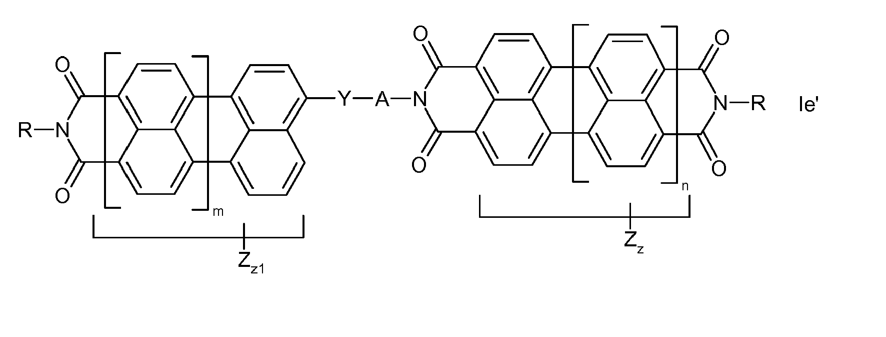 in which n = 2 or 3 and z = from 2 to 8 and the further variables are each as defined in claim 1, which comprises reacting a rylenetetracarboxylic monoanhydride monoimide of the general formula IIe' 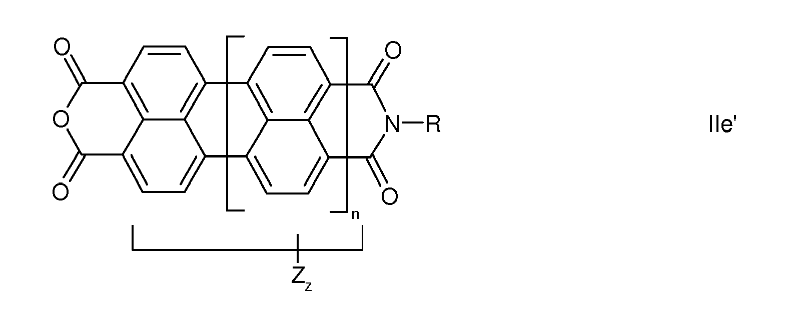 in which n = from 2 to 8, in the presence of a high-boiling solvent and of a Lewis acid, with from 0.8 to 1.2 mol per mole of a rylenedicarboximide derivative of the general formula IIIc 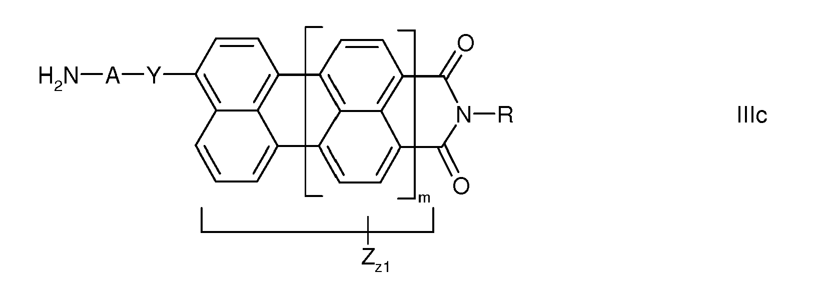 to give a rylenetetracarboximide of the formula Ie'.

13. A process for preparing polychromophores based on rylenedicarboxylic esters of the general formula If 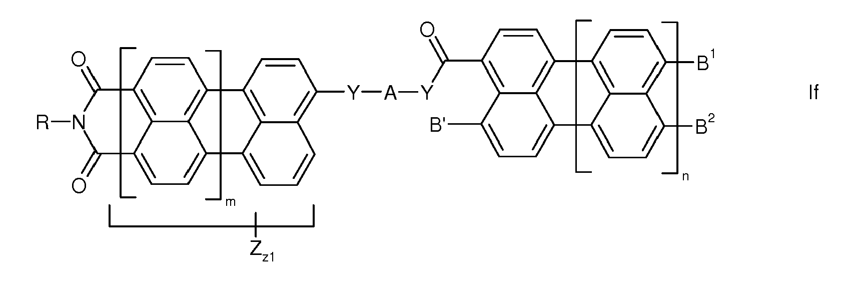 in which n = 1, B' is hydrogen, halogen or cyano, one of the two B¹ and B² radicals is a radical of the formula 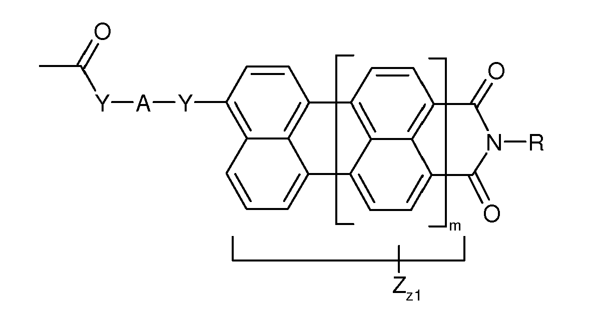 and the other radical is hydrogen, halogen or cyano and the further variables are each as defined in claim 1, which comprises converting a rylenedicarboximide derivative of the general formula IIIa 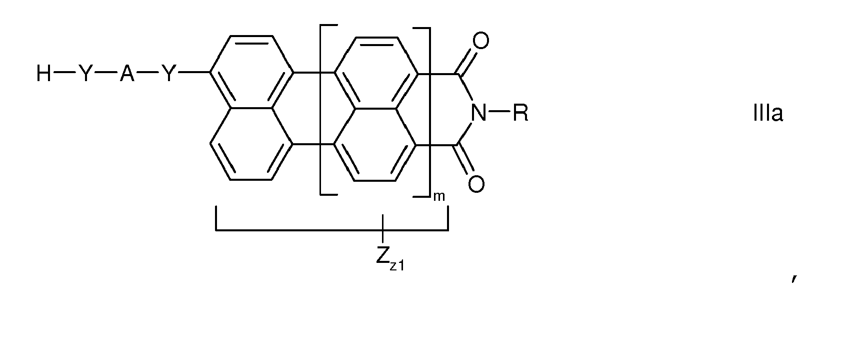 in the presence of an alkali metal base and of an aprotic solvent, to the alkali metal salt of the general formula IIIf 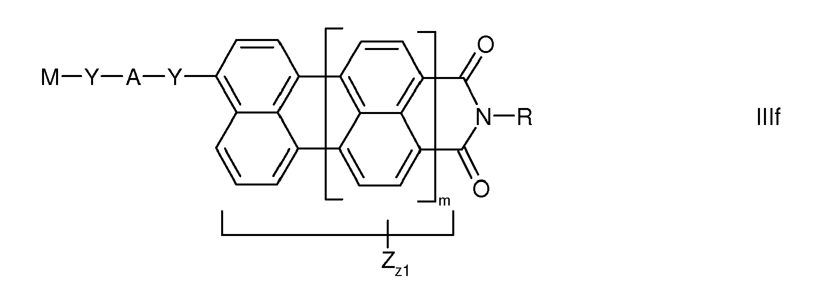 in which M is an alkali metal cation, and then reacting it with a rylenedicarbonyl chloride of the general formula IIf 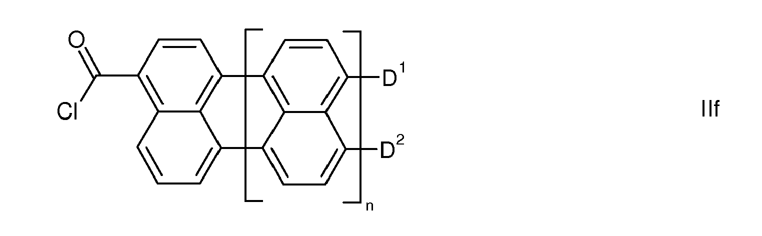 in which n = 1 and one of the two D¹ and D² radicals is -COCl and the other radical is hydrogen, in a molar ratio of from 0.8:1 to 1.2:1, to give a rylenedicarboxylic ester of the formula If in which B' and the remaining B¹ or B² radical are each hydrogen,
and, if desired, halogenating the resulting rylenedicarboxylic ester, after intermediate isolation, in the presence of a polar organic solvent and of a Lewis acid, with N-halosuccinimide to give the rylenedicarboxylic ester of the formula If in which B' and the remaining B¹ or B² radical are each halogen,
and, if desired, converting the resulting halogenated rylenedicarboxylic ester, with a metal cyanide in the presence of an aprotic solvent and of a transition metal catalyst, to the corresponding cyano-substituted rylenedicarboxylic ester of the formula If in which B' and the remaining B¹ or B² radical are each cyano.

14. A process for preparing polychromophores based on rylenedicarboxamides of the general formula Ig 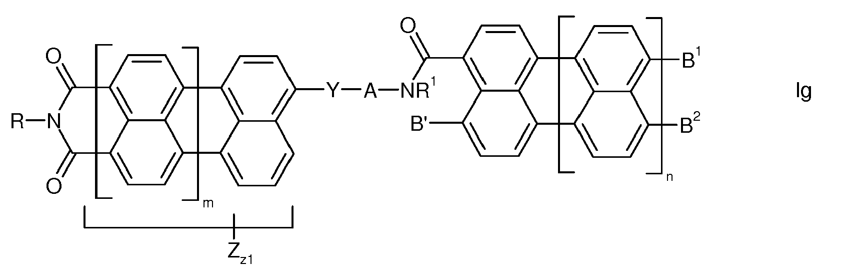 in which n = 1, B' is hydrogen, halogen or cyano, one of the two B¹ and B² radicals is a radical of the formula 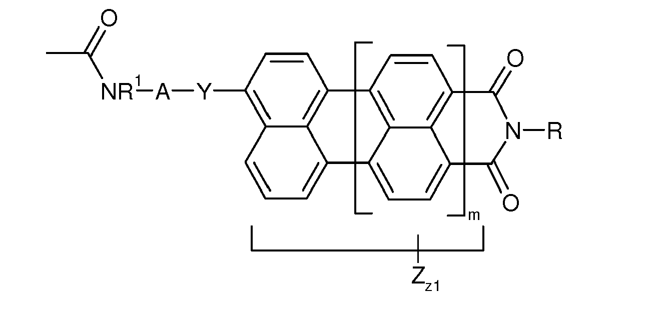 and the other radical is hydrogen, halogen or cyano, and the further variables are each as defined in claim 1, which comprises reacting a rylenedicarbonyl chloride of the general formula IIf 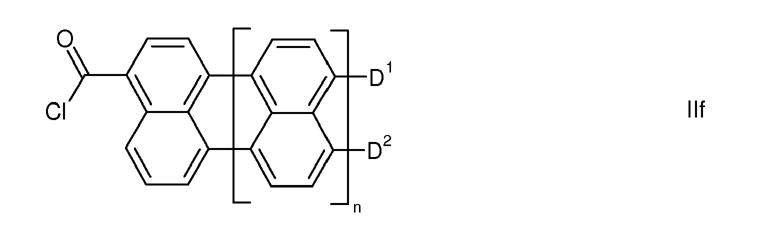 in which n = 1 and one of the two D¹ and D² radicals is -COCl and the other radical is hydrogen, in the presence of a non-nucleophilic base and, if desired, additionally of an aprotic solvent, with from 0.8 to 1.2 mol per mole of a rylenedicarboximide derivative of the general formula IIIg 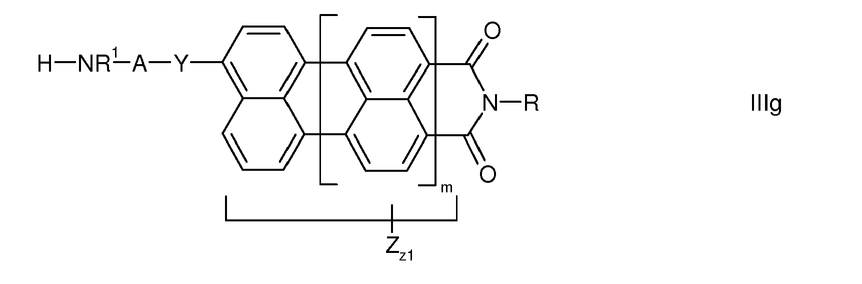 to give a rylenedicarboxamide of the formula Ig in which B' and the remaining B¹ or B² radicals are each hydrogen,
and, if desired, halogenating the resulting rylenedicarboxamide, after intermediate isolation, in the presence of a polar organic solvent and of a Lewis acid, with N-halosuccinimide to give the rylenedicarboxamide of the formula Ig in which B' and the remaining B¹ or B² radical are each halogen,
and, if desired, converting the resulting halogenated rylenedicarboxamide, with a metal cyanide in the presence of an aprotic solvent and of a transition metal catalyst, to the corresponding cyano-substituted rylenedicarboxamide of the formula Ig in which B' and the remaining B¹ or B² radical are each cyano.

15. A rylenedicarboximide derivative of the general formula III 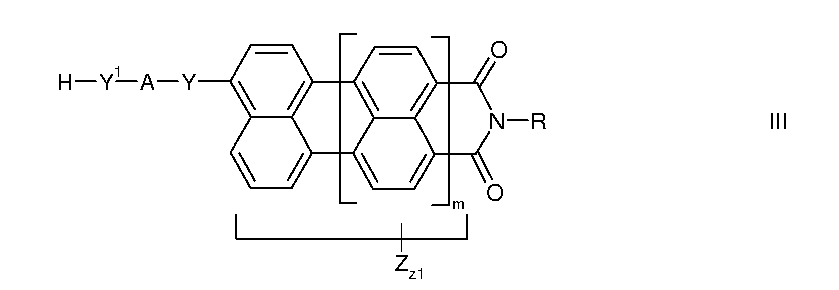 in which the variables are each defined as follows:
Z is aryloxy, arylthio, hetaryloxy or hetarylthio, to each of which may be fused further saturated or unsaturated 5- to 7-membered rings whose carbon skeleton may be interrupted by one or more -O-, -S-, -NR¹-, -N=CR¹-, -CO-, -SO- and/or -SO₂- moieties, where the entire ring system may be mono- or polysubstituted by the (i), (ii), (iii), (iv) and/or (v) radicals:
(i) C₁-C₃₀-alkyl whose carbon chain may be interrupted by one or more -O-, -S-, -NR¹-, -N=CR¹-, -C≡C-, -CR¹=CR¹-, -CO-, -SO- and/or -SO₂- moieties and which may be mono- or polysubstituted by: C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², aryl and/or saturated or unsaturated C₄-C₇-cycloalkyl whose carbon skeleton may be interrupted by one or more -O-, -S-, -NR¹-, -N=CR¹-, -CR¹=CR¹-, -CO-, -SO- and/or -SO₂-moieties, where the aryl and cycloalkyl radicals may each be mono- or polysubstituted by C₁-C₁₈-alkyl and/or the above radicals specified as substituents for alkyl;
(ii) C₃-C₈-cycloalkyl whose carbon skeleton may be interrupted by one or more -O-, -S-, -NR¹-, -N=CR¹-, -CR¹=CR¹-, -CO-, -SO- and/or -SO₂- moieties and to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR² and/or -SO₃R²;
(iii) aryl or hetaryl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², aryl and/or hetaryl, each of which may be mono- or polysubstituted by C₁-C₁₈-alkyl, C₁-C₁₂-alkoxy, hydroxyl, mercapto, halogen, cyano, nitro, -NR²R³, -NR²COR3, -CONR²R³, -SO₂NR²R³, -COOR² and/or -SO₃R²;
(iv) a -U-aryl radical which may be mono- or polysubstituted by the above radicals specified as substituents for the aryl radicals (iii), where U is a -O-, -S-, -NR¹-, -CO-, -SO- or -SO₂- moiety;
(v) C₁-Cₗ₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR² or -SO₃R²,
where the Z radicals may be the same or different when z > 1 and/or z1 > 1;
A is a bridging member having at least one aromatic or heteroaromatic radical, where the Y or Y and Y¹ groups are bonded to the aromatic or heteroaromatic radical;
Y is -O- or -S-;
Y¹ is -O-, -S- or -NR¹-;
R is hydrogen;
C₁-C₃₀-alkyl whose carbon chain may be interrupted by one or more -O-, -S-, -NR¹-, -N=CR¹-, -C≡C-, -CR¹=CR¹-, -CO-, -SO- and/or -SO₂- moieties and which may be mono- or polysubstituted by the (ii), (iii), (iv) and/or (v) radicals specified as substituents for the Z radicals;
C₃-C₈-cycloalkyl to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by the (i), (ii), (iii), (iv) and/or (v) radicals specified as substituents for the Z radicals;
aryl or hetaryl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by the (i), (ii), (iii), (iv), (v) radicals specified as substituents for the Z radicals, and/or aryl- and/or hetarylazo, each of which may be mono- or polysubstituted by C₁-C₁₀-alkyl, C₁-C₆-alkoxy and/or cyano;
where the R radicals may be the same or different when they occur repeatedly in formula I;
R¹ is hydrogen or C₁-C₁₈-alkyl, where the R¹ radicals may be the same or different when they occur more than once;
R², R³ are each independently hydrogen;
C₁-C₁₈-alkyl whose carbon chain may be interrupted by one or more -O-, -S-, -CO-, -SO- and/or -SO₂- moieties and which may be mono- or polysubstituted by C₁-C₁₂-alkoxy, C₁-C₆-alkylthio, hydroxyl, mercapto, halogen, cyano, nitro and/or -COOR¹;
aryl or hetaryl, to each of which may be fused further saturated or unsaturated 5- to 7-membered rings whose carbon skeleton may be interrupted by one or more -O-, -S-, -CO- and/or -SO₂- moieties, where the entire ring system may be mono- or polysubstituted by C₁-C₁₂-alkyl and/or the above radicals specified as substituents for alkyl;
m is 0, 1 or 2;
z1 when m = 0: 0;
when m = 1: from 0 to 2;
when m = 2: from 2 to 4.

16. A process for preparing rylene derivatives of the formula III according to claim 15, which comprises reacting a peri-halogenated rylenedicarboximide of the general formula V 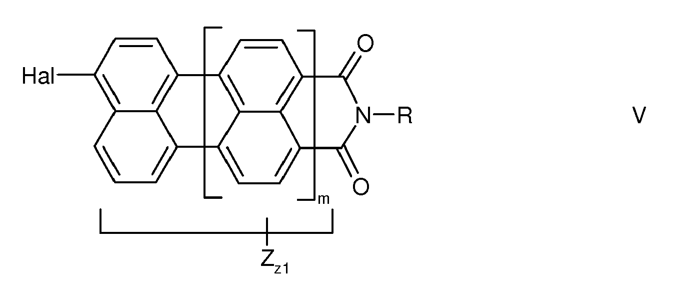 in which Hal is chlorine or bromine, in the presence of a base and of a non-nucleophilic solvent, with from 2 to 5 mol per mole of halogen atom to be exchanged of an at least bifunctional aromatic compound of the general formula VI
H-Y¹-A-Y-H VI.

17. The use of polychromophores according to claims 1 to 3 for coloring organic and inorganic materials.

18. The use according to claim 17, wherein the organic materials are coatings, printing inks or plastics.

19. The use of polychromophores according to claims 1 to 3 for producing aqueous polymer dispersions which absorb and/or emit electromagnetic radiation.

20. The use of polychromophores according to claims 1 to 3 for obtaining markings and inscriptions invisible to the human eye.

21. The use of polychromophores according to claims 1 to 3 as filters or emitters in display applications.

22. The use of polychromophores according to claims 1 to 3 as emitters in chemiluminescence applications.

23. The use of polychromophores according to claims 1 to 3 as active components in photovoltaics.

## Revendications

1. Multichromophores à base de Rylène de formule générale I' 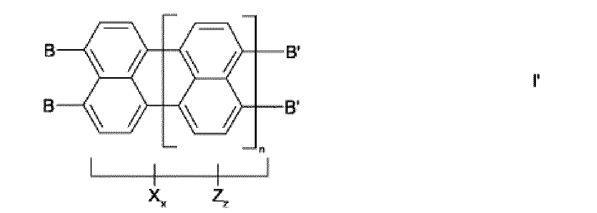 dans laquelle les variables ont la signification suivante :
X représente un radical Rylènedicarboximido de formule 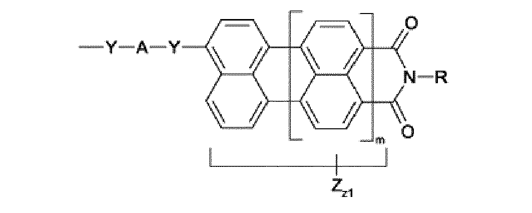 où les radicaux X peuvent être identiques ou différents ;
Z représente un groupe aryloxy, arylthio, hétéroaryloxy ou hétéroarylthio, à chacun desquels peuvent être soudés d'autres cycles saturés ou insaturés à 5 à 7 chaînons, dont le squelette carboné peut être interrompu par un ou plusieurs groupements -O-, -S-, -NR¹-, -N=CR¹-, -CO-, -SO- et/ou -SO₂-, le système cyclique dans son ensemble pouvant être une ou plusieurs fois substitué par les radicaux (i), (ii), (iii), (iv) et/ou (v) :
(i) alkyle en C₁-C₃₀, dont la chaîne carbonée peut être interrompue par un ou plusieurs groupements -O-, -S-, -NR¹-, -N=CR¹-, -C≡C-, -CR¹=CR¹-, -CO-, -SO- et/ou -SO₂- et qui peut être une ou plusieurs fois substitué par : alcoxy en C₁-C₁₂, alkyl (C₁-C₆) thio, -C≡CR¹, -CR¹=CR¹₂, hydroxy, mercapto, halogéno, cyano, nitro, -NR²R³, -NR²COR³, -CONR²R³, -SO₂NR²R³, -COOR², -SO₃R², aryle et/ou cycloalkyle en C₄-C₇ saturé ou insaturé, dont le squelette carboné peut être interrompu par un ou plusieurs groupements -O-, -S-, -NR¹-, -N=CR¹-, -CR¹=CR¹-, -CO-, -SO- et/ou -SO₂-, les radicaux aryle et cycloalkyle pouvant être une ou plusieurs fois substitués chacun par alkyle en C₁-C₁₈ et/ou par les radicaux précédents, nommés en tant que substituants pour alkyle ;
(ii) cycloalkyle en C₃-C₈, dont le squelette carboné peut être interrompu par un ou plusieurs groupements -O-, -S-, -NR¹-, -N=CR¹-, -CR¹=CR¹-, -CO-, -SO- et/ou -SO₂- et auquel peuvent être soudés d'autres cycles saturés ou insaturés à 5 à 7 chaînons, dont le squelette carboné peut être interrompu par un ou plusieurs groupements -O-, -S-, -NR¹-, -N=CR¹-, -CR¹=CR¹-, -CO-, -SO-et/ou -SO₂-, le système cyclique dans son ensemble pouvant être une ou plusieurs fois substitué par : alkyle en C₁-C₁₈, alcoxy en C₁-C₁₂, alkyl (C₁-C₆) thio, -C≡CR¹, -CR¹=CR¹₂, hydroxy, mercapto, halogéno, cyano, nitro, -NR²R³, -NR²COR³, -CONR²R³, SO₂NR²R³, -COOR² et/ou -SO₃R²;
(iii) aryle ou hétéroaryle, auquel peuvent être soudés d'autres cycles saturés ou insaturés à 5 à 7 chaînons, dont le squelette carboné peut être interrompu par un ou plusieurs groupements -O-, -S-, -NR¹-, -N=CR¹-, -CR¹=CR¹-, -CO-, -SO-et/ou -SO₂-, le système cyclique dans son ensemble pouvant être une ou plusieurs fois substitué par : alkyle en C₁-C₁₈, alcoxy en C₁-C₁₂, alkyl (C₁-C₆) thio, -CºCR¹, -CR¹=CR¹₂, hydroxy, mercapto, halogéno, cyano, nitro, -NR²R³, -NR²COR³, -CONR²R³, -SO₂NR²R³, -COOR², -SO₃R², aryle et/ou hétéroaryle, qui peut dans chaque cas être une ou plusieurs fois substitué par alkyle en C₁-C₁₈, alcoxy en C₁-C₁₂, hydroxy, mercapto, halogéno, cyano, nitro, -NR²R³, -NR²COR³, -CONR²R³, -SO₂NR²R³, -COOR² et/ou -SO₃R²;
(iv) un radical -U-aryle qui peut être une ou plusieurs fois substitué par les radicaux précédents, nommés en tant que substituants pour les radicaux aryle (iii), U représentant un groupement -O-, -S-, -NR¹-, -CO-, -SO- ou -SO₂- ;
(v) alcoxy en C₁-C₁₂, alkyl (C₁-C₆) thio, -CºCR¹, -CR¹=CR¹₂, hydroxy, mercapto, halogéno, cyano, nitro, -NR²R³, -NR²COR³, -CONR²R³, -SO₂NR²R³, -COOR² et/ou -SO₃R²,
les radicaux Z pour z > 1 et/ou z1 > 1 pouvant être identiques ou différents ;
B pour n = 1, 2 ou 3 :
sont liés l'un à l'autre avec formation d'un cycle hexagonal en un radical de formule (a) ou (b) 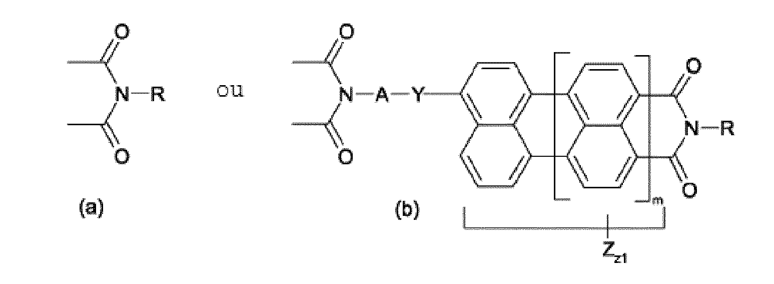 ou
un radical B représente un atome d'hydrogène et l'autre radical un radical X ;
pour n = 1 en outre :
les deux représentent un atome d'hydrogène, un radical B est un atome d'hydrogène et l'autre radical est Z ou un radical B est un atome d'hydrogène ou d'halogène ou le groupe cyano et l'autre radical est un radical de formule (c) 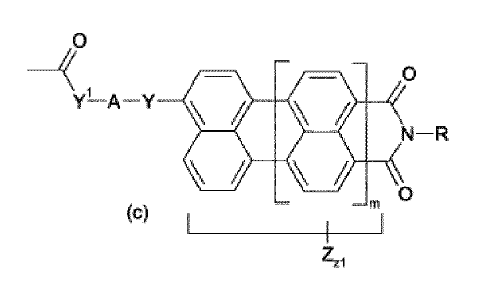
B' pour n = 1, 2 ou 3 :
sont liés l'un à l'autre avec formation d'un cycle hexagonal en un radical de formule (a), lorsque les radicaux B représentent ensemble un radical de formule (a) ;
sont liés l'un à l'autre avec formation d'un cycle hexagonal en un radical de formule (a), lorsqu'un radical B représente un atome d'hydrogène et l'autre radical représente un radical X, en ayant pour n = 2 ou 3 : x = 0 et z ≠ 0 ;
sont liés l'un à l'autre avec formation d'un cycle hexagonal en un radical de formule (b), lorsque les radicaux B représentent ensemble un radical de formule (a) ou (b) ou un radical B représente un atome d'hydrogène et l'autre radical représente un radical X ou Z, en ayant pour n = 2 ou 3 : x = 0 et z ≠ 0 ;
pour n = 1 en outre :
sont liés l'un à l'autre avec formation d'un cycle hexagonal en un radical de formule (a) ou (b), lorsqu'un radical B représente un atome d'hydrogène et l'autre radical représente un radical Z :
un radical B' représente un atome d'hydrogène ou d'halogène ou le groupe cyano et l'autre radical représente un radical de formule (c), lorsqu'un radical B représente un atome d'hydrogène ou d'halogène ou le groupe cyano et l'autre radical représente un radical de formule (c) ;
A représente un chaînon pontant comportant au moins un radical aromatique ou hétéroaromatique, les groupes Y ou Y et Y¹ étant liés au radical aromatique ou hétéroaromatique ;
Y est -O- ou -S- ;
Y¹ est -O- ; -S- ou -NR¹- ;
R représente un atome d'hydrogène ;
un groupe alkyle en C₁-C₃₀, dont la chaîne carbonée peut être interrompue par un ou plusieurs groupements -O-, -S-, -NR¹-, -N=CR¹-, -CºC-, -CR¹=CR¹-, -CO-, -SO- et/ou -SO₂- et qui peut être une ou plusieurs fois substitué par les radicaux (ii), (iii), (iv) et/ou (v) nommés en tant que substituants pour les radicaux Z ;
cycloalkyle en C₃-C₈, auquel peuvent être soudés d'autres cycles saturés ou insaturés à 5 à 7 chaînons, dont le squelette carboné peut être interrompu par un ou plusieurs groupements -O-, -S-, -NR¹-, -N=CR¹-, -CR¹=CR¹-, -CO-, -SO- et/ou -SO₂-, le système cyclique dans son ensemble pouvant être une ou plusieurs fois substitué par les radicaux (i), (ii), (iii), (iv) et/ou (v) nommés en tant que substituants pour les radicaux Z ;
aryle ou hétéroaryle, auquel peuvent être soudés d'autres cycles saturés ou insaturés à 5 à 7 chaînons, dont le squelette carboné peut être interrompu par un ou plusieurs groupements -O-, -S-, -NR¹-, -N=CR¹-, -CR¹=CR¹-, -CO-, -SO- et/ou -SO₂-, le système cyclique dans son ensemble pouvant être une ou plusieurs fois substitué par les radicaux (i), (ii), (iii), (iv), (v) nommés en tant que substituants pour les radicaux Z ;
et/ou aryl- et/ou hétéroarylazo, qui peut dans chaque cas être une ou plusieurs fois substitué par alkyle en C₁-C₁₀, alcoxy en C₁-C₆ et/ou cyano, les radicaux R pouvant être identiques ou différents lorsqu'ils apparaissent plusieurs fois dans la formule I ;
R¹ représente un atome d'hydrogène ou un groupe alkyle en C₁-C₁₈, les radicaux R¹ pouvant être identiques ou différents lorsqu'ils apparaissent plusieurs fois ;
R², R³ représentent indépendamment l'un de l'autre un atome d'hydrogène ;
un groupe alkyle en C₁-C₁₈, dont la chaîne carbonée peut être interrompue par un ou plusieurs groupements -O-, -S-, -CO-, -SO- et/ou -SO₂- et qui peut être une ou plusieurs fois substitué par alcoxy en C₁-C₁₂, alkyl(C₁-C₆)thio, hydroxy, mercapto, halogéno, cyano, nitro et/ou -COOR¹;
un groupe aryle ou hétéroaryle, à chacun desquels peuvent être soudés d'autres cycles saturés ou insaturés à 5 à 7 chaînons, dont le squelette carboné peut être interrompu par un ou plusieurs groupements -O-, -S-, -CO- et/ou -SO₂-, le système cyclique dans son ensemble pouvant être une ou plusieurs fois substitué par alkyle en C₁-C₁₂ et/ou par les radicaux précédents, nommés en tant que substituants pour alkyle ;
n vaut 1, 2 ou 3 ;
m vaut 0, 1 ou 2 ;
x pour n = 1 :
vaut de 2 à 4 ou bien 0, lorsque les radicaux B et les radicaux B' représentent chacun un radical de formule (b) ou un radical B et un radical B' représentent chacun un radical de formule (c) ;
pour n = 2 :
vaut de 2 à 6, lorsque les radicaux B et les radicaux B' représentent chacun un radical de formule (a) ;
vaut 0 pour toutes les autres significations des radicaux B et B' ;
pour n = 3 :
vaut de 2 à 4, lorsque les radicaux B et les radicaux B' représentent chacun un radical de formule (a) ;
vaut 0 pour toutes les autres significations des radicaux B et B' ;
z vaut de 0 à 8, x + z étant ≤ 8 et pour n = 2 ou 3 on a : z ≠ 0, lorsque x est égal à 0 ;
z1 pour m = 0 : 0 ;
pour m = 1 : 0 à 2 ;
pour m = 2 : 2 à 4.

2. Multichromophores de formule générale I' selon la revendication 1, dans laquelle les variables ont la signification suivante :
Z représente un groupe phénoxy ou thiophénoxy, qui peut dans chaque cas être une ou plusieurs fois substitué par des radicaux (i), (ii), (iii), (iv) et/ou (v) identiques ou différents :
(i) alkyle en C₁-C₃₀, dont la chaîne carbonée peut être interrompue par un ou plusieurs groupements -O-, -S-, -NR¹-, -CºC-, -CR¹=CR¹-et/ou -CO- et/ou -SO₂- et qui peut être une ou plusieurs fois substitué par : alcoxy en C₁-C₁₂, hydroxy, halogéno, cyano et/ou aryle qui peut être une ou plusieurs fois substitué par alkyle en C₁-C₁₈ et/ou alcoxy en C₁-C₆ ;
(ii) cycloalkyle en C₃-C₈, dont le squelette carboné peut être interrompu par un ou plusieurs groupements -O-, -S-, -NR¹-, -CR¹=CR¹- et/ou -CO-et qui peut être une ou plusieurs fois substitué par alkyle en C₁-C₁₈, alcoxy en C₁-C₁₂ et/ou alkyl (C₁-C₆) thio ;
(iii) aryle ou hétéroaryle, à chacun desquels peuvent être soudés d'autres cycles saturés ou insaturés à 5 à 7 chaînons, dont le squelette carboné peut être interrompu par un ou plusieurs groupements -O-, -S-, -NR¹-, -N=CR¹-, -CR¹=CR¹-, -CO-, -SO- et/ou -SO₂-, le système cyclique dans son ensemble pouvant être une ou plusieurs fois substitué par : alkyle en C₁-C₁₈, alcoxy en C₁-C₁₂, -C=CR¹-, -CR¹=CR¹-, hydroxy, halogéno, cyano, -NR²R³, -NR²COR³, -CONR²R³, -SO₂NR²R³, -COOR², -SO₃R², aryle et/ou hétéroaryle, qui peut dans chaque cas être une ou plusieurs fois substitué par alkyle en C₁-C₁₈, alcoxy en C₁-C₁₈ et/ou cyano ;
(iv) un radical -U-aryle qui peut être une ou plusieurs fois substitué par les radicaux précédents, nommés en tant que substituants pour les radicaux aryle (iii), U représentant un groupement -O-, -S-, -NR¹-, -CO-, -SO- ou -SO₂- ;
(v) alcoxy en C₁-C₁₂, alkyl (C₁-C₆) thio, -CºCR¹, -CR¹=CR¹₂, hydroxy, mercapto, halogéno, cyano, nitro, -NR²R³, -NR²COR³, -CONR²R³, -SO₂NR²R³, -COOR² ou -SO₃R²,
les radicaux Z pour z > 1 et/ou z1 > 1 pouvant être identiques ou différents ;
A représente un radical arylène ou hétéroarylène de formules 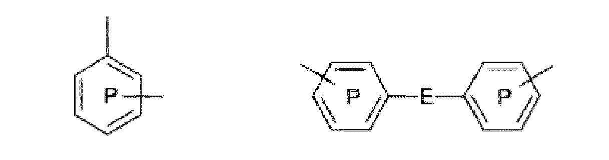 dans lesquelles les cycles P peuvent être identiques ou différents, peuvent contenir des hétéroatomes en tant qu'atomes formant le cycle et/ou peuvent comporter des cycles soudés à 5 à 7 chaînons, dont le squelette carboné peut être interrompu par un ou plusieurs groupements -O-, -S-, -NR¹-, -N=CR¹-, -CR¹=CR¹-, -CO-, -SO- et/ou -SO₂-, le système cyclique dans son ensemble pouvant être une ou plusieurs fois substitué par les radicaux (i), (ii), (iii) et/ou (v) nommés en tant que substituants pour les radicaux Z, les radicaux A pouvant être identiques ou différents, lorsqu'ils apparaissent plusieurs fois dans la formule I ;
E représente une liaison chimique, un groupement -O-, -S-, -NR¹-, -N=CR¹-, -CºC-, -CR¹=CR¹-, -CO-, -SO- ou -SO₂- ou alkylène en C₁-C₁₂ ou cycloalkylène en C₄-C₇, dont la chaîne carbonée peut dans chaque cas être une ou plusieurs fois interrompue par ces groupements et qui peut dans chaque cas être une ou plusieurs fois substitué par les radicaux (i), (ii), (iii) et/ou (v) nommés en tant que substituants pour les radicaux Z ;
un groupe arylène ou hétéroarylène, qui peut dans chaque cas être une ou plusieurs fois substitué par les radicaux (i), (ii), (iii) et/ou (v) nommés en tant que substituants pour les radicaux Z, les groupes hydroxy et mercapto étant exclus en tant que radicaux (v) ;
Y est -O- ;
Y¹ est -O- ou -NR¹- ;
R représente un groupe alkyle en C₁-C₃₀, dont la chaîne carbonée peut être interrompue par un ou plusieurs groupements -O- et/ou -CO- et qui peut être une ou plusieurs fois substitué par : alcoxy en C₁-C₆, cyano et/ou aryle qui peut être une ou plusieurs fois substitué par alkyle en C₁-C₁₈ et/ou alcoxy en C₁-C₆ ;
phényle, naphtyle, pyridyle ou pyrimidyle, qui peut dans chaque cas être une ou plusieurs fois substitué par : alkyle en C₁-C₁₈, alcoxy en C₁-C₆, halogéno, cyano, nitro, -CONR²R³, -SO₂NR²R³ et/ou phénylazo et/ou naphtylazo, qui peut dans chaque cas être une ou plusieurs fois substitué par alkyle en C₁-C₁₀, alcoxy en C₁-C₆ et/ou cyano ;
cycloalkyle en C₅-C₈ qui peut être une ou plusieurs fois substitué par alkyle en C₁-C₆, les radicaux R pouvant être identiques ou différents lorsqu'ils apparaissent plusieurs fois dans la formule I ;
R¹ représente un atome d'hydrogène ou un groupe alkyle en C₁-C₆ ;
R², R³ représentent indépendamment l'un de l'autre un atome d'hydrogène ;
un groupe alkyle en C₁-C₁₈ qui peut être une ou plusieurs fois substitué par alcoxy en C₁-C₆, hydroxy, halogéno et/ou cyano ;
un groupe aryle ou hétéroaryle, qui peut dans chaque cas être une ou plusieurs fois substitué par alkyle en C₁-C₆ et/ou par les radicaux précédents, nommés en tant que substituants pour alkyle ;
n vaut 1 ou 2 ;
m vaut 0 ou 1, m étant égal à n - 1 ;
x pour n = 1 :
vaut de 2 à 4 ou bien 0, lorsque les radicaux B et les radicaux B' représentent chacun un radical de formule (b) ou un radical B et un radical B' représentent chacun un radical de formule (c) ;
pour n = 2 :
vaut de 2 à 4, lorsque les radicaux B et les radicaux B' représentent chacun un radical de formule (a) ;
vaut 0 pour toutes les autres significations des radicaux B et B' ;
z vaut de 0 à 4, x + z étant ≤ 4 et pour n = 2 on a : z ≠ 0, lorsque x est égal à 0 ;
z1 pour m = 0 : 0 ;
pour m = 1 : 0 à 2.

3. Multichromophores de formule générale I' selon la revendication 1, dans laquelle les variables ont la signification suivante :
Z représente un groupe phénoxy qui peut être une ou plusieurs fois substitué par des radicaux (i), (ii), (iii), (iv) et/ou (v) identiques ou différents :
(i) alkyle en C₁-C₁₈, dont la chaîne carbonée peut être interrompue par un ou plusieurs groupements -O-, -S-, -NR¹- et/ou -CO- et qui peut être une ou plusieurs fois substitué par alcoxy en C₁-C₁₂, hydroxy et/ou halogéno ;
(ii) cycloalkyle en C₃-C₈ qui peut être une ou plusieurs fois substitué par alkyle en C₁-C₁₈ et/ou alcoxy en C₁-C₁₂ ;
(iii) aryle ou hétéroaryle, qui peut être une ou plusieurs fois substitué par alkyle en C₁-C₁₈, alcoxy en C₁-C₁₂, hydroxy et/ou halogéno ;
(iv) un radical -U-aryle qui peut être une ou plusieurs fois substitué par les radicaux précédents, nommés en tant que substituants pour les radicaux aryle (iii), U représentant un groupement -O-, -S- ou -NR¹- ;
(v) alcoxy en C₁-C₁₂, hydroxy, halogéno ou cyano ;
B, B' sont chaque fois liés l'un à l'autre en un radical de formule (a) ou (b) ;
A représente un radical arylène de formules 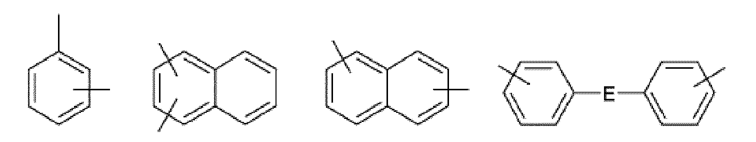 dans lesquelles les cycles phénylène ou naphtylène peuvent être une ou plusieurs fois substitués par alkyle en C₁-C₁₈, les radicaux A pouvant être identiques ou différents, lorsqu'ils apparaissent plusieurs fois dans la formule I ;
E représente une liaison chimique, un groupe méthylène ou isopropylène ;
Y, Y¹ représentent -O- ;
R représente des radicaux identiques :
alkyle en C₁-C₃₀, dont la chaîne carbonée peut être interrompue par un ou plusieurs groupements -O- et/ou -CO- et qui peut être une ou plusieurs fois substitué par : alcoxy en C₁-C₆, cyano et/ou aryle qui peut être une ou plusieurs fois substitué par alkyle en C₁-C₁₈ et/ou alcoxy en C₁-C₆ ;
phényle, naphtyle, pyridyle ou pyrimidyle, qui peut dans chaque cas être une ou plusieurs fois substitué par : alkyle en C₁-C₁₈, alcoxy en C₁-C₆, halogéno, cyano, nitro, -CONR²R³, -SO₂NR²R³ et/ou phénylazo et/ou naphtylazo, qui peut dans chaque cas être une ou plusieurs fois substitué par alkyle en C₁-C₁₀, alcoxy en C₁-C₆ et/ou cyano ;
cycloalkyle en C₅-C₈ qui peut être une ou plusieurs fois substitué par alkyle en C₁-C₆ ;
R¹ représente un atome d'hydrogène ou un groupe alkyle en C₁-C₆ ;
R², R³ représentent indépendamment l'un de l'autre un atome d'hydrogène ;
un groupe alkyle en C₁-C₁₈ qui peut être une ou plusieurs fois substitué par alcoxy en C₁-C₆, hydroxy, halogéno et/ou cyano ;
un groupe aryle ou hétéroaryle, qui peut dans chaque cas être une ou plusieurs fois substitué par alkyle en C₁-C₆ et/ou par les radicaux précédents, nommés en tant que substituants pour alkyle ;
n vaut 1 ou 2 ;
m vaut 0 ou 1, m étant égal à n - 1 ;
x pour n = 1 :
vaut de 2 à 4 ou bien 0, lorsque les radicaux B et les radicaux B' représentent chacun un radical de formule (b) ;
pour n = 2 :
vaut de 2 à 4, lorsque les radicaux B et les radicaux B' représentent chacun un radical de formule (a) ;
vaut 0 lorsque les radicaux B et les radicaux B' représentent chacun un radical de formule (b) ;
z vaut de 0 à 4, x + z étant ≤ 4 ;
z1 est 0.

4. Procédé pour la préparation de multichromophores à base de Rylènetétracarbodiimides de formule générale Ia 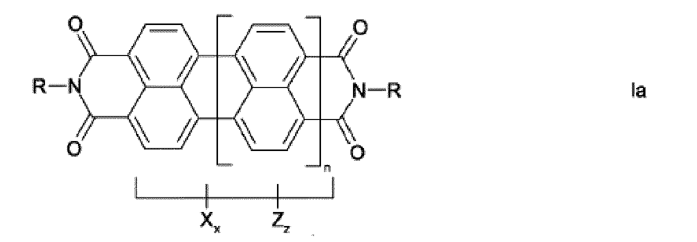 dans laquelle les variables ont la signification indiquée dans la revendication 1, **caractérisé en ce qu'**on fait réagir un Rylènetétracarbodiimide halogéné de formule générale IIa 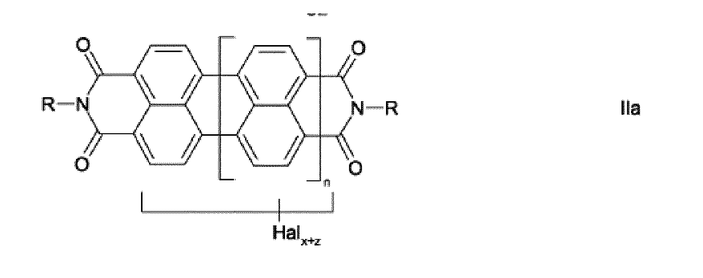 dans laquelle Hal représente le chlore ou le brome, en présence d'une base et d'un solvant non nucléophile
a) avec 0,8 à 1,2 mole, par mole d'atome d'halogène à remplacer, d'un dérivé de Rylènedicarboximide de
formule générale IIIa 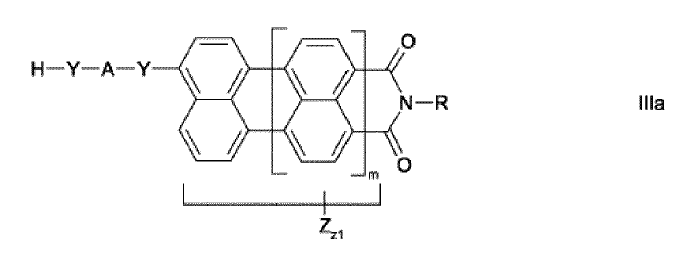 dans laquelle les variables ont la signification indiquée dans la revendication 2, pour obtenir un Rylènetétracarbodiimide de formule Ia, dans laquelle z est égal à 0,
ou
b) d'abord avec 0,8 à 1,2 mole, par mole d'atome d'halogène à remplacer, d'un dérivé de Rylènedicarboximide de formule IIIa et ensuite avec 0,8 à 3 moles, par mole d'autre atome d'halogène à remplacer, d'un composé de formule générale IV
H-Z IV
ou d'abord avec 0,8 à 1,2 mole, par mole d'atome d'halogène à remplacer, d'un composé de formule IV et ensuite avec 0,8 à 1,2 mole, par mole d'autre atome d'halogène à remplacer, d'un dérivé de Rylènedicarboximide de formule IIIa
ou simultanément avec respectivement 0,8 à 1,2 mole, par mole d'atome d'halogène à remplacer, du dérivé de Rylènedicarboximide de formule IIIa et du composé de formule IV
pour obtenir un Rylènetétracarbodiimide de formule Ia, dans laquelle z est ≠ 0.

5. Procédé pour la préparation de multichromophores à base de Rylènedicarboximides de formule générale Ib 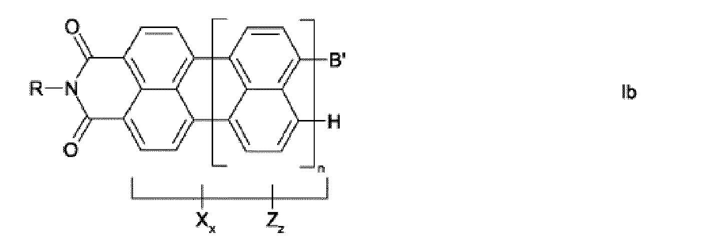 dans laquelle n est égal à 1, B' représente un atome d'hydrogène, un radical X ou un radical Z et les autres variables ont la signification indiquée dans la revendication 1, **caractérisé en ce qu'**on fait réagir un Rylènedicarboximide halogéné de formule générale IIb 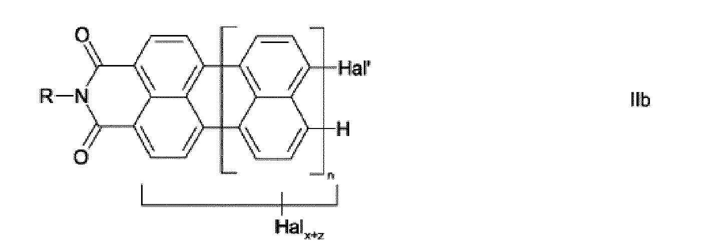 dans laquelle n est égal à 1 et Hal' représente un atome d'hydrogène ou Hal, Hal représentant le chlore ou le brome, en présence d'une base et d'un solvant non nucléophile
a) avec 0,8 à 1,2 mole, par mole d'atome d'halogène à remplacer, d'un dérivé de Rylènedicarboximide de formule générale IIIa 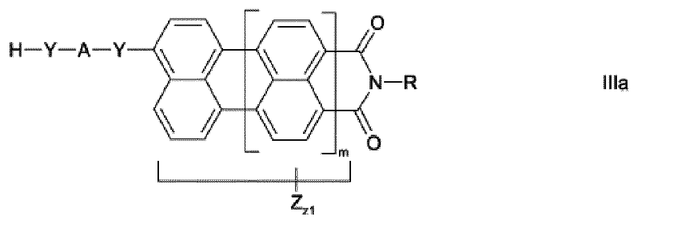 dans laquelle les variables ont la signification indiquée dans la revendication 2, pour obtenir un Rylènedicarboximide de formule Ib, dans laquelle z est égal à 0,
ou
b) d'abord avec 0,8 à 1,2 mole, par mole d'atome d'halogène à remplacer, d'un dérivé de Rylènedicarboximide de formule IIIa et ensuite avec 0,8 à 3 moles, par mole d'autre atome d'halogène à remplacer, d'un composé de formule générale IV
H-Z IV
ou d'abord avec 0,8 à 1,2 mole, par mole d'atome d'halogène à remplacer, d'un composé de formule IV et ensuite avec 0,8 à 1,2 mole, par mole d'autre atome d'halogène à remplacer, d'un dérivé de Rylènedicarboximide de formule IIIa
ou simultanément avec respectivement 0,8 à 1,2 mole, par mole d'atome d'halogène à remplacer, du dérivé de Rylènedicarboximide de formule IIIa et du composé de formule IV
pour obtenir un Rylènedicarboximide de formule Ib, dans laquelle z est ≠ 0.

6. Procédé pour la préparation de multichromophores à base de Rylènedicarboximides de formule générale Ib' 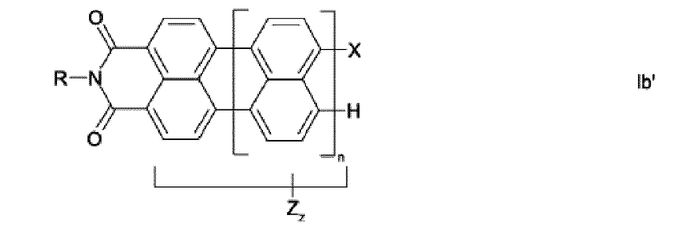 dans laquelle n = 2 ou 3 et z est égal à 2 à 8 et les autres variables ont la signification indiquée dans la revendication 1, **caractérisé en ce qu'**on fait réagir un péri-halogénoRylènedicarboximide de formule générale IIb' 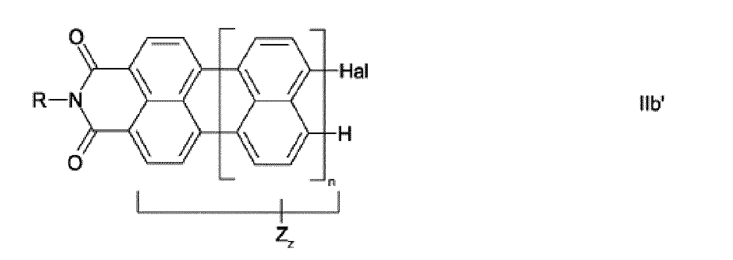 dans laquelle n = 2 ou 3 et z est égal à 2 à 8 et Hal représente le chlore ou le brome, en présence d'une base et d'un solvant non nucléophile, avec 0,8 à 1,2 mole par mole d'un dérivé de Rylènedicarboximide de formule générale IIIa

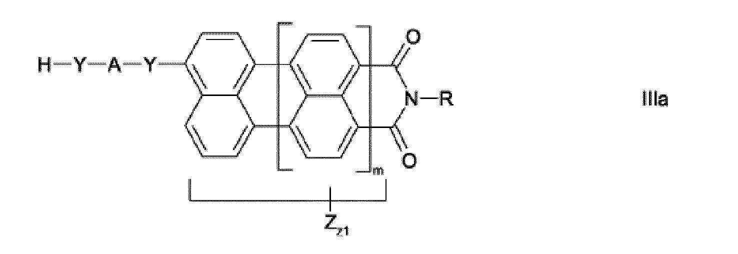

7. Procédé pour la préparation de multichromophores à base de Rylènetétracarbodiimides de formule générale Ic 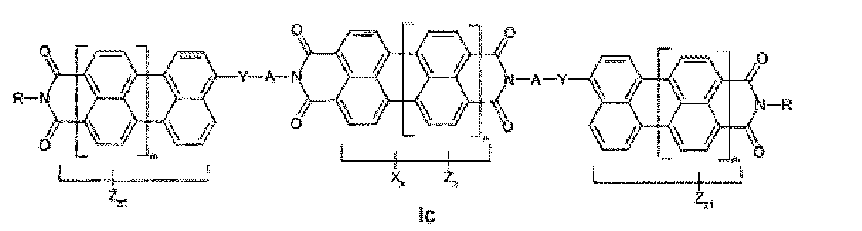 dans laquelle n est égal à 1, x représente 0 ou 2 à 4 et les autres variables ont la signification indiquée dans la revendication 1, **caractérisé en ce qu'**on fait réagir un dianhydride d'acide Rylènetétracarboxylique éventuellement halogéné de formule générale IIc 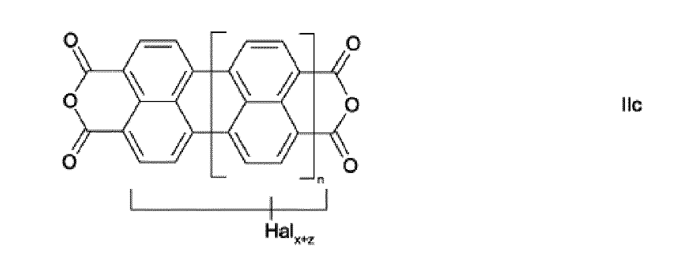 dans laquelle n est égal à 1 et Hal représente le chlore ou le brome, en présence d'un solvant à haut point d'ébullition et si on le désire d'un acide de Lewis, avec 1,8 à 3 moles par mole d'un dérivé de Rylènedicarboximide de formule générale IIIc 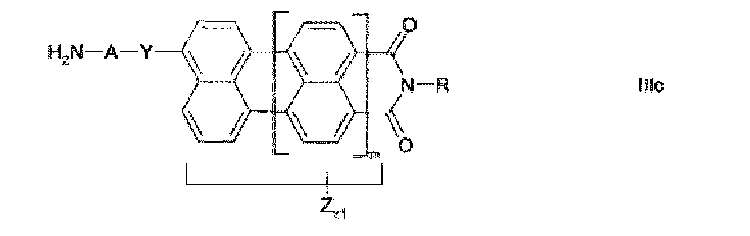 dans laquelle les variables ont la signification indiquée dans la revendication 2, pour obtenir un Rylènetétracarbodiimide de formule Ic, dans laquelle x = z = 0,
et éventuellement on fait réagir le Rylènetétracarbodiimide le halogéné sur le noyau Rylène, obtenu, après isolement intermédiaire, en présence d'une base et d'un solvant non nucléophile
a) avec 0,8 à 1,2 mole, par mole d'atome d'halogène à remplacer, d'un dérivé de Rylènedicarboximide de formule générale IIIa 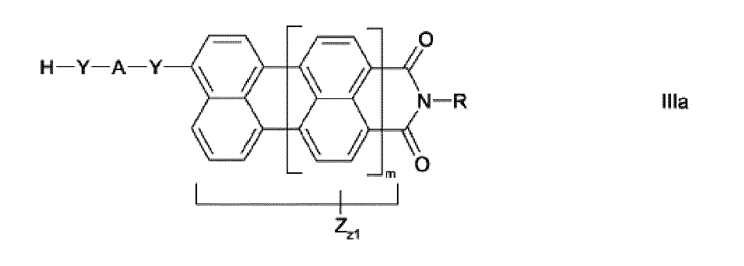 pour obtenir un Rylènetétracarbodiimide de formule Ic, dans laquelle x est ≠ 0 et z est égal à 0,
ou
b) avec 0,8 à 3 moles, par mole d'atome d'halogène à remplacer, d'un composé de formule générale IV
H-Z IV
pour obtenir un Rylènetétracarbodiimide de formule Ic, dans laquelle z est ≠ 0 et x est égal à 0,
ou
c) d'abord avec 0,8 à 1,2 mole, par mole d'atome d'halogène à remplacer, d'un dérivé de Rylènedicarboximide de formule IIIa et ensuite avec 0,8 à 3 moles, par mole d'autre atome d'halogène à remplacer, d'un composé de formule IV
ou d'abord avec 0,8 à 1,2 mole, par mole d'atome d'halogène à remplacer, d'un composé de formule IV et ensuite avec 0,8 à 1,2 mole, par mole d'autre atome d'halogène à remplacer, d'un dérivé de Rylènedicarboximide de formule IIIa
ou simultanément avec respectivement 0,8 à 1, 2 mole, par mole d'atome d'halogène à remplacer, du dérivé de Rylènedicarboximide de formule IIIa et du composé de formule IV
pour obtenir un Rylènetétracarbodiimide de formule Ic, dans laquelle x est ≠ 0 et z est ≠ 0.

8. Procédé pour la préparation de multichromophores à base de Rylènedicarboximides de formule générale Id 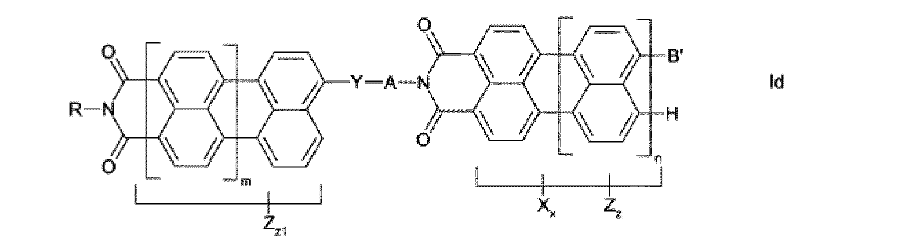 dans laquelle n est égal à 1, B' représente un atome d'hydrogène, un radical X ou un radical Z, x représente 0 ou 2 à 4 et les autres variables ont la signification indiquée dans la revendication 1, **caractérisé en ce qu'**on fait réagir un anhydride d'acide Rylènedicarboxylique éventuellement halogéné de formule générale IId 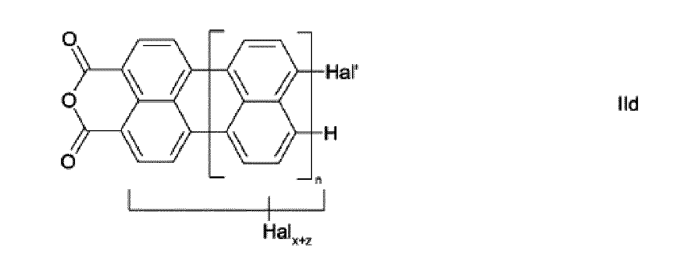 dans laquelle n est égal à 1 et Hal' représente un atome d'hydrogène ou Hal, Hal représentant le chlore ou le brome, en présence d'un solvant à haut point d'ébullition et si on le désire d'un acide de Lewis, avec 0,8 à 1,2 mole par mole d'un dérivé de Rylènedicarboximide de formule générale IIIc 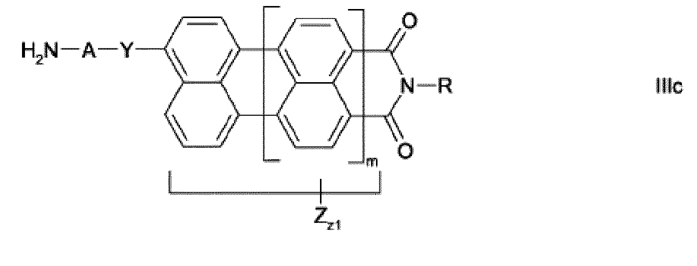 pour obtenir un Rylènedicarboximide de formule Id, dans laquelle x = z = 0,
et éventuellement on fait réagir le Rylènedicarboximide Id halogéné sur le noyau Rylène, obtenu, après isolement intermédiaire, en présence d'une base et d'un solvant non nucléophile
a) avec 0,8 à 1,2 mole, par mole d'atome d'halogène à remplacer, d'un dérivé de Rylènedicarboximide de formule générale IIIa 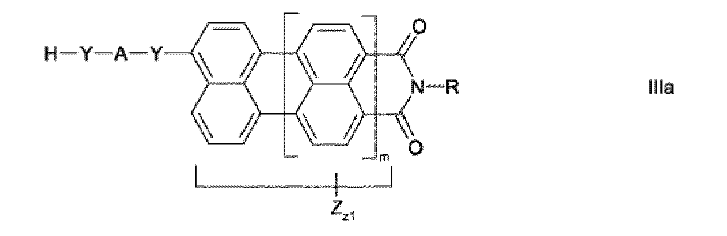 pour obtenir un Rylènedicarboximide de formule Id, dans laquelle x est ≠ 0 et z est égal à 0,
ou
b) avec 0,8 à 3 moles, par mole d'atome d'halogène à remplacer, d'un composé de formule générale IV
H-Z IV
pour obtenir un Rylènedicarboximide de formule Id, dans laquelle z est ≠ 0 et x est égal à 0,
ou
c) d'abord avec 0,8 à 1,2 mole, par mole d'atome d'halogène à remplacer, d'un dérivé de Rylènedicarboximide de formule IIIa et ensuite avec 0,8 à 3 moles, par mole d'autre atome d'halogène à remplacer, d'un composé de formule IV
ou d'abord avec 0,8 à 1,2 mole, par mole d'atome d'halogène à remplacer, d'un composé de formule IV et ensuite avec 0,8 à 1,2 mole, par mole d'autre atome d'halogène à remplacer, d'un dérivé de Rylènedicarboximide de formule IIIa
ou simultanément avec chaque fois 0,8 à 1,2 mole, par mole d'atome d'halogène à remplacer, du dérivé de Rylènedicarboximide de formule IIIa et du composé de formule IV
pour obtenir un Rylènedicarboximide de formule Id, dans laquelle x est ≠ 0 et z est ≠ 0.

9. Procédé pour la préparation de multichromophores à base de Rylènetétracarbodiimides de formule générale Ie 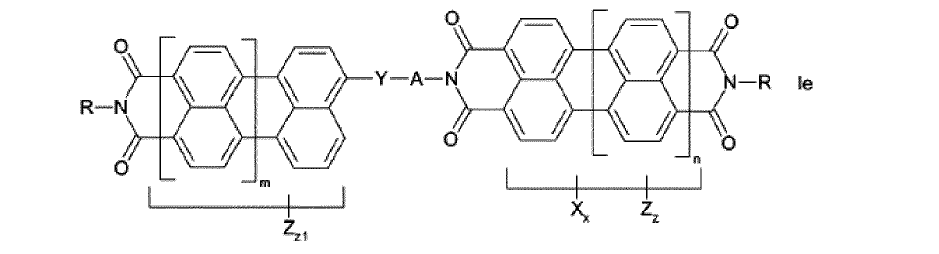 dans laquelle n est égal à 1 et les autres variables ont la signification indiquée dans la revendication 1, **caractérisé en ce qu'**on fait réagir un monoimide-monoanhydride d'acide Rylènetétracarboxylique éventuellement halogéné de formule générale IIe 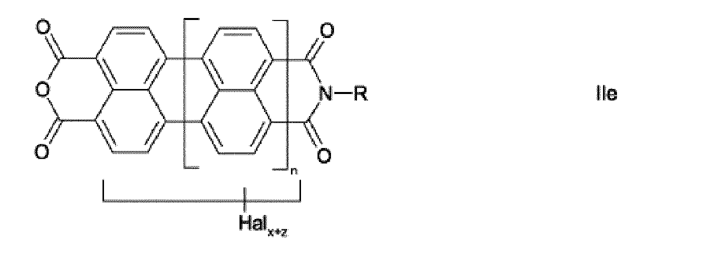 dans laquelle n est égal à 1 et Hal représente le chlore ou le brome, en présence d'un solvant à haut point d'ébullition et si on le désire d'un acide de Lewis, avec 0,8 à 1,2 mole par mole d'un dérivé de Rylènedicarboximide de formule générale IIIc 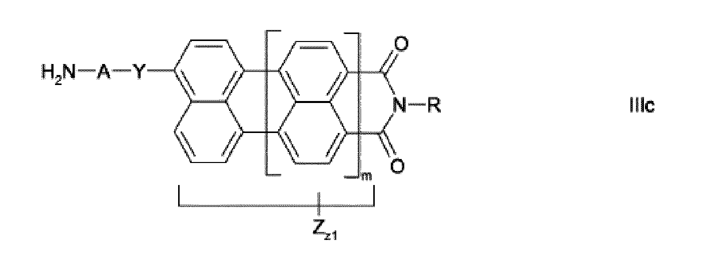 pour obtenir un Rylènetétracarbodiimide de formule Ie, dans laquelle x = z = 0,
et éventuellement on fait réagir le Rylènedicarboximide Ie halogéné sur le noyau Rylène, obtenu, après isolement intermédiaire, en présence d'une base et d'un solvant non nucléophile
a) avec 0,8 à 1,2 mole, par mole d'atome d'halogène à remplacer, d'un dérivé de Rylènedicarboximide de formule générale IIIa 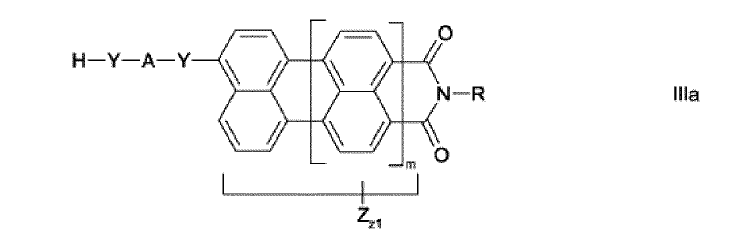 pour obtenir un Rylènetétracarbodiimide de formule Ie, dans laquelle x est ≠ 0 et z est égal à 0,
ou
b) avec 0,8 à 3 moles, par mole d'atome d'halogène à remplacer, d'un composé de formule générale IV
H-Z IV
pour obtenir un Rylènetétracarbodiimide de formule Ie, dans laquelle z est ≠ 0 et x est égal à 0,
ou
c) d'abord avec 0,8 à 1,2 mole, par mole d'atome d'halogène à remplacer, d'un dérivé de Rylènedicarboximide de formule IIIa et ensuite avec 0,8 à 3 moles, par mole d'autre atome d'halogène à remplacer, d'un composé de formule IV
ou d'abord avec 0,8 à 1,2 mole, par mole d'atome d'halogène à remplacer, d'un composé de formule IV et ensuite avec 0,8 à 1,2 mole, par mole d'autre atome d'halogène à remplacer, d'un dérivé de Rylènedicarboximide de formule IIIa
ou simultanément avec respectivement 0,8 à 1, 2 mole, par mole d'atome d'halogène à remplacer, du dérivé de Rylènedicarboximide de formule IIIa et du composé de formule IV
pour obtenir un Rylènetétracarbodiimide de formule Ie, dans laquelle x est ≠ 0 et z est ≠ 0.

10. Procédé pour la préparation de multichromophores à base de Rylènetétracarbodiimides de formule générale Ic' 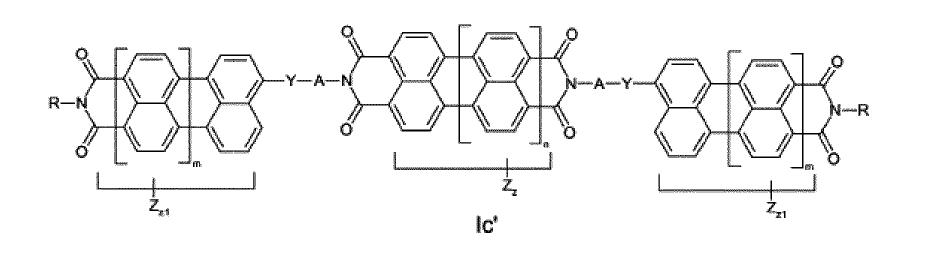 dans laquelle n = 2 ou 3 et z est égal à 2 à 8 et les autres variables ont la signification indiquée dans la revendication 1, **caractérisé en ce qu'**on fait réagir un dianhydride d'acide Rylènetétracarboxylique de formule générale IIc' 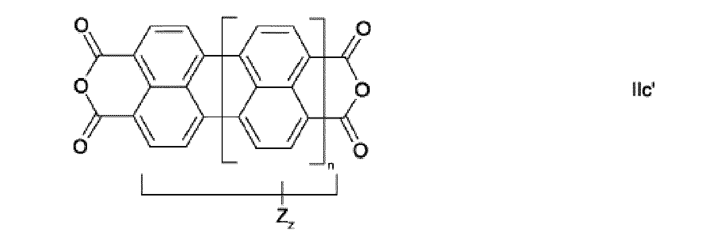 dans laquelle n = 2 ou 3 et z est égal à 2 à 8, en présence d'un solvant à haut point d'ébullition et d'un acide de Lewis, avec 0,8 à 1,2 mole par mole d'un dérivé de Rylènedicarboximide de formule générale IIIc

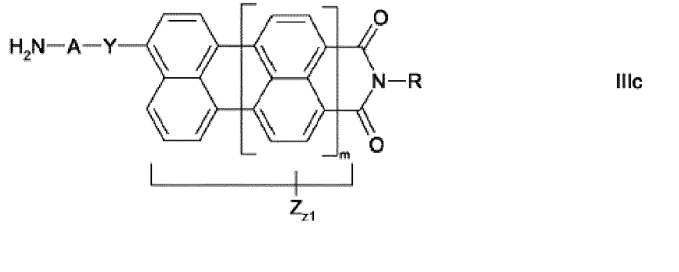

11. Procédé pour la préparation de multichromophores à base de Rylènedicarboximides de formule générale Id' 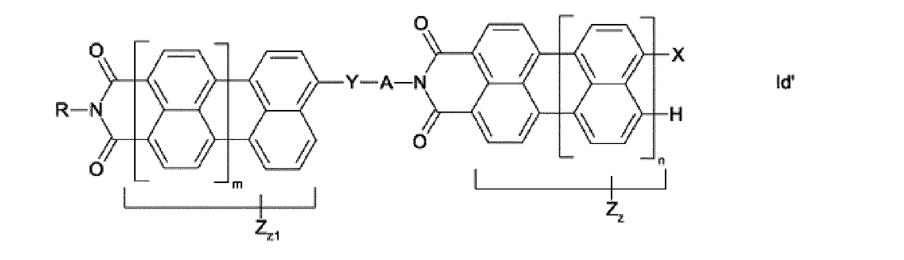 dans laquelle n = 2 ou 3 et z est égal à 2 à 8 et les autres variables ont la signification indiquée dans la revendication 1, **caractérisé en ce qu'**on fait réagir un anhydride d'acide périhalogénoRylènedicarboxylique de formule générale IId' 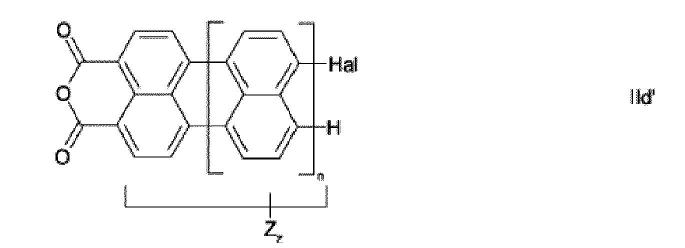 dans laquelle n = 2 ou 3 et z est égal à 2 à 8 et Hal représente le chlore ou le brome, en présence d'un solvant à haut point d'ébullition et d'un acide de Lewis, avec 0,8 à 1,2 mole par mole d'un dérivé de Rylènedicarboximide de formule générale IIIc

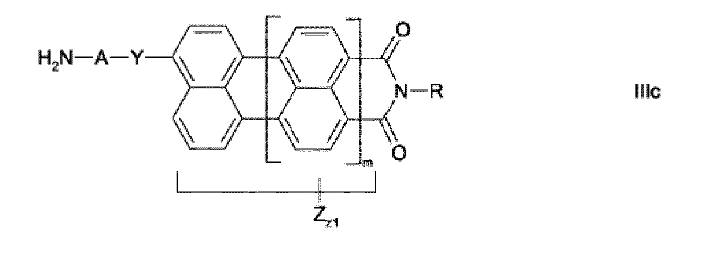

12. Procédé pour la préparation de multichromophores à base de Rylènetétracarbodiimides de formule générale Ie' 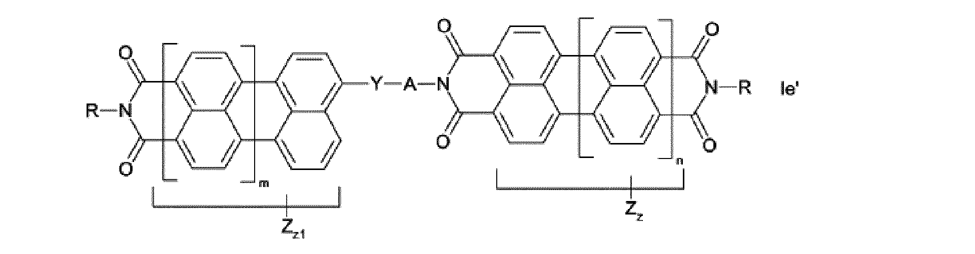 dans laquelle n = 2 ou 3 et z est égal à 2 à 8 et les autres variables ont la signification indiquée dans la revendication 1, **caractérisé en ce qu'**on fait réagir un monoimide-monoanhydride d'acide Rylènetétracarboxylique de formule générale IIe' 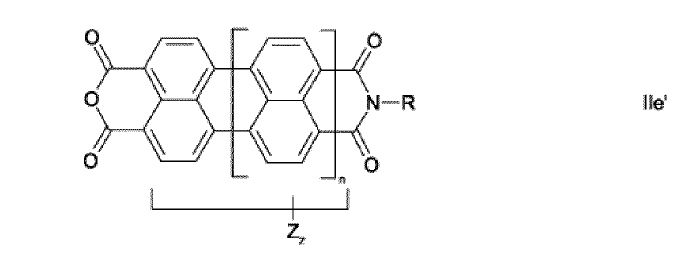 dans laquelle n est égal à 2 à 8, en présence d'un solvant à haut point d'ébullition et d'un acide de Lewis, avec 0,8 à 1,2 mole par mole d'un dérivé de Rylènedicarboximide de formule générale IIIc 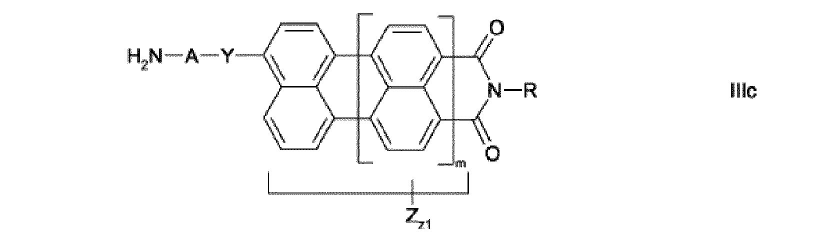 pour obtenir un Rylènetétracarbodiimide de formule Ie'.

13. Procédé pour la préparation de multichromophores à base d'esters d'acides Rylènedicarboxyliques de formule générale If 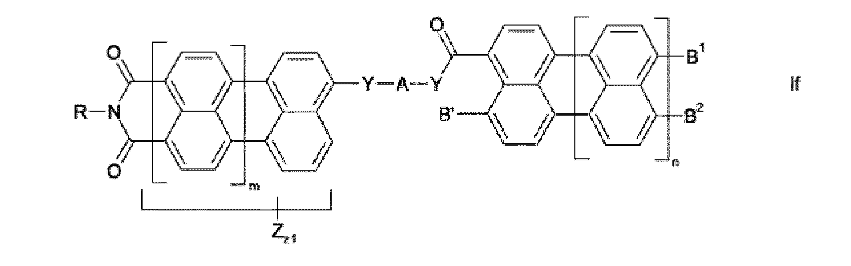 dans laquelle n = 1, B' représente un atome d'hydrogène ou d'halogène ou le groupe cyano, l'un des deux radicaux B¹ ou B² représente un radical de formule 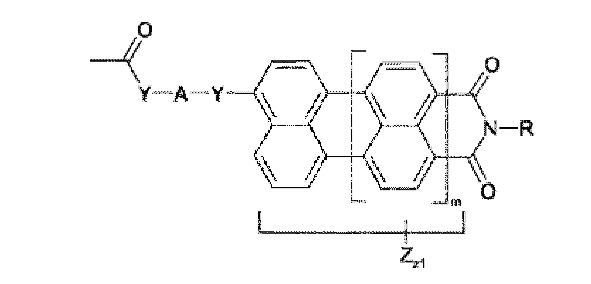 et l'autre radical représente un atome d'hydrogène ou d'halogène ou le groupe cyano et les autres variables ont la signification indiquée dans la revendication 1, **caractérisé en ce qu'**on convertit un dérivé de Rylènedicarboximide de formule générale IIIa 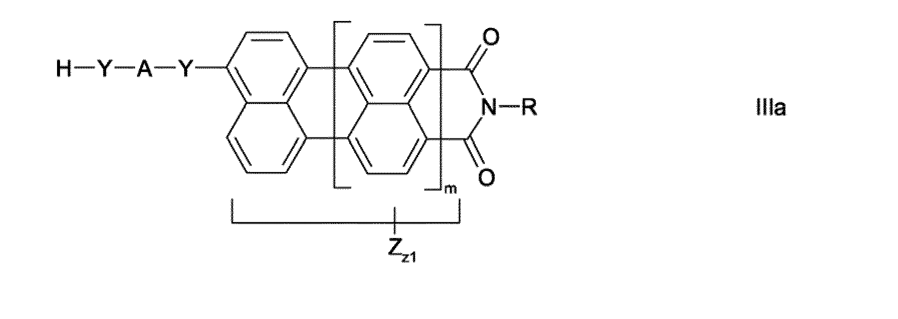 en présence d'une base contenant un métal alcalin et d'un solvant aprotique, en le sel de métal alcalin de formule générale IIIf 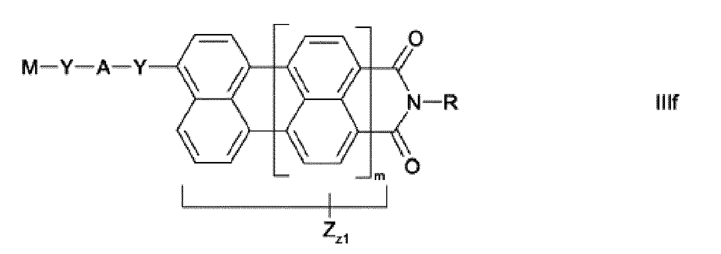 dans laquelle M représente un cation de métal alcalin, et ensuite on fait réagir ce dernier avec un chlorure d'acide Rylènedicarboxylique de formule générale IIf 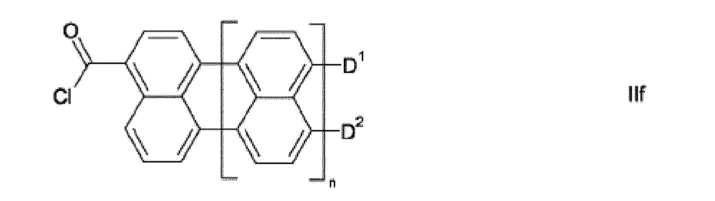 dans laquelle n est égal à 1 et l'un des deux radicaux D¹ ou D² représente -COCl et l'autre radical représente un atome d'hydrogène, en un rapport molaire de 0,8 : 1 à 1,2 : 1, pour aboutir à un ester d'acide Rylènedicarboxylique de formule If, dans laquelle B' et le radical B¹ ou B² restant représentent un atome d'hydrogène
et si on le désire on soumet à une halogénation l'ester d'acide Rylènedicarboxylique obtenu, après isolement intermédiaire, en présence d'un solvant organique polaire et d'un acide de Lewis, à l'aide d'un N-halogénosuccinimide, pour aboutir à l'ester d'acide Rylènedicarboxylique de formule If, dans laquelle B' et le radical B ¹ ou B² restant représentent un atome d'halogène
et si on le désire on convertit l'ester d'acide Rylènedicarboxylique halogéné obtenu, à l'aide d'un cyanure métallique, en présence d'un solvant aprotique et d'un catalyseur à métal de transition, en l'ester d'acide Rylènedicarboxylique correspondant, substitué par cyano, de formule If, dans laquelle B' et le radical B¹ ou B² restant représentent le groupe cyano.

14. Procédé pour la préparation de multichromophores à base de Rylènedicarboxamides de formule générale Ig 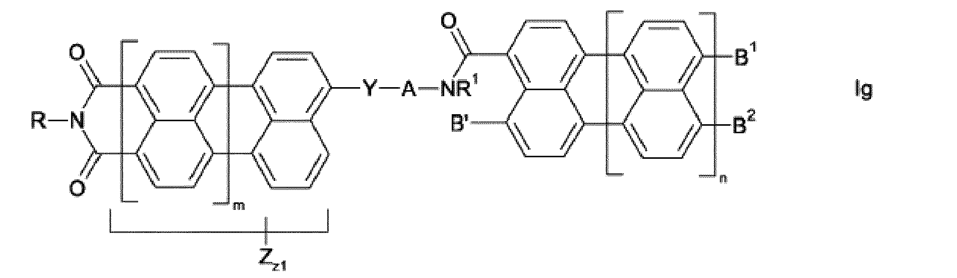 dans laquelle n est égal à 1, B' représente un atome d'hydrogène ou d'halogène ou le groupe cyano, l'un des deux radicaux B¹ ou B² représente un radical de formule 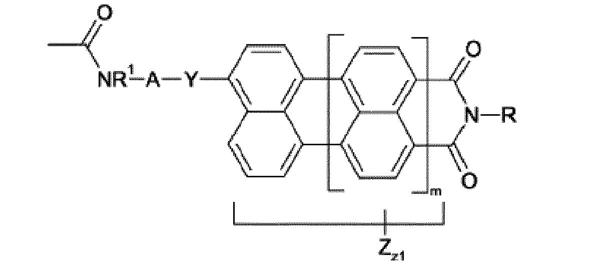 et l'autre radical représente un atome d'hydrogène ou d'halogène ou le groupe cyano et les autres variables ont la signification indiquée dans la revendication 1, **caractérisé en ce qu'**on fait réagir un chlorure d'acide Rylènedicarboxylique de formule générale IIf 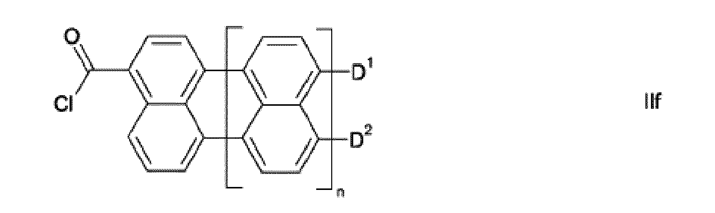 dans laquelle n est égal à 1 et l'un des deux radicaux D¹ ou D² représente -COCl et l'autre radical représente un atome d'hydrogène, en présence d'une base non nucléophile et si on le désire en outre d'un solvant aprotique, avec 0,8 à 1, 2 mole par mole d'un dérivé de Rylènedicarboximide de formule générale IIIg 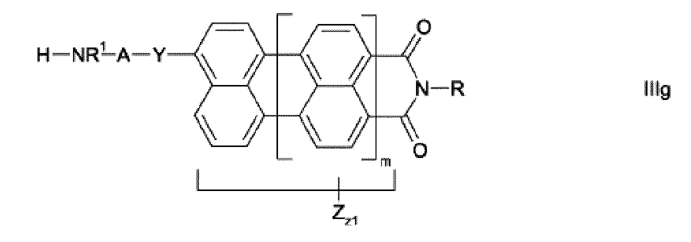 pour aboutir à un Rylènedicarboxamide de formule Ig, dans laquelle B' et le radical B¹ ou B² restant représentent un atome d'hydrogène
et si on le désire on soumet à une halogénation le Rylènedicarboxamide obtenu, après isolement intermédiaire, en présence d'un solvant organique polaire et d'un acide de Lewis, à l'aide d'un N-halogénosuccinimide, pour aboutir au Rylènedicarboxamide de formule Ig, dans laquelle B' et le radical B¹ ou B² restant représentent un atome d'halogène
et si on le désire on convertit le Rylènedicarboxamide halogéné obtenu, à l'aide d'un cyanure métallique, en présence d'un solvant aprotique et d'un catalyseur à métal de transition, en le Rylènedicarboxamide correspondant, substitué par cyano, de formule Ig, dans laquelle B' et le radical B¹ ou B² restant représentent le groupe cyano.

15. Dérivés de Rylènedicarboximides de formule générale III 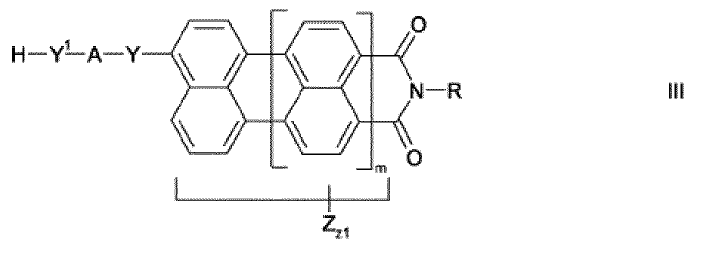 dans laquelle les variables ont la signification suivante :
Z représente un groupe aryloxy, arylthio, hétéroaryloxy ou hétéroarylthio, à chacun desquels peuvent être soudés d'autres cycles saturés ou insaturés à 5 à 7 chaînons, dont le squelette carboné peut être interrompu par un ou plusieurs groupements -O-, -S-, -NR¹-, -N=CR¹-, -CO-, -SO- et/ou -SO₂-, le système cyclique dans son ensemble pouvant être une ou plusieurs fois substitué par les radicaux (i), (ii), (iii), (iv) et/ou (v) :
(i) alkyle en C₁-C₃₀, dont la chaîne carbonée peut être interrompue par un ou plusieurs groupements -O-, -S-, -NR¹-, -N=CR¹-, -CºC-, -CR¹=CR¹-, -CO-, -SO- et/ou -SO₂- et qui peut être une ou plusieurs fois substitué par : alcoxy en C₁-C₁₂, alkyl (C₁-C₆) thio, -CºCR¹, -CR¹=CR¹₂, hydroxy, mercapto, halogéno, cyano, nitro, -NR²R³, -NR²COR³, -CONR²R³, -SO₂NR²R³, -COOR², -SO₃R², aryle et/ou cycloalkyle en C₄-C₇ saturé ou insaturé, dont le squelette carboné peut être interrompu par un ou plusieurs groupements -O-, -S-, -NR¹-, -N=CR¹-, -CR¹=CR¹-, -CO-, -SO- et/ou -SO₂-, les radicaux aryle et cycloalkyle pouvant être une ou plusieurs fois substitués chacun par alkyle en C₁-C₁₈ et/ou par les radicaux précédents, nommés en tant que substituants pour alkyle ;
(ii) cycloalkyle en C₃-C₈, dont le squelette carboné peut être interrompu par un ou plusieurs groupements -O-, -S-, -NR¹-, -N=CR¹-, -CR¹=CR¹-, -CO-, -SO- et/ou -SO₂- et auquel peuvent être soudés d'autres cycles saturés ou insaturés à 5 à 7 chaînons, dont le squelette carboné peut être interrompu par un ou plusieurs groupements -O-, -S-, -NR¹-, -N=CR¹-, -CR¹=CR¹-, -CO-, -SO-et/ou -SO₂-, le système cyclique dans son ensemble pouvant être une ou plusieurs fois substitué par : alkyle en C₁-C₁₈, alcoxy en C₁-C₁₂, alkyl (C₁-C₆) thio, -CºCR¹, -CR¹=CR¹₂, hydroxy, mercapto, halogéno, cyano, nitro, -NR²R³, -NR²COR³, -CONR²R³, SO₂NR²R³, -COOR² et/ou -SO₃R²;
(iii) aryle ou hétéroaryle, auquel peuvent être soudés d'autres cycles saturés ou insaturés à 5 à 7 chaînons, dont le squelette carboné peut être interrompu par un ou plusieurs groupements -O-, -S-, -NR¹-, -N=CR¹-, -CR¹=CR¹-, -CO-, -SO-et/ou -SO₂-, le système cyclique dans son ensemble pouvant être une ou plusieurs fois substitué par : alkyle en C₁-C₁₈, alcoxy en C₁-C₁₂, alkyl (C₁-C₆) thio, -CºCR¹, -CR¹=CR¹₂, hydroxy, mercapto, halogéno, cyano, nitro, -NR²R³, -NR²COR³, -CONR²R³, -SO₂NR²R³, -COOR², -SO₃R², aryle et/ou hétéroaryle, qui peut dans chaque cas être une ou plusieurs fois substitué par alkyle en C₁-C₁₈, alcoxy en C₁-C₁₂, hydroxy, mercapto, halogéno, cyano, nitro, -NR²R³, -NR²COR³, -CONR²R³, -SO₂NR²R³, -COOR² et/ou -SO₃R²;
(iv) un radical -U-aryle qui peut être une ou plusieurs fois substitué par les radicaux précédents, nommés en tant que substituants pour les radicaux aryle (iii), U représentant un groupement -O-, -S-, -NR¹-, -CO-, -SO- ou -SO₂- ;
(v) alcoxy en C₁-C₁₂, alkyl (C₁-C₆) thio, -CºCR¹, -CR¹=CR¹₂, hydroxy, mercapto, halogéno, cyano, nitro, -NR²R³, -NR²COR³, -CONR²R³, -SO₂NR²R³, -COOR² ou -SO₃R²,
les radicaux Z pour z > 1 et/ou z1 > 1 pouvant être identiques ou différents ;
A représente un chaînon pontant comportant au moins un radical aromatique ou hétéroaromatique, les groupes Y ou Y et Y¹ étant liés au radical aromatique ou hétéroaromatique ;
Y est -O- ou -S- ;
Y¹ est -O-, -S- ou -NR¹- ;
R représente un atome d'hydrogène ;
un groupe alkyle en C₁-C₃₀, dont la chaîne carbonée peut être interrompue par un ou plusieurs groupements -O-, -S-, -NR¹-, -N=CR¹-, -CºC-, -CR¹=CR¹-, -CO-, -SO- et/ou -SO₂- et qui peut être une ou plusieurs fois substitué par les radicaux (ii), (iii), (iv) et/ou (v) nommés en tant que substituants pour les radicaux Z ;
cycloalkyle en C₃-C₈, auquel peuvent être soudés d'autres cycles saturés ou insaturés à 5 à 7 chaînons, dont le squelette carboné peut être interrompu par un ou plusieurs groupements -O-, -S-, -NR¹-, -N=CR¹-, -CR¹=CR¹-, -CO-, -SO- et/ou -SO₂-, le système cyclique dans son ensemble pouvant être une ou plusieurs fois substitué par les radicaux (i), (ii), (iii), (iv) et/ou (v) nommés en tant que substituants pour les radicaux Z ;
aryle ou hétéroaryle, auquel peuvent être soudés d'autres cycles saturés ou insaturés à 5 à 7 chaînons, dont le squelette carboné peut être interrompu par un ou plusieurs groupements -O-, -S-, -NR¹-, -N=CR¹-, -CR¹=CR¹-, -CO-, -SO- et/ou -SO₂-, le système cyclique dans son ensemble pouvant être une ou plusieurs fois substitué par les radicaux (i), (ii), (iii), (iv), (v) nommés en tant que substituants pour les radicaux Z ;
et/ou aryl- et/ou hétéroarylazo, qui peut dans chaque cas être une ou plusieurs fois substitué par alkyle en C₁-C₁₀, alcoxy en C₁-C₆ et/ou cyano, les radicaux R pouvant être identiques ou différents lorsqu'ils apparaissent plusieurs fois dans la formule I ;
R¹ représente un atome d'hydrogène ou un groupe alkyle en C₁-C₁₈, les radicaux R¹ pouvant être identiques ou différents lorsqu'ils apparaissent plusieurs fois ;
R², R³ représentent indépendamment l'un de l'autre un atome d'hydrogène ;
un groupe alkyle en C₁-C₁₈, dont la chaîne carbonée peut être interrompue par un ou plusieurs groupements -O-, -S-, -CO-, -SO- et/ou -SO₂- et qui peut être une ou plusieurs fois substitué par alcoxy en C₁-C₁₂, alkyl(C₁-C₆)thio, hydroxy, mercapto, halogéno, cyano, nitro et/ou -COOR¹ _{;}
un groupe aryle ou hétéroaryle, à chacun desquels peuvent être soudés d'autres cycles saturés ou insaturés à 5 à 7 chaînons, dont le squelette carboné peut être interrompu par un ou plusieurs groupements -O-, -S-, -CO- et/ou -SO₂-, le système cyclique dans son ensemble pouvant être une ou plusieurs fois substitué par alkyle en C₁-C₁₂ et/ou par les radicaux précédents, nommés en tant que substituants pour alkyle ;
m vaut 0, 1 ou 2 ;
z1 pour m = 0 : 0 ;
pour m = 1 : 0 à 2 ;
pour m = 2 : 2 à 4.

16. Procédé pour la préparation de dérivés Rylène de formule III selon la revendication 15, **caractérisé en ce qu'**on fait réagir un Rylènedicarboximide péri-halogéné de formule générale V 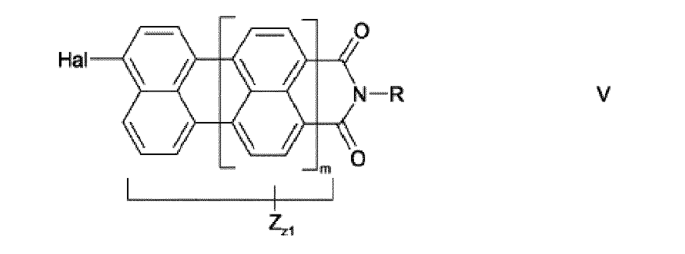 dans laquelle Hal représente le chlore ou le brome, en présence d'une base et d'un solvant non nucléophile, avec 2 à 5 moles, par mole d'atome d'halogène à remplacer, d'un composé aromatique au moins bifonctionnel de formule générale VI
H-Y¹-A-Y-H VI.

17. Utilisation de multichromophores selon les revendications 1 à 3 pour la coloration de matériaux organiques et de matériaux inorganiques.

18. Utilisation selon la revendication 17, **caractérisée en ce que** les matériaux organiques sont des peintures, des encres d'impression ou des matières plastiques.

19. Utilisation de multichromophores selon les revendications 1 à 3 pour la production de dispersions aqueuses de polymérisats absorbant et/ou émettant un rayonnement électromagnétique.

20. Utilisation de multichromophores selon les revendications 1 à 3 pour la production de marquages et d'inscriptions non visibles pour l'oeil humain.

21. Utilisation de multichromophores selon les revendications 1 à 3 en tant que filtres ou émetteurs dans des applications d'affichage.

22. Utilisation de multichromophores selon les revendications 1 à 3 en tant qu'émetteurs dans des applications de chimiluminescence.

23. Utilisation de multichromophores selon les revendications 1 à 3 en tant que composants actifs en photovoltaïque.
